# FASCICULE DE BREVET EUROPEEN

(11) **EP 1 856 065 B1**
(45) Date de publication et mention de la délivrance du brevet: **11.11.2009**
(21) Numéro de dépôt: 06726008.3
(22) Date de dépôt: 02.03.2006
(51) Int. Cl.: C07D 253/06, A61K 31/66, A61P 3/06, A61P 3/04, A61P 3/10, A61P 17/00

(54) **DERIVES DE 1 ,2,4-TRIAZINES, LEUR PREPARATION ET LEUR APPLICATION EN THERAPEUTIQUE HUMAINE**
1,2,4-TRIAZINDERIVATE, IHRE HERSTELLUNG UND IHRE VERWENDUNG IN DER HUMANTHERAPIE
1,2,4-TRIAZINE DERIVATIVES, PREPARATION AND USE THEREOF IN HUMAN THERAPY

(30) Priorité: 03.03.2005 FR 0502152
(43) Date de publication de la demande: 21.11.2007
(73) Titulaire: PIERRE FABRE MEDICAMENT, 92100 Boulogne-Billancourt (FR)
(72) Inventeur: DUPONT-PASSELAIGUE, Elisabeth, F-81100 Castres (FR); LEROY, Isabelle, F-81710 Saix (FR); PATOISEAU, Jean-François, F-81100 Castres (FR); JUNQUERO, Didier, 81100 Castres (FR); RIVAL, Yves, F-81090 Lagarrigue (FR); DELHON, André, F-81100 Castres (FR)
(74) Mandataire: Ahner, Francis
(86) Numéro de dépôt international: PCT/FR2006/000469
(87) Numéro de publication internationale: WO 2006/092507

(56) Documents cités:
- WO-A-95/01965
- WO-A-02/096894
- WO-A-2005/080354

## Description

La présente invention a pour objet de nouveaux dérivés de la 3,5-dioxo-(2H,4H)-1,2,4-triazine fonctionnalisés en 2, 4 et 6, activant les récepteurs PPAR alpha et/ou gamma, leur préparation et leur application en thérapeutique humaine.

Le syndrome métabolique est le résultat d'une résistance périphérique à l'insuline accrue, et se caractérise par une hyper-insulinémie, une intolérance au glucose, une altération du métabolisme des lipides, une hypertension artérielle (Grundy, S.M. : Hypertriglyceridemia, insulin résistance, and the metabolic syndrome. Am. J. Cardiol.15 1999, 83, 25F-29F*).* L'obésité est souvent associée à ces troubles métaboliques, et la conjonction de ces multiples facteurs de risque favorise le développement de la pathologie athéromateuse à l'origine de la thrombose artérielle, aujourd'hui première cause de mortalité dans les régions industrialisées. Les récepteurs aux agents proliférateurs de peroxisomes (PPAR) appartiennent à la superfamille des récepteurs nucléaires des facteurs de transcription. Après activation, ils forment un hétérodimère avec le récepteur de l'acide 9-cis rétinoïque (RXR) ; ce complexe (PPAR-RXR) se lie spécifiquement sur des séquences d'ADN situées dans des régions régulatrices de gènes impliqués dans le métabolisme des lipides et glucides (Pineda Torra, I., Gervois, P. and Staels, B. :Peroxisome proliferator-activated receptor alpha in metabolic disease, inflammation, atherosclerosis and aging. Curr. Opin. Lipidol. 1999, 10, 151-159. Vamecq, J. and Latruffe, N. : Medical signifiance of peroxisome proliferator-activated receptors. Lancet 1999, 354, 141-148*.).* L'activation des PPARs d'une part restaure certaines voies métaboliques altérées qui prédisposent à l'athérosclérose, d'autre part réduit les événements inflammatoires qui favorisent le développement et la fissuration de la plaque d'athérome.

La demande internationale WO 95/01965 décrit des composés possédant une structure 35-dioxo-(2H,4H)-1,2,4-triazine utiles pour les maladies liées à la prise de nourriture.

Les composés de la présente invention se caractérisent par leur structure originale, leur affinité vis-à-vis des récepteurs PPAR alpha et/ou PPAR gamma et leur profil pharmacologique.

Les composés de l'invention correspondent à la formule générale **I.** 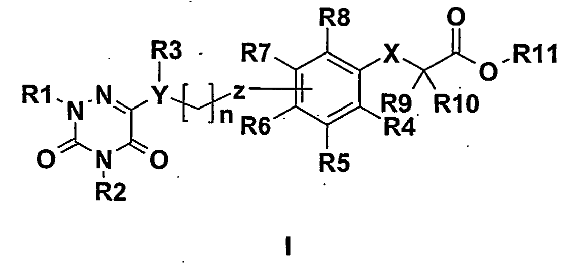 dans laquelle
- **R₁** et **R₂** peuvent être identiques ou différents et représentent un radical alkyle ou alkènyle linéaire ou branché en C₁-C₇, un radical alkyle en C₁-C₆ substitué par des groupements tels que, trifluorométhyle, cycloalkyle en C₅-C₆, nitrile, alkoxycarbonylvinyle en C₁-C₄, hydroxycarbonylvinyle, alkoxycarbonyle en C₁-C₄, carboxylate, benzyloxy ou phényle (pour lesquels le noyau phényle est éventuellement substitué par un ou plusieurs groupements tels que alcoyle en C₁-C₄, alkoxy en C₁-C₄, nitro, halogène, trifluorométhyle).
- **YR₃** représente l'oxygène ou **NR₃** pour lequel **R₃** représente l'hydrogène, un radical alcoyle ou alkènyle linéaire ou branché en C₁-C₇, un radical alkyle en C₁-C₆ substitué par des groupements tels que, trifluorométhyle ou phényle (pour lesquels le noyau phényle est éventuellement substitué par un ou plusieurs groupements tels que alcoyle en C₁-C₄, alkoxy en C₁-C₄, nitro, halogène, trifluorométhyle)
- **Z** représente un atome d'oxygène ou un atome de carbone pouvant être lié aux positions ortho, méta ou para du groupement phényle de la formule **I**
- **n** peut-être égal à 0 à 5 lorsque **Z**=C ou 2 à 4 lorsque **Z**=O,
- **X** représente l'oxygène ou le soufre
- **R₄, R₅, R₆, R₇ et R₈** représentent l'hydrogène ou le fluor,
- **R₉, R₁₀** et **R₁₁** représentent l'hydrogène ou un groupement alkyle en C₁-C₅ linéaire ou branché
ainsi que les sels d'addition avec les bases pharmaceutiquement acceptables, et les différents énantiomères des composés possédant des carbones asymétriques, ainsi que leurs mélanges en toutes proportions incluant notamment les mélanges racémiques.

L'invention concerne en particulier les composés de formule **1** dans laquelle
- **R₁** et **R₂** représentent indépendamment l'un de l'autre, un radical alkyle ou alkènyle linéaire ou branché en C₁-C₇; un radical alkyle en C₁-C₆ substitué par des groupements tels que, trifluorométhyle, cycloalkyle en C₆, nitrile, phényle (pour lesquels le noyau phényle est éventuellement substitué par un ou plusieurs groupements tels que alcoyle en C₁-C₄, alkoxy en C₁-C₄, nitro, halogène, trifluorométhyle),
- **YR₃** représente l'oxygène ou **NR₃** pour lequel **R₃** représente l'hydrogène, un radical alcoyle ou alkènyle linéaire ou branché en C₁-C₇, un radical alkyle en C₁-C₆ substitué par des groupements tels que, trifluorométhyle où phényle,
- **Z** représente un atome d'oxygène ou un atome de carbone pouvant être lié aux positions ortho, méta ou para du groupement phényle de la formule **I**
- **n** peut-être égal à 0 à 5 lorsque **Z=C** ou 2 à 4 lorsque **Z**=O,
- **X** représente l'oxygène ou le soufre
- **R₄, R₅, R₆, R₇ et R₈** représentent l'hydrogène ou le fluor,
- **R₉, R₁₀** et **R₁₁** représentent l'hydrogène ou un groupement alkyle en C₁-C₅ linéaire ou branché

L'invention concerne plus particulièrement les composés de formule 1 dans laquelle :
- **R₁** et **R₂** représentent indépendamment l'un de l'autre, un radical alkyle ou alkènyle linéaire ou branché en C₁-C₇, un radical alkyle en C₁-C₆ substitué par des groupements tels que, trifluorométhyle ou nitrile,
- **YR₃** représente l'oxygène ou **NR₃** pour lequel **R₃** représente l'hydrogène ou un radical alcoyle linéaire ou branché en C₁-C₇,
- **Z** représente un atome de carbone pouvant être lié aux positions ortho, méta ou para du groupement phényle de la formule **I**
- **n** peut-être égal à 0 à 5,
- **X** représente l'oxygène ou le soufre
- **R₄, R₅, R₆, R₇** et **R₈** représentent l'hydrogène ou le fluor,
- **R₉, R₁₀** et **R₁₁** représentent l'hydrogène ou un groupements alkyle en C₁-C₅ linéaire ou branché, en particulier **R₉** et **R₁₀** représentent le groupement méthyle et **R₁₁** l'hydrogène ou le groupement éthyle,

L'invention concerne encore plus particulièrement des dérivés de la 3,5-dioxo-(2H,4H)-1,2,4-triazine de formule **I** dans laquelle :
- **R₁** et **R₂** représentent indépendamment l'un de l'autre, un radical alkyle ou alkènyle linéaire ou ramifié en C₁-C₇ éventuellement substitué en bout de chaîne par un groupement trifluorométhyle,
- **YR₃** représente l'oxygène ou **NR₃** pour lequel **R₃** représente l'hydrogène ou un radical alcoyle linéaire ou branché en C₁-C₇,
- **Z** représente un atome de carbone pouvant être lié aux positions méta ou para du groupement phényle de la formule **I**
- **n** peut-être égal à 1 à 5,
- **X** représente l'oxygène ou le soufre
- **R₄** à **R₈** représentent l'hydrogène,
- **R₉** et **R₁₀** représentent un radical méthyl
- **R₁₁** représente l'hydrogène ou un radical éthyl

L'invention couvre les sels des composés de formule générale **I** avec les bases pharmaceutiquement acceptables, ainsi que les différents énantiomères des composés possédant des carbones asymétriques, ainsi que leurs mélanges en toutes proportions incluant notamment les mélanges racémiques.

### SYNTHESE

Les composés de la présente invention peuvent être synthétisés en utilisant les voies de synthèse décrites ci-dessous ou en utilisant des méthodes de synthèse connues de l'homme de métier.

### Méthode 1

La synthèse des composés de formule générale **I** est caractérisée (schéma 1) en ce que l'on condense un dérivé de formule générale **II** 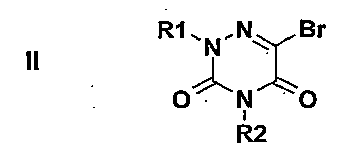 dans lequel **R₁** et **R₂** représentent les groupements tels que décrits précédemment dans la formule **I** avec un dérivé de formule générale **III** 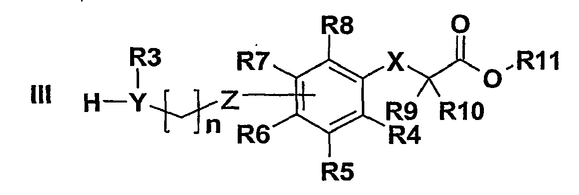 où **YR₃**, **n, Z, X, R₄, R₅, R₆, R₇, R₈, R₉, R₁₀** et **R₁₁** sont tels que décrits précédemment dans la formule **I.** Cette réaction peut être effectuée en présence d'une base telle que la triéthylamine dans le nbutanol (lorsque **Y**=N) ou de carbonate de potassium dans le diméthylformamide (lorsque **YR₃**=O);

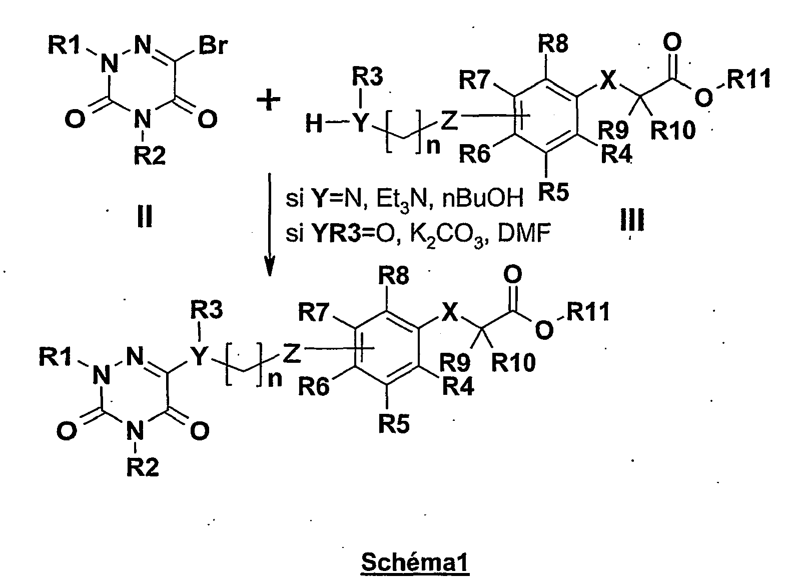

### Méthode 2

Cette méthode de synthèse des composés dé formule générale **I** pour lesquels **Z**=O (schéma 2) est caractérisée en ce que:
1) l'on condense un dérivé de formule générale **II** 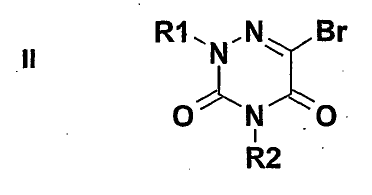 dans lequel **R₁** et **R₂** représentent les groupements tels que décrits précédemment dans la formule **I** avec un dérivé de formule générale **IV** 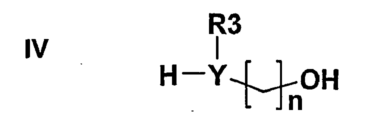 dans lequel **R₃Y** peut être égal à NH ou O et n est tel que décrit précédemment dans la formule **I.** Cette réaction peut être effectuée en l'absence de solvant sans ajouter de base (dans le cas où **R₃Y**=NH) ou en présence d'une base telle que K₂CO₃ (dans le cas où **R₃Y**=O).
2) l'on condense le dérivé obtenu **V** 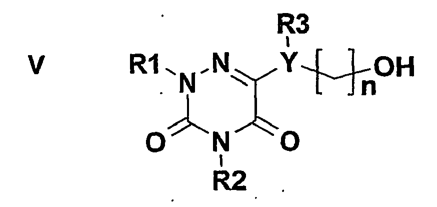 avec un composé de formule générale **VI** 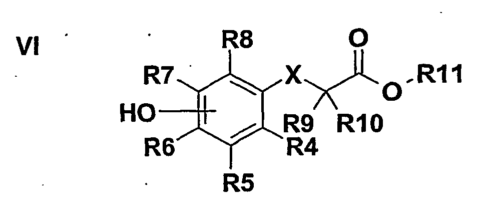 où **X, R₄, R₅, R₆, R₇, R₈, R₉, R₁₀** et **R₁₁** sont tels que décrits précédemment dans la formule **I.** Cette réaction peut être effectuée dans des conditions telles que celles de la réaction de Mitsunobu en présence de triphénylphosphine et de diéthylazodicarboxylate dans le **THF**.

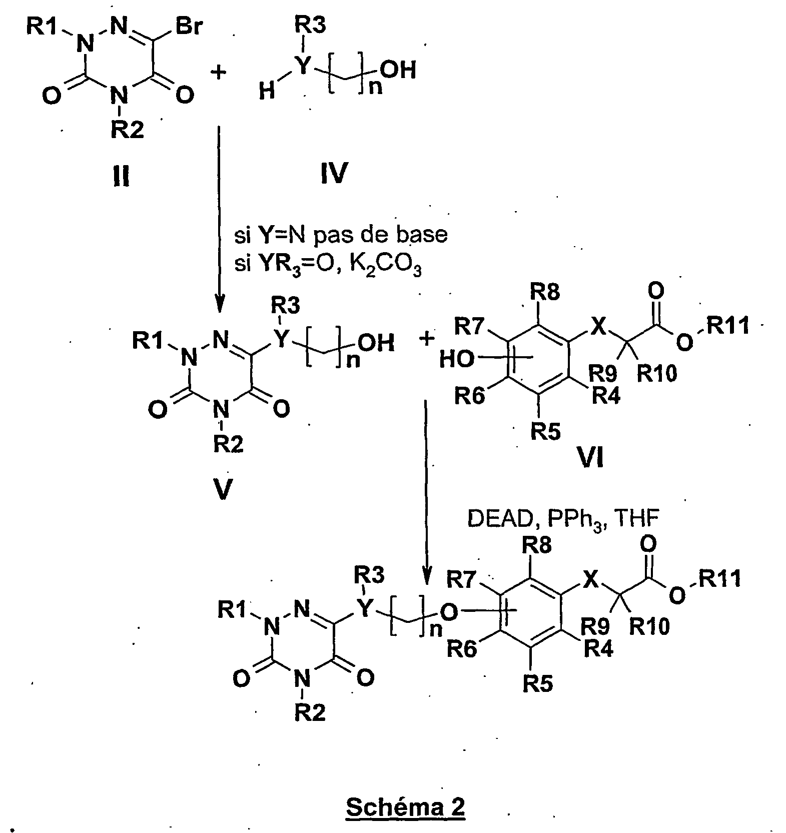

### Méthode 3

Cette méthode de synthèse des composés de formule générale **I** pour lesquels **Z**=O (schéma 3) est caractérisée en ce que:
1) l'on protège la fonction alcool d'un dérivé de formule générale **VII** 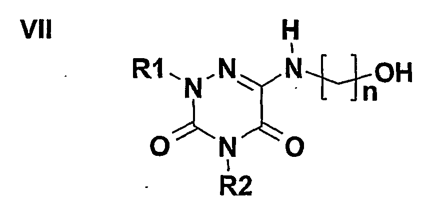 dans lequel **R₁, R₂** et n sont tels que décrit précédemment dans la formule **I** par un groupement protecteur parmi lesquels le tert-butyldiméthylsilane. Cette réaction peut-être effectuée dans des conditions telles que le THF en utilisant le chlorotertbutyldiméthylsilane et l'imidazole. 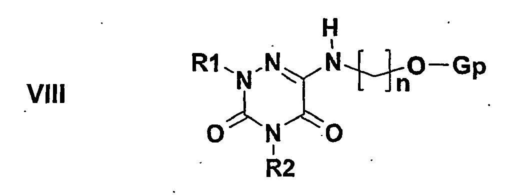
2) l'on alkyle l'azote du composé VIII précédemment obtenu par un dérivé halogéné **R₃**Hal dans lequel le groupement **Hal** représente un halogène tel que CI, Br ou **I** est **R₃** est tel que décrit précédemment dans la formule **I**, dans des conditions opératoires telles que NaH ou tBuOK dans le DMF. 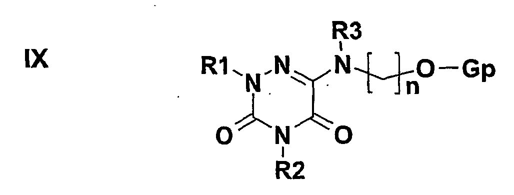
3) l'on déprotège le composé **IX** ainsi obtenu dans des conditions opératoires telles que le fluorure de tétrabutylammonium dans le THF.
4) l'on condense le dérivé obtenu **X** 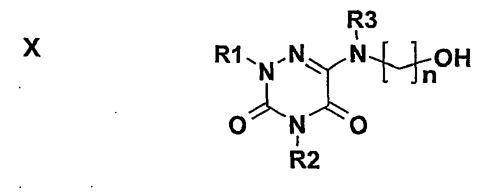 avec un composé de formule générale **VI** 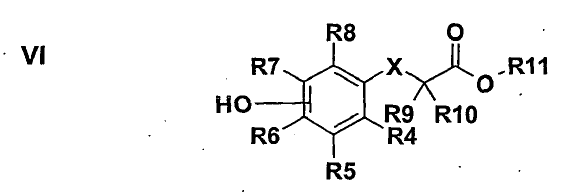 où **X, R₄, R₅, R₆, R₇, R₈, R₉, R₁₀ et R₁₁** sont tels que décrits précédemment dans la formule **I**. Cette réaction peut être effectuée dans des conditions telles que celles de la réaction de Mitsunobu en présence de triphénylphosphine et de diéthylazodicarboxylate dans le THF.

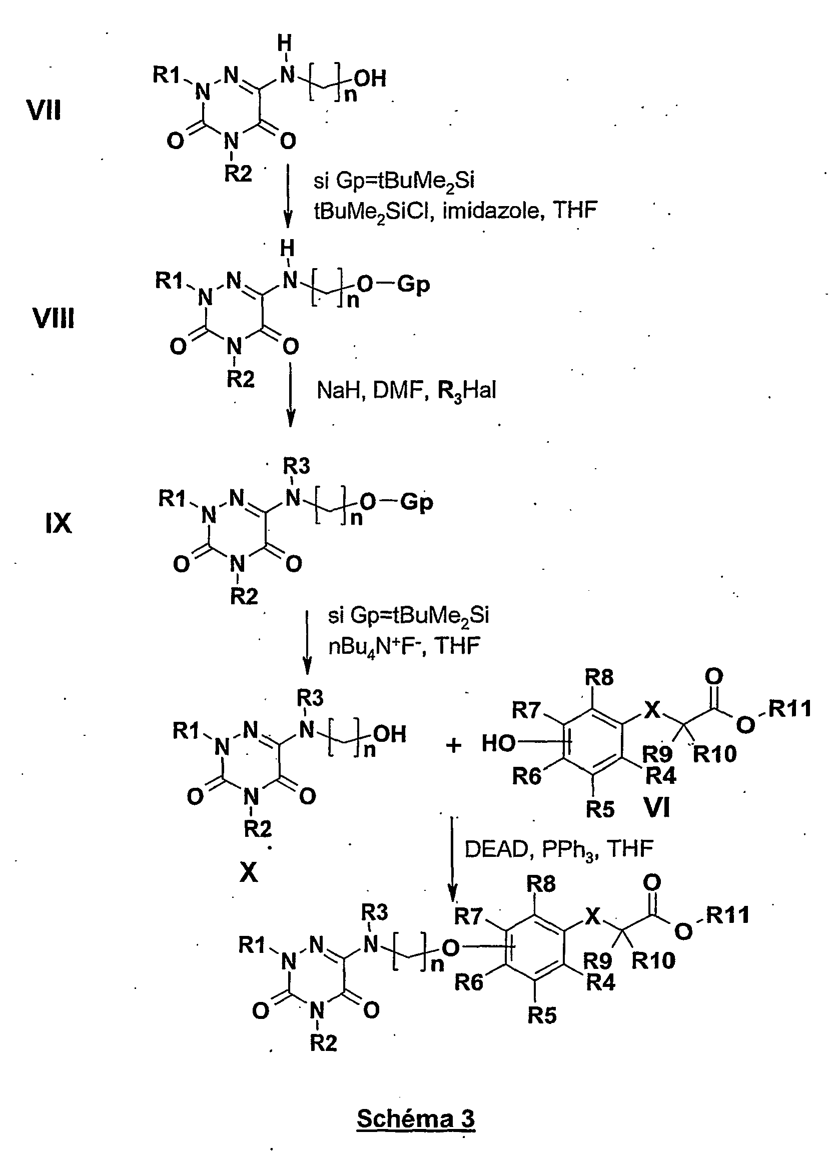

### Méthode 4

Cette méthode s'applique lorsque Y=N, **Z**=C et elle est caractérisée (schéma 4) en ce que :
1) l'on condense un dérivé de formule générale **II** 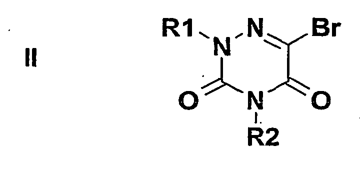 dans lequel **R₁** et **R₂** représentent les groupements tels que décrits précédemment dans la formule I avec un dérivé de formule générale XI 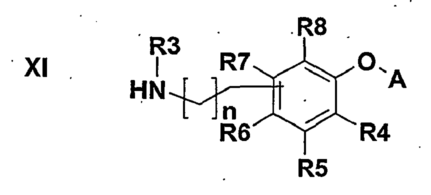 dans lequel **n, R₃ R₄, R₅, R₆, R₇,** et **R₈** sont tels que décrit précédemment dans la formule **I** et **A** peut être l'hydrogène ou un groupement méthyle. Cette réaction peut être effectuée en présence d'une base telle que la triéthylamine dans le nbutanol.
2) après déméthylation (si **A**=Me, dans des conditions telles que BBr₃ dans le dichlorométhane), on alkyle la fonction phénol du dérivé **XII** 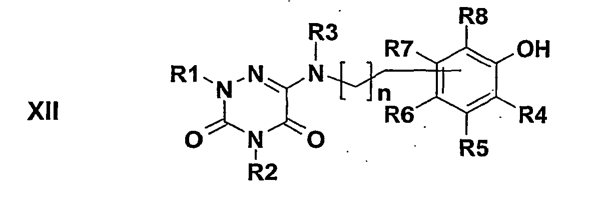 par un dérivé halogéné de formule générale **XIII** (utilisé comme solvant en présence d'une base telle que le carbonate de potassium) 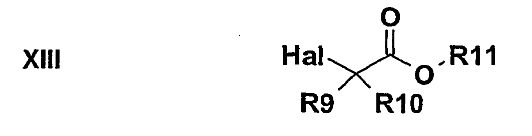 pour lequel le groupement **Hal** représente un halogène tel que Cl, Br ou I, et **R₉, R₁₀** et **R₁₁** sont tels que décrits précédemment dans la formule générale **I**.

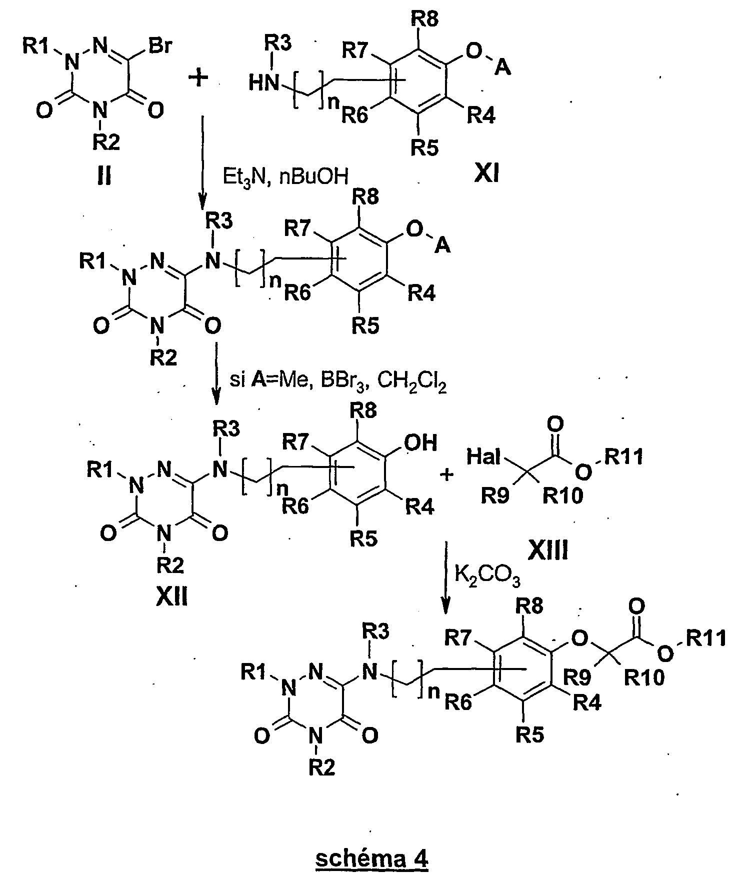

### Méthode 5

Cette méthode s'applique lorsque **Y**=N, **Z**=C et elle est caractérisée (schéma 5) en ce que :
1) l'on alkyle un dérivé de formule générale **XIV** 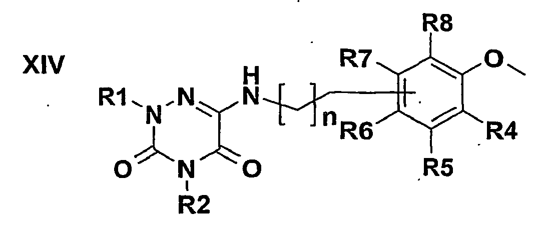 dans lequel **R₁, R₂, R₄, R₅, R₆, R₇, R₈** et n sont tels que décrits précédemment dans la formule **I** avec un dérivé de formule **R₃**Hal dans lequel le groupement **Hal** représente un halogène tel que CI, Br ou **I** et **R₃** est tel que décrit précédemment dans la formule **I,** dans des conditions opératoires telles que NaH ou tBuOK dans le DMF.
2) après déméthylation dans des conditions telles que BBr₃ dans le dichlorométhane, on alkyle la fonction phénol du dérivé **XII** ainsi obtenu 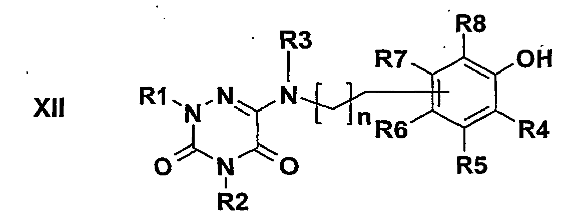 par un dérivé halogéné de formule générale **XIII** (utilisé comme solvant en présence d'une base telle que le carbonate dé potassium) 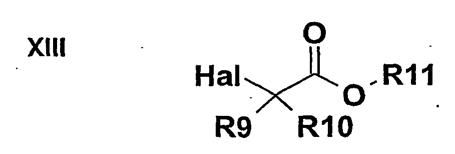 pour lequel le groupement **Hal** représente un halogène tel que Cl, Br ou **I**, et **R₉**, **R₁₀** et **R₁₁** sont tels que décrits précédemment dans la formule générale **I**.

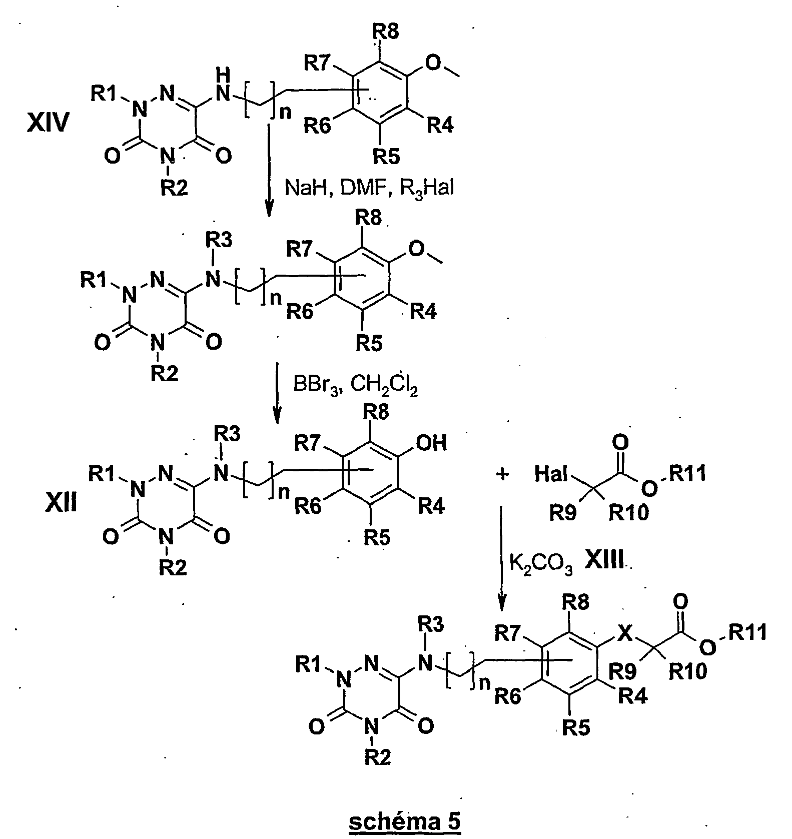

### Méthode 6

Cette méthode est caractérisée (schéma 6) en ce que :
1) l'on place un dérivé de formule générale **I** 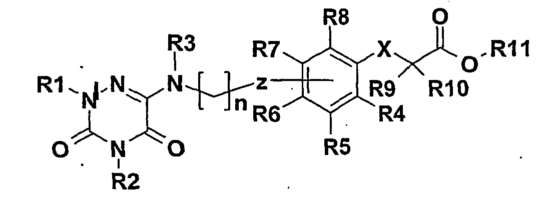 dans lequel R₁=(CH₂)₂CN et **R₂, R₃, n, Z, X, R₄, R₅, R₆, R₇, R₈, R₉, R₁₀** et **R₁₁** sont tels que décrits précédemment dans la formule **I** ou **R₂**=(CH₂)₂CN et **R₁, R₃, n, Z, X, R₄, R₅, R₆, R₇, R₈, R₉, R₁₀** et **R₁₁** sont tels que décrits précédemment dans la formule **I** dans des conditions opératoires telles que en présence d'une base NaH dans le DMF.
2) on alkyle ensuit l'azote de la triazine du dérivé **XIVa** ou **XIVb** ainsi obtenu 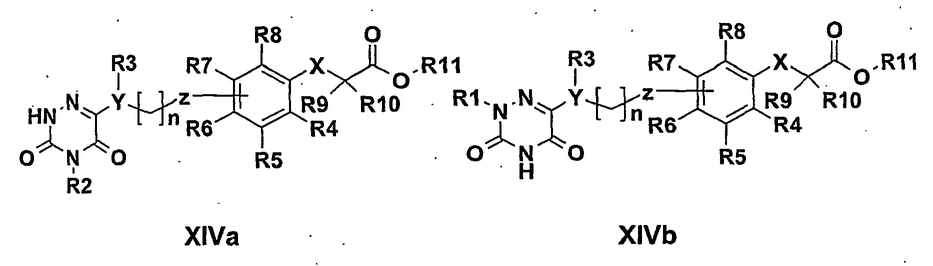 par un dérivé halogéné de formule générale R₁Hal dans le cas de l'intermédiaire **XIVa** et de formule générale **R₂Hal** dans le cas de l'intermédiaire **XIVb** pour lequel le groupement **Hal** représente un halogène tel que Cl, Br ou **I** et **R₁** et **R₂** sont tels que décrits précédemment dans la formule **I,** dans des conditions opératoires telles que NaH ou tBuOK dans le DMF.

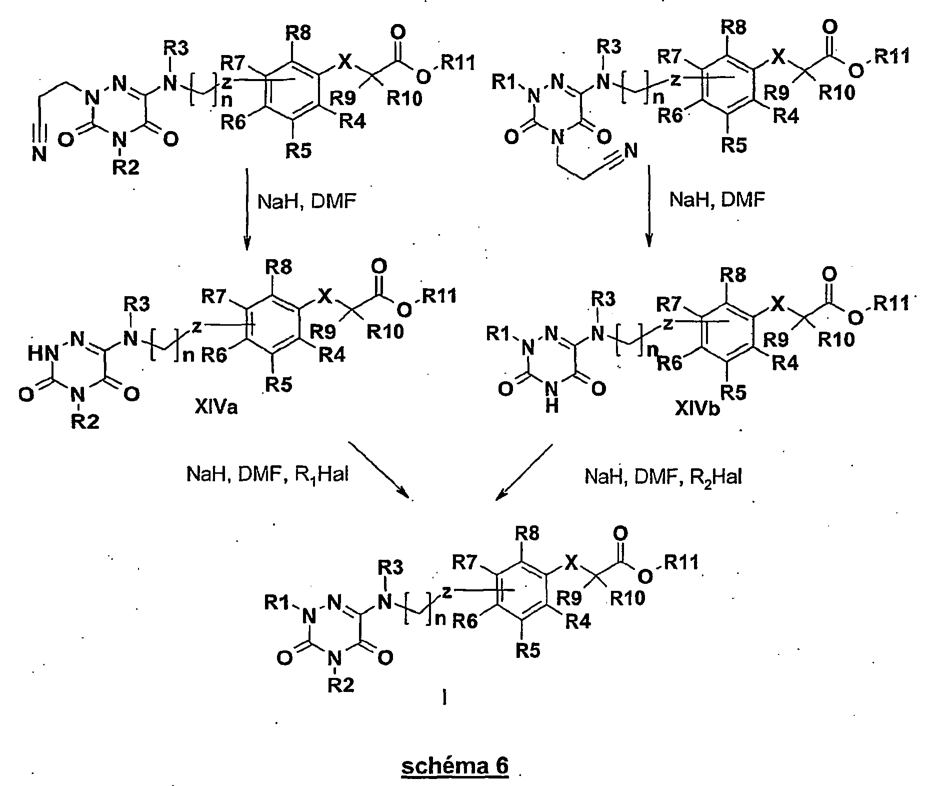

Les composés intermédiaires et finaux peuvent être, si on le désire, purifiés suivant une ou plusieurs méthodes de purification choisies parmi, l'extraction, la filtration, la chromatographie sur gel de silice, l'HPLC préparative sur phase normale ou inverse, la cristallisation.

Les matières premières utilisées dans les procédés décrits précédemment sont commerciales ou aisément accessibles à l'homme de métier selon des procédés décrits dans la littérature.

Les exemples suivants illustrent l'invention sans en limiter la portée.

Les analyses élémentaires et les spectres IR et RMN confirment les structures des composés.

### INTERMEDIAIRES

### Intermédiaires 1 :

### a) 6-Bromo-2H-[1,2,4]triazine-3,5-dione (1a)

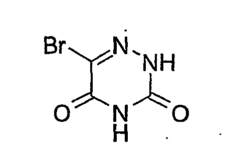

La 2H-[1,2,4]triazin-3,5-dione (50 g, 442 mmol) est placée en présence de 60 ml de brome dans 800 ml d'eau à 60°C pendant 10 h. Le milieu réactionnel est ensuite coulé lentement sur une solution d'ammoniaque jusqu'à pH=5. Il est ensuite extrait à l'acétate d'éthyle et les phases organiques sont séchées sur MgSO₄. Après filtration et concentration à sec, **1a** est isolé sous forme de solide blanc (79,2 g, rendement=93%). CCM gel de silice 60 F 254 Merck, CH₂Cl₂-MeOH :90-10, Rf = 0,32.

### b) 6-Bromo-2,4-diméthyl-2H-[1,2,4]triazine-3,5-dione (1b)

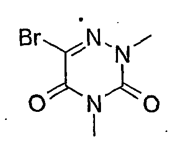

11,8g (295mmol) de NaH (60% dans la paraffine) sont placés en suspension à 0°C dans 250ml de DMF sous azote. 25,80g (135mmol) d'intermédiaire **1a** dilués dans 150ml de DMF sont coulés goutte à goutte. Cette solution est ensuite placée à température ambiante, puis 18,4mil (296mmol) d'iodure de méthyle sont coulés goutte à goutte. Après une nuit d'agitation et concentration à sec du milieu réactionnel, le résidu obtenu est repris par l'eau et extrait à l'acétate d'éthyle. Les phases organique sont lavées à la saumure, séchées sur sulfate de magnésium puis concentrées à sec. Le résidu obtenu, repris par l'éther, cristallise et une première fraction de cristaux est isolée. Le filtrat est concentré à sec puis purifié par chromatographie flash sur silice (heptane-AcOEt :50-50). 24g d'intermédiaire **1b** sont ainsi isolés (rendement 81 %). CCM gel de silice 60 F 254 Merck, CH₂Cl₂-AcOEt: 80-20, Rf = 0,59.

### c) Intermédiaires (1c)-(1g)

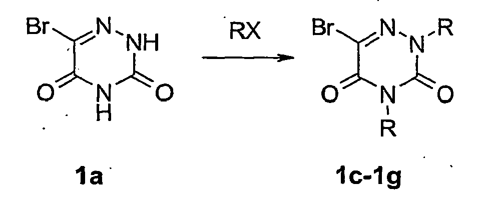

La synthèse des intermédiaires **1c-1g** est réalisée à partir de **1a** suivant le mode opératoire décrit pour la synthèse de **1 b** en utilisant divers agents d'alkylation **RX.**

**Tableau 1 : intermédiaires 1c-1g**

| **RX** | Rdt | CCM | Etat | **Intermédiaires 1c-1g** |
|---|---|---|---|---|
| 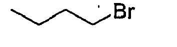 | 80% | EP-AcOEt :80-20 Rf=0,42 | huile | **1** c:6-Bromo-2,4-dibutyl-2H-[1,2,4]triazine-3,5-dione |
| 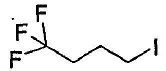 | 95% | EP-AcOEt :80-20 Rf=0,75 | solide | **1d:** 6-Bromo-2,4-bis-(4,4,4-trifluoro-butyl)-2H-[1,2,4]-triazine-3,5-dione |
| 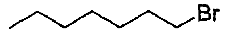 | 95% | EP-AcOEt :90-10 Rf=0,81 | huile | **1e**:6-Bromo-2,4-diheptyl-2H-[1,2,4]triazine-3,5-dione |
| 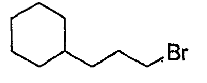 | 92% | EP-AcOEt :90-10 Rf=0,82 | huile | **1f**:6-Bromo-2,4-bis-(3-cyclo-hexyl-propyl)-2H-[1,2,4] triazine-3,5-dione |
| 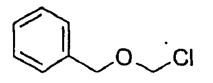 | 98% | EP-AcOEt :70-30 Rf=0,62 | huile | **1g**:2,4-Bis-benzyloxyméthyl-6-bromo-2H-[1,2,4] triazine-3,5-dione |

| | | | | |
|---|---|---|---|---|
| CCM : gel de silice 60 F 254 Merck, EP=éther de pétrole | | | | |

### Intermédiaires 2 :

### a) 6-Bromo-4-méthyl-2H-[1,2,4]triazine-3,5-dione (2a)

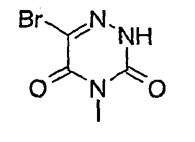

20,3 g (105,7 mmol) de triazine **1a** sont placés dans 150 ml d'anhydride acétique à reflux pendant 4,5 h. Après concentration à sec du milieu réactionnel, un précipité est isolé puis recristallisé dans l'éther : on isole 24,3 g de cristaux (rendement=98%). 4,5 g (114,2 mmol) de NaH (60% dans la paraffine) sont placés dans 50 ml de DMF sous azote. Une solution de 24,3 g (103,8 mmol) de cristaux isolés précédemment, dans 150 ml de DMF est coulée goutte à goutte. Le milieu réactionnel est agité 45mn à température ambiante puis 7 ml (114,2 mmol) d'iodure de méthyle sont additionnés, ensuite l'agitation est poursuivie 21 h à température ambiante. Après concentration à sec, le résidu obtenu est repris par H₂O et extrait à l'acétate d'éthyle. Après séchage sur MgSO₄, les phases organiques sont évaporées et l'huile claire obtenue est purifiée par chromatographie flash sur silice (CH₂Cl₂-AcOEt :90-10). On isole 22,9 g de cristaux (rendement=89%) qui sont placés dans 300 ml d'éthanol en présence de 0,6 g d'acide paratoluène sulfonique. Ce mélange est chauffé à reflux pendant 4,5h puis concentré à sec. Le résidu est repris par H₂O puis extrait à l'acétate d'éthyle. Après séchage et évaporation des phases organiques, on isole 17 g d'intermédiaire **2a** sous forme de solide (rendement=89%). CCM gel de silice 60 F 254 Merck, CH₂Cl₂-AcOEt : 90-10, Rf = 0,29.

### b) Intermédiaires (2b)-(2f)

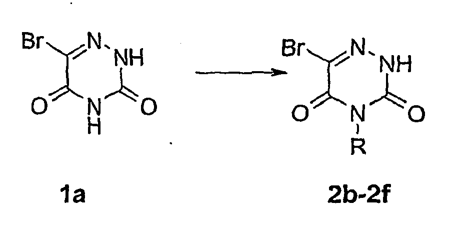

La synthèse des intermédiaires **2b-2f** est réalisée à partir de **1a** suivant le mode opératoire décrit pour la synthèse de **2a** en utilisant divers agents d'alkylation **RX.**

**Tableau 2: intermédiaires 2b-2f**

| RX | Rdt total | CCM | Etat | **Intermédiaires 2b-2f** |
|---|---|---|---|---|
| 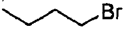 | 73% | EP-AcOEt:80-20 Rf=0,28 | solide | **2b:** 6-Bromo-4-butyl-2H-[1,2,4] triazine-3,5-dione |
| 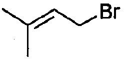 | 60% | EP-AcOEt:80-20 Rf=0,26 | solide | **2c:** 6-Bromo-4-(3-méthyl-but-2-ènyl)-2H-[1,2,4]triazine-3,5-dione |
| 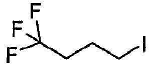 | 76% | CH₂Cl₂-AcOEt:90-10 Rf=0,45 | solide | **2d:** 6-Brbmo-4-(4,4,4-trifluoro-butyl)-2H-[1,2,4] triazine-3,5-dione |
| 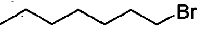 | 84% | EP-AcOEt:70-30 Rf=0,73 | solide | **2e:** 6-Bromo-4-heptyl-2H-[1,-2,4]triazine-3,5-dione |
| 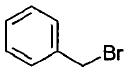 | 82% | EP-AcOEt:70-30 Rf=0,25 | solide | **2f:** 4-Benzyl-6-bromo-2H-[1,2,4]triazine-3,5-dione |

| | | | | |
|---|---|---|---|---|
| CCM : gel de silice 60 F 254 Merck, EP=éther de pétrole | | | | |

### Intermédiaires 3 :

### a) 3-(6-Bromo-4-méthyl-3,5-dioxo-4,5-dihydro-3H-[1,2,4]triazin-2-yl)-propionitrile (3a)

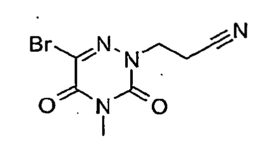

2,4 g (11,6 mmol) de triazine **2a** et 7ml (106mmol) d'acrylonitrile sont placés dans 24ml d'une solution de pyridine et d'eau (1/1) à reflux pendant 3 h. Après concentration, le milieu réactionnel est extrait par l'AcOEt puis après séchage sur MgSO₄, les phases organiques sont concentrées à sec. On isole 2,8 g de solide **3a** qui sont lavés à l'éther (rendement=93%). CCM gel de silice 60 F 254 Merck, éther de pétrole-AcOEt :70-30, Rf=0,18.

### b) Intermédiaires 3b et 3c

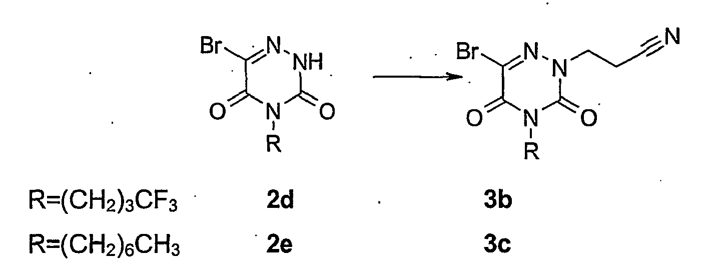

La synthèse des intermédiaires **3b** et **3c** est réalisée respectivement à partir des intermédiaires **2d** et **2e** suivant le mode opératoire décrit pour la synthèse de **3a**.

**Tableau 3: intermédiaires 3b et 3c**

| **Synthons de départ** | Rdt | CCM | Etat | **Intermédiaires 3b-3c** |
|---|---|---|---|---|
| **2d** | 91 % | EP-AcOEt:70-30 Rf=0,34 | solide | **3b:** 3-[6-Bromo-3,5-dioxo-4-(4,4,4-trifluoro-butyl)-4,5-dihydro-3H-[1,2,4]triazin-2-yl]-propionitrile |
| **2e** | 95% | CH₂Cl₂-AcOEt:70-30 Rf=0,51 | solide | **3c**: 3-(6-Bromo-4-heptyl-3,5-dioxo-4,5-dihydro-3H-[1,2,4] triazin-2-yl)-propionitrile |

| | | | | |
|---|---|---|---|---|
| CCM : gel de silice 60 F 254 Merck, EP=éther de pétrole | | | | |

### Intermédiaires 4 :

### a) 6-Bromo-4-méthyl-2-(4,4,4-trifluoro-butyl)-2H-[1,2,4]triazine-3,5-dione (4a)

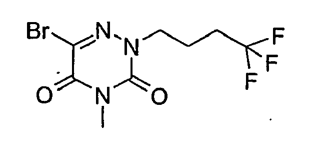

0,85 g (21,3 mmol) de NaH (60% dans la paraffine) sont placés dans 10 ml de DMF sous azote. Une solution de 4 g (19,4 mmol) d'intermédiaire **2a** dans 40 ml de DMF est coulée goutte à goutte. Le milieu réactionnel est agité 1h à température ambiante puis 5g (21,3 mmol) de 1,1,1-trifluoro-4-iodo-butane sont additionnés goutte à goutte, ensuite l'agitation est poursuivie 3h à température ambiante. Après concentration à sec, le résidu obtenu est repris par H₂O et extrait à l'acétate d'éthyle. Après séchage sur MgSO₄, les phases organiques sont évaporées et l'huile obtenue est purifiée par chromatographie flash sur silice (éther de pétrole-AcOEt :80-20). On isole 5,3 g de cristaux correspondant au composé **4a** (rendement=87%). CCM gel de silice 60 F 254 Merck, éther de pétrole-AcOEt :70-30, Rf=0,58.

### b) 6-Bromo-2-heptyl-4-méthyl-2H-[1,2,4]triazine-3,5-dione (4b)

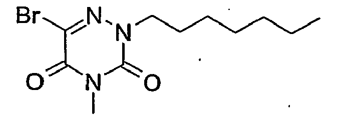

La synthèse de l'intermédiaire **4b** est réalisée à partir de **2a** suivant le mode opératoire décrit pour la synthèse de **4a** en utilisant le 1-bromoheptane pour l'étape d'alkylation (rendement=91 %). CCM gel de silice 60 F 254 Merck, éther de pétrole-AcOEt :70-30, Rf=0,76.

### Intermédiaires 5 :

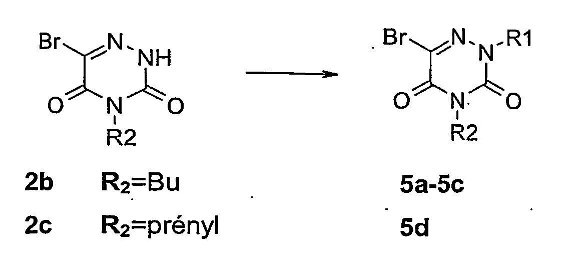

La synthèse des intermédiaires **5a-5d** est réalisée à partir des intermédiaires **2b** et 2c suivant le mode opératoire décrit pour la synthèse de **4a** en utilisant divers dérivés halogénés **R₁X.**

**Tableau 4: intermédiaires 5a-5d**

| **R₁X** | Rdt | CCM | Etat | **Intermédiaires 5a-5c** |
|---|---|---|---|---|
| -I | 64% | CH₂Cl₂- AcOEt : 95-5 Rf=0,75 | solide | **5a**: 6-Bromo-4-butyl-2-méthyl-2H-[1,2,4] triazine-3,5-dione |
| 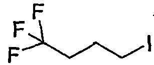 | 95% | EP-AcOEt:80-20 Rf=0,61 | huile | **5b**: 6-Bromo-4-butyl-2-(4,4,4-trifluoro-butyl)-2H-[1,2,4]triazine-3,5-dione |
| 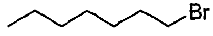 | 72% | EP-AcOEt:80-20 Rf=0,75 | huile | **5c**: 6-Bromo-4-butyl-2-heptyl-2H-[1,2,4] triazine-3,5-dione |
| 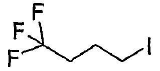 | 90% | EP-AcOEt:80-20 Rf=0,58 | huile | 5d: 6-Bromo-4-(3-méthyl-but-2-ènyl)-2-(4,4,4-trifluoro-butyl)-2H-[1,2,4]triazine-3,5-dione |

| | | | | |
|---|---|---|---|---|
| CCM : gel de silice 60 F 254 Merck, EP=éther de pétrole | | | | |

### Intermédiaires 6 :

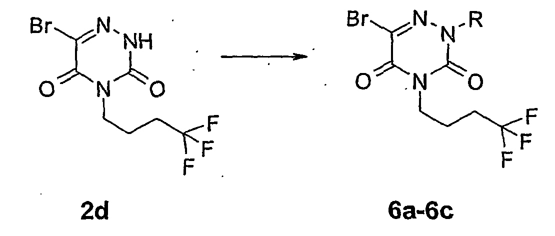

La synthèse des intermédiaires **6a-6c** est réalisée à partir de l'intermédiaire 2d suivant le mode opératoire décrit pour la synthèse de **4a** en utilisant divers dérivés halogénés **RX.**

**Tableau 5: intermédiaires 6a-6c**

| **RX** | Rdt | CCM | Etat | **Intermédiaires 6a-6b** |
|---|---|---|---|---|
| -I | 91 % | EP-AcOEt:70-30 Rf=0,61 | Solide | **6a:** 6-Bromo-2-méthyl-4-(4,4,4-trifluoro-butyl)-2H-[1,2,4]triazine-3,5-dione |
| 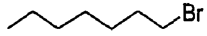 | 88% | EP-AcOEt:60-40 Rf=0,53 | Huile | **6b**: 6-Bromo-2-heptyl-4-(4,4,4-trifluoro-butyl)-2H-[1,2,4]triazine-3,5-dione |
| 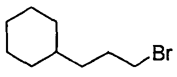 | 80% | EP-AcOEt:60-40 Rf=0,43 | Huile | **6c**:6-Bromo-2-(3-cyclohexyl-propyl)-4-(4,4,4-trifluoro-butyl)-2H-[1,2,4]triazine-3,5-dione |

| | | | | |
|---|---|---|---|---|
| CCM : gel de silice 60 F 254 Merck, EP=éther de pétrole | | | | |

### Intermédiaires 7 :

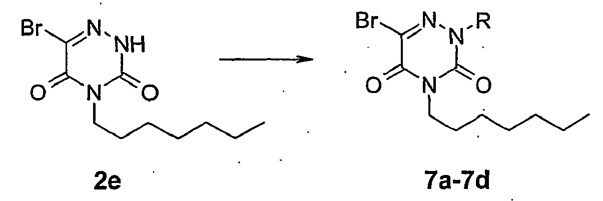

La synthèse des intermédiaires **7a-7d** est réalisée à partir de l'intermédiaire **2e** suivant le mode opératoire décrit pour la synthèse de **4a** en utilisant divers dérivés halogénés **RX.**

**Tableau 6: intermédiaires 7a-7d**

| **RX** | Rdt | CCM | Etat | **Intermédiaires 7a-7d** |
|---|---|---|---|---|
| -I | 92% | CH₂Cl₂-MeOH: 95-5 Rf=0,65 | huile | **7a:** 6-Bromo-4-heptyl-2-méthyl-2H-[1,2,4]triazine-3,5-dione |
| 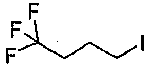 | 98% | EP-AcOEt:70-30 Rf=0,81 | huile | **7b**: 6-Bromo-4-heptyl-2-(4,4,4-trifluoro-butyl)-2H-[1,2,4]triazine-3,5-dione |
| 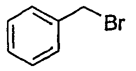 | 80% | EP-AcOEt:80-20 Rf=0,60 | huile | **7c:** 2-Benzyl-6-bromo-4-heptyl-2H-[1,2,4]triazine-3,5-dione |
| 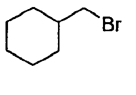 | 78% | EP-AcOEt:80-20 Rf=0,59 | huile | 7d: 6-Bromo-2-cyclohexylméthyl-4-heptyl-2H-[1,2,4]triazine-3,5-dione |

| | | | | |
|---|---|---|---|---|
| CCM : gel de silice 60 F 254 Merck, EP=éther de pétrole | | | | |

### Intermédiaires 8 :

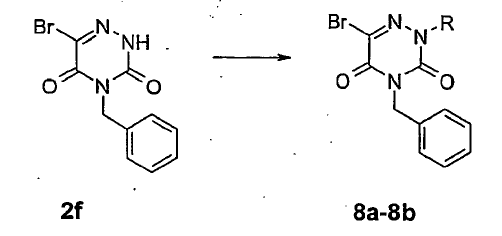

La synthèse des intermédiaires **8a-8b** est réalisée à partir de l'intermédiaire **2f** suivant le mode opératoire décrit pour la synthèse de **4a** en utilisant divers dérivés halogénés **RX.**

**Tableau 7: intermédiaires 8a-8b**

| **RX** | Rdt | CCM | Etat | **Intermédiaires 8a-8b** |
|---|---|---|---|---|
| 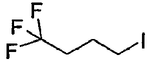 | 93% | EP-AcOEt:80-20 Rf=0,46 | huile | **8a**:4-Benzyl-6-bromo-2-(4,4,4-trifluoro-butyl)-2H-[1,2,4]triazine-3,5-dione |
| 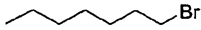 | 98% | EP-AcOEt:80-20 Rf=0,66 | huile | **8b:** 4-Benzyl-6-bromo-2-heptyl-2H-[1,2,4]triazine-3,5-dione |

| | | | | |
|---|---|---|---|---|
| CCM : gel de silice 60 F 254 Merck, EP=éther de pétrole | | | | |

### Intermédiaires 9 :

### a) 6-(2-Hydroxy-éthylamino)-2,4-bis-(4,4,4-trifluoro-butyl)-2H-[1,2,4]triazine-3,5-dione (9a)

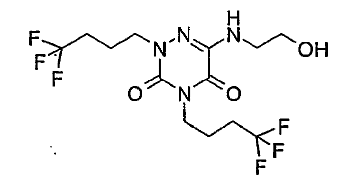

3 g (7,3 mmol) de triazine **1d** sont placés dans 1,3 ml d'éthanolamine à 130°C pendant 5 h. Après refroidissement, 50ml d'eau sont ajoutés au milieu réactionnel qui est ensuite extrait par l'AcOEt. Après séchage sur MgSO₄, les phases organiques sont concentrées à sec et le résidu obtenu est purifié par chromatographie flash sur silice (éther de pétrole-AcOEt :70-30). 1,9g d'huile correspondant à l'intermédiaire 9a sont ainsi isolés (rendement 66%). CCM gel de silice 60 F 254 Merck, éther de pétrole-AcOEt :70-30, Rf=0,28.

### b) Intermédiaires 9b-9I

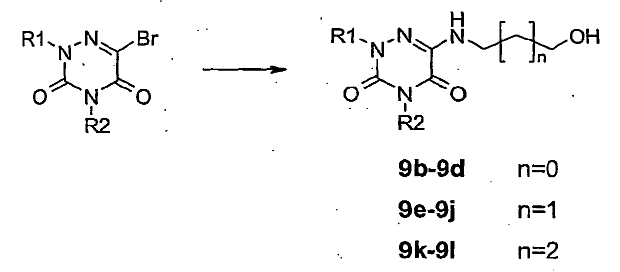

La synthèse des intermédiaires **9b-91** est réalisée à partir des composés de départ listés dans le tableau 8 suivant le mode opératoire décrit pour la synthèse de **9a.**

**Tableau 8: intermédiaires 9b-9I**

| **Synthons de départ** | **Amino alcool** | Rdt | CCM | Etat | **Intermédiaires 9b-9k** |
|---|---|---|---|---|---|
| **4b** | Ethanol-amine | 77% | CH₂Cl₂-AcOEt:60-40 Rf=0,28 | solide | **9b**:2-Heptyl-6-(2-hydroxy-éthylamino)-4-méthyl-2H-[1,2,4]triazine-3,5-dione |
| **le** | Ethanol-amine | 67% | EP-AcOEt:70-30 Rf=0,56, | huile | **9c**: 2,4-Diheptyl-6-(2-hydroxy éthylamino)-2H-[1,2,4] triazine-3,5-dione |
| **1f** | Ethanol-amine | 56% | EP-AcOEt:80-20 Rf=0,18 | huile | **9d**:2,4-Bis-(3-cyclohexyl-propyl)-6-(2-hydroxy-éthylamino)-2H-[1,2,4]triazine-3,5-dione |
| **1b** | Amino-propanol | 18% | AcOEt Rf=0,42 | huile | **9e**:6-(3-Hydroxy-propyl-amino)-2,4-diméthyl-2H-[1,2,4] triazine-3,5-dione |
| **4a** | Amino-propanol | 44% | EP-AcOEt:70-30 Rf=0,13 | solide | **9f:** 6-(3-Hydroxy-propylamino)-4-méthyl-2-(4,4,4-trifluoro-butyl)-2H-[1,2,4]triazine-3,5-dione |
| **4b** | Amino-propanol | 67% | CH₂Cl₂-AcOEt:60-40 Rf=0,32 | solide | **9g:** 2-Heptyl-6-(3-hydroxy-propylamino)-4-méthyl-2H-[1,2,4]triazine-3,5-dione |
| **6a** | Amino-propanol | 45% | EP-AcOEt:80-20 Rf=0,05 | solide | **9h:** 6-(3-Hydroxy-propyl-amino)-2-méthyl-4-(4,4,4-trifluoro-butyl)-2H-[1,2,4]triazine-3,5-dione |
| **7a** | Amino-propanol | 34% | CH₂Cl₂-MeOH:90-10 Rf=0,47 | solide | 9i:4-Heptyl-6-(3-hydroxy-propylamino)-2-méthyl-2H-[1,2,4]triazine-3,5-dione |
| **7b** | Amino-propanol | 64% | EP-AcOEt:70-30 Rf=0,30 | solide | **9j**: 4-Heptyl-6-(3-hydroxy-propylamino)-2-(4,4;4-trifluoro-butyl)-2H-[1,2,4]triazine-3,5-dione |
| **1b** | Amino-butanol | 50% | AcOEt Rf=0,35 | solide | **9k :** 6-(4-Hydroxy-butyl-amino)-2,4-diméthyl-2H-. [1,2,4]triazine-3,5-dione |
| **7a** | Amino-butanol | 21% | CH₂Cl₂-MeOH:90-10 Rf=0,45 | solide | **9I:** 4-Heptyl-6-(4-hydroxy-butylamino)-2-méthyl-2H- [1,2,4]triazine-3,5-dione |

| | | | | | |
|---|---|---|---|---|---|
| CCM : gel de silice 60 F 254 Merck, EP=éther de pétrole | | | | | |

### c) 2-Heptyl-6-[(3-hydroxy-propyl)-(4,4,4-trifluoro-butyl)-amino] 4-méthyl-2H-[1,2,4]triazine-3,5-dione (9m)

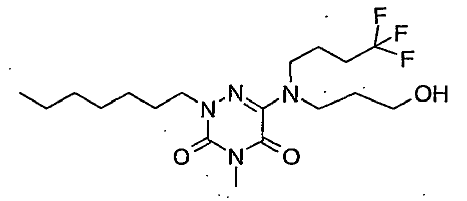

7,3 g (24,2 mmol) de triazine **9g** sont placés en présence tert-butyl-chloro-diméthyl-silane (4g, 26,5mmol) dans 50 ml de dichlorométhane à température ambiante durant une nuit. Le milieu réactionnel est ensuite lavé avec de l'eau puis de la saumure. Après séchage sur MgSO₄, la phase organique est concentrée à sec et le résidu obtenu est purifié par chromatographie flash sur silice (CH₂Cl₂-AcOEt :95-5). 10g d'huile sont isolés (rendement quantitatif). 4,1g (10mmol) de ce composé sont placés dans 40ml de DMF à 0°C sous azote puis, 0,4g (10mmol) de NaH (60% dans la paraffine) sont additionnés par fractions et ce mélange est agité 10mn. 2,4g (10mmol) de 1,1,1-trifluoro-4-iodo-butane sont ajoutés et la solution est agitée à température ambiante pendant 3h. 0,5 équivalent de NaH ainsi que de 1,1,1-trifluoro-4-iodo-butane sont à nouveau ajoutés et l'agitation est poursuivie pendant 2h. Après concentration à sec, le résidu est repris par H₂O puis extrait avec l'AcOEt. Après séchage sur MgSO₄, les phases organiques sont concentrées à sec et l'huile obtenue est purifiée par chromatographie flash sur silice (éther de pétrole-AcOEt :90-10). On isole 2g de composé (rendement=40%) qui est ensuite dilué dans 30ml de THF et 7,4ml d'une solution de fluorure de tétrabutylammonium (1M dans le THF) est coulée goutte à goutte. Ce mélange est agité 2h à température ambiante puis, 50ml d'eau sont additionnés et le milieu est extrait à l'AcOEt. Après séchage sur MgSO₄, les phases organiques sont concentrées à sec et l'huile obtenue est purifiée par chromatographie flash sur silice (éther de pétrole-AcOEt :70-30). 1,5g de triazine 9m sont ainsi isolés sous forme d'huile (rendement quantitatif). CCM gel de silice 60 F 254 Merck, éther de pétrole-AcOEt :90-10, Rf=0,38.

### d) Intermédiaires 9n-9o

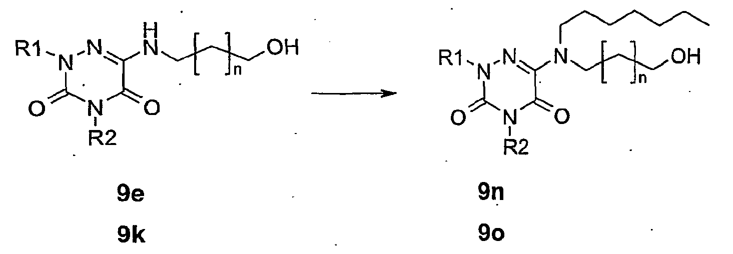

La synthèse des intermédiaires **9n** et **9o** est réalisée respectivement à partir des intermédiaires **9e** et **9k** suivant le mode opératoire décrit pour la synthèse de 9m en utilisant le bromoheptane.

**Tableau 9: intermédiaires 9n-9o**

| **Synthons de départ** | Rdt | CCM | Etat | **Intermédiaires 9n-9o** |
|---|---|---|---|---|
| **9e** | 59% | heptane-AcOEt:50-50 Rf=0,16 | huile | **9n :** 6-[Heptyl-(3-hydroxy-propyl)-amino]-2,4-diméthyl-2H-[1,2,4]triazine-3, 5-dione |
| **9k** | 59% | heptane-AcOEt:50-50 Rf=0,24 | huile | **9o:** 6-[Heptyl-(4-hydroxy-butyl)-amino]- 2,4-diméthyl-2H-[1,2,4]triazine-3,5-dione |

| | | | | |
|---|---|---|---|---|
| CCM : gel de silice 60 F 254 Merck, EP=éther de pétrole | | | | |

### e) 2-Heptyl-6-(2-hydroxy-éthoxy)-4-méthyl-2H-[1,2,4]triazine-3,5-dione (9p)

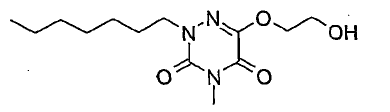

1,5 g (4,9 mmol) de triazine **4b** et 0,8g (5,8mmol) de K₂CO₃ sont placés dans 1,5 ml d'éthylèneglycol à 130°C pendant 0,5 h. 50ml d'eau sont ajoutés au milieu réactionnel qui est ensuite extrait par l'AcOEt . Après séchage sur MgSO₄, les phases organiques sont concentrées à sec et le résidu obtenu est purifié par chromatographie flash sur silice (CH₂Cl₂-AcOEt :70-30). 0,5g d'huile correspondant à l'intermédiaire **9p** est ainsi isolé (rendement 39%). CCM gel de silice 60 F 254 Merck, heptane-AcOEt :50-50, Rf=0,08.

### f) Intermédiaires 9g-9s

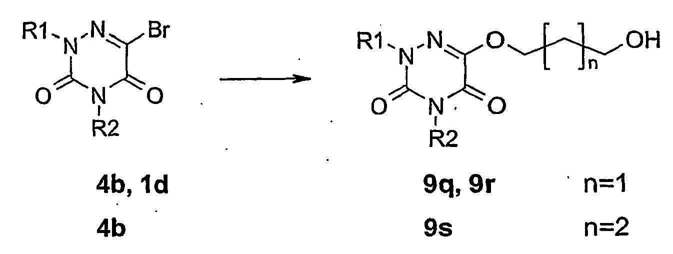

La synthèse des intermédiaires **9q-9s** est réalisée à partir des composés de départ **4b** et **1d** suivant le mode opératoire décrit pour la synthèse de **9p** en utilisant différents diols.

**Tableau 10: intermédiaires 9q-9s**

| **Synthons de départ** | **diol** | Rdt | CCM | Etat | **Intermédiaires 9q-9s** |
|---|---|---|---|---|---|
| **4b** | 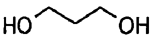 | 78% | AcOEt Rf=0,41 | huile | **9q:** 2-Heptyl-6-(3-hydroxy-propoxy)-4-méthyl-2H-[1,2,4] triazine-3,5-dione |
| **1d** | 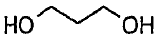 | 67% | AcOEt Rf=0,44 | huile | **9r:** 6-(3-Hydroxy-propoxy)-2,4-bis-(4,4,4-trifluoro-butyl)-2H-[1,2,4]triazine-3,5-dione |
| **4b** | 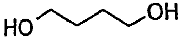 | 70% | AcOEt Rf=0,31 | huile | **9s:** 2-Heptyl-6-(4-hydroxy-butoxy)-4-méthyl-2H-[1,2,4]triazine-3,5-dione |

| | | | | | |
|---|---|---|---|---|---|
| CCM : gel de silice 60 F 254 Merck | | | | | |

### Intermédiaires 10 :

### a) 3-(2-Hydroxy-éthyl)-phénol (10a)

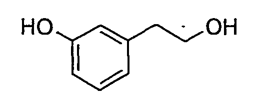

11,3 g d'acide (3-hydroxy-phényl)-acétique (74,2 mmol) sont placés dans 100 ml de THF à 0°C sous azote. 100ml d'une solution de LiAIH₄ (1M dans le THF) sont coulés goutte à goutte à cette température. Le mélange est ensuite placé à 60°C pendant 2h. Il est ensuite neutralisé lentement avec une solution HCl 6N puis extrait avec l'éther diéthylique. Les phases organiques sont lavées à l'eau, séchées sur MgSO₄, puis concentrées à sec. Le résidu obtenu est purifié par chromatographie flash sur silice (CH₂Cl₂-AcOEt :70-30). 9g d'huile correspondant à l'intermédiaire **10a** sont ainsi isolés (rendement 88%). CCM gel de silice 60 F 254 Merck, CH₂Cl₂-AcOEt :70-30, Rf=0,18.

### b) 2-(3-Hydroxy-phénoxy)-2-méthyl-propionate d'éthyle (10b)

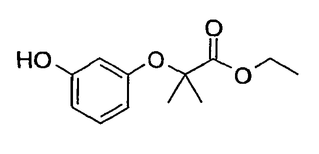

15 g de résorcinol (136 mmol) sont additionnés à 120ml d'une solution de sodium (6,3g, 274mmol) dans l'éthanol. Le mélange est placé à reflux pendant 1h puis une solution de bromoisobutyrate d'éthyle (13,2ml, 90mmol) dans 30ml d'éthanol est coulée goutte à goutte. Le chauffage est maintenu pendant 3h puis le milieu réactionnel est concentré à sec. Le résidu obtenu est repris par une solution d'eau et d'acide acétique puis extrait avec l'AcOEt. Les phases organiques sont lavées à l'eau, séchées sur MgSO₄, puis concentrées à sec. Le résidu obtenu est purifié par chromatographie flash sur silice (CH₂Cl₂-AcOEt :90-10): 14,4g d'huile correspondant à l'intermédiaire **10b** sont ainsi isolés (rendement 72%). CCM gel de silice 60 F 254 Merck, CH₂Cl₂-AcOEt :70-30, Rf=0,66.

### c) 2-(4-Hydroxy-phénoxy)-2-méthyl-propionate d'éthyle (10c)

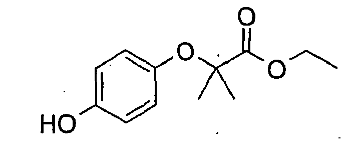

11g d'hydroquinone (100 mmol) dans 100ml de DMF sont placés à 80°C pendant 2h. Ce mélange est refroidi à température ambiante puis une solution de bromoisobutyrate d'éthyle (14,7ml, 100mmol) dans 30ml de DMF est coulée goutte à goutte. Le mélange est agité pendant 3h puis le milieu réactionnel est concentré à sec. Le résidu obtenu est repris par une solution d'HCl 1N puis extrait avec l'AcOEt. Les phases organiques sont lavées à l'eau, séchées sur MgSO₄, puis concentrées à sec. Le résidu obtenu est purifié par chromatographie flash sur silice (éther de pétrole-AcOEt :80-20). 9g d'huile correspondant à l'intermédiaire **10c** sont ainsi isolés (rendement 40%). CCM gel de silice 60 F 254 Merck, éther de pétrole-AcOEt :70-30, Rf=0,50.

### d) 2-(3-Bromo-phénoxy)-2-méthyl-propionate d'éthyle (10d)

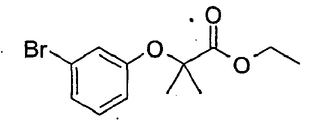

25 g (144,5 mmol) de 3-bromophénol sont placés en présence de K₂CO₃ (21 g, 152 mmol) dans 75 ml de 2-bromoisobutyrate d'éthyle et chauffés à reflux pendant 7 h. Après élimination de K₂CO₃ par filtration, le milieu réactionnel est concentré à sec. Après purification par chromatographie flash sur silice (éther de pétrole-AcOEt :90-10), on recueille 37 g d'intermédiaire 10d sous forme d'huile claire (rendement=89%). CCM gel de silice 60 F 254 Merck, éther de pétrole-AcOEt: 90-10, Rf = 0,40.

### d) Intermédiaires 10e-10j

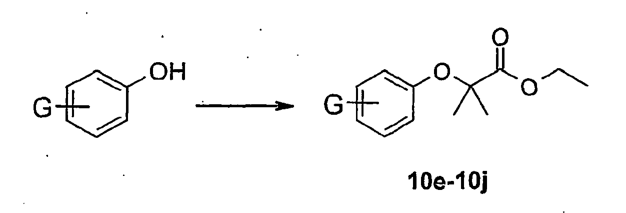

La synthèse des intermédiaires **10e-10j** est réalisée à partir de phénols diversement substitués listés dans le tableau **8** suivant le mode opératoire décrit pour la synthèse de **10d.**

**Tableau 11: intermédiaires 10e-10j**

| **Phénols de départ** | Rdt | CCM | Etat | **Intermédiaires 10e-10j** |
|---|---|---|---|---|
| 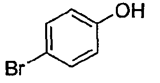 | 98% | EP-AcOEt:90-10 Rf=0,52 | huile | **10e**:2-(4-Bromo-phénoxy)-2-méthyl-propionate d'éthyle |
| 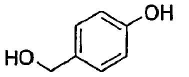 | 52% | CH₂Cl₂-AcOEt:80-20 Rf=0,36 | huile | **10f**:2-(4-Hydroxyméthyl-phénoxy)-2-méthyl-propionate d'éthyle |
| 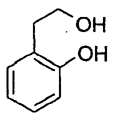 | 19% | CH₂Cl₂-AcOEt:90-10 Rf=0,50 | huile | **10g**:2-[2-(2-Hydroxy-éthyl)-phénoxy]-2-méthyl-propionate d'éthyle |
| **10a** | 9% | CH₂Cl₂-AcOEt:70-30 Rf=0,68 | huile | **10h**: 2-[3-(2-Hydroxy-éthyl)-phénoxy]-2-méthyl-propionate d'éthyle |
| 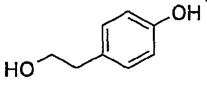 | 61 % | CH₂Cl₂-AcOEt:90-10 Rf=0,21 | huile | **10j**: 2-[4-(2-Hydroxy-éthyl)-phénoxy]-2-méthyl-propionate d'éthyle |

| | | | | |
|---|---|---|---|---|
| CCM : gel de silice 60 F 254 Merck, EP=éther de pétrole | | | | |

### Intermédiaires 11 :

### a) 2-(3-Bromo-phénylsulfanyl)-2-méthyl-propionate d'éthyle (11a)

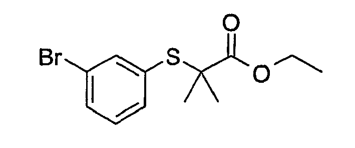

10 g (52,9 mmol) de 3-bromothiophénol sont placés en présence de 9,4 ml (63,5 mmol) de bromoisobutyrate d'éthyle et 8 g (57,9 mmol) de K₂CO₃ dans 100 ml d'EtOH. Ce mélange est agité à reflux pendant 4h puis concentré à sec. Le résidu est repris par l'eau et après extraction à l'AcOEt, puis séchage sur MgSO₄, les phases organiques sont concentrées à sec. L'huile obtenue est purifiée par chromatographie flash sur silice (éther de pétrole-AcOEt :90-10) et **11a** est isolé sous forme d'huile claire (16,8 g, rendement quantitatif). CCM gel de silice 60 F 254 Merck, éther de pétrole-AcOEt :90-10, Rf=0,72.

### c) Intermédiaires 11b-11e

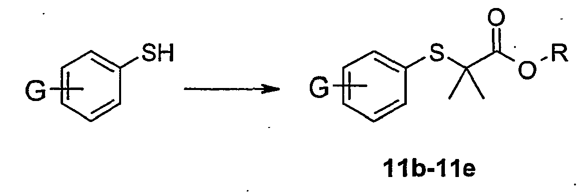

La synthèse des intermédiaires **11b-11e** est réalisée à partir de thiophénols diversement substitués (G) listés dans le tableau **12** suivant le mode opératoire décrit pour la synthèse de **11a** en utilisant le bromoisobutyrate d'éthyle ou de tert-butyle.

**Tableau 12: intermédiaires 11b-11e**

| **Thiophénols de départ** | 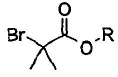 | Rdt | CCM | Etat | **Intermédiaires 11b-11e** |
|---|---|---|---|---|---|
| 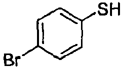 | bromoisobutyrate d'éthyle | 87% | EP-AcOEt:90-10 Rf=0,70 | huile | **11b** 2-(4-Bromo-phénylsulfanyl)-2-méthyl-propionate d'éthyle |
| 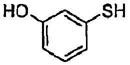 | bromoisobutyrate d'éthyle | 74% | Heptane-AcOEt: 80-20 Rf=0,50 | huile | **11c**:2-(3-Hydroxy-phénylsulfanyl)-2-méthyl-propionate d'éthyle |
| 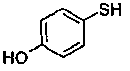 | bromoisobutyrate d'éthyle | 64% | Heptane-AcOEt: 80-20 Rf=0,20 | huile | **11d**:2-(4-Hydroxy-phénylsulfanyl)-2-méthyl-propionate d'éthyle |
| 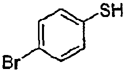 | bromoisobutyrate de tert-butyle | 98% | EP-AcOEt:90-10 Rf=0,70 | solide | **11e**:2-(4-Bromo-phényl-sulfanyl)-2-méthyl-propionate de tert-butyle |

| | | | | | |
|---|---|---|---|---|---|
| CCM : gel de silice 60 F 254 Merck, EP=éther de pétrole | | | | | |

### Intermédiaires 12 :

### a) 2-[3-(3-Hydroxy-propyl)-phénoxy]-2-méthyl-propionate d'éthyle (12a)

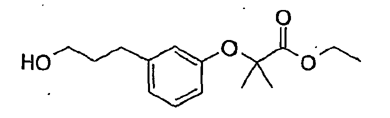

**10d** (50g, 175 mmol) est placé en présence de 2-propynol (12 ml, 210 mmol) dans 400 ml de diisopropylamine, sous azote. Pd(PPh₃)₂Cl₂ (3,5 g) et Cul (500 mg) sont ajoutés et le milieu réactionnel est agité à reflux pendant 5 h. Le précipité formé en cours de réaction est filtré sur célite et le milieu réactionnel est concentré à sec. L'huile obtenue est purifiée par chromatographie flash sur silice (éther de pétrole-AcOEt :80-20). Elle est ensuite placée dans une solution de 250ml de THF et 150ml d'EtOH en présence de Pd/C sous 6bars d'hydrogène. Ce mélange est agité 24h à température ambiante. Après filtration sur célite, le milieu réactionnel est concentré à sec et **12a** est isolé sous forme d'huile claire (32 g, rendement=69%). CCM gel de silice 60 F 254 Merck, heptane-AcOEt : 80-20, Rf=0,56.

### b) Intermédiaires 12b-12e

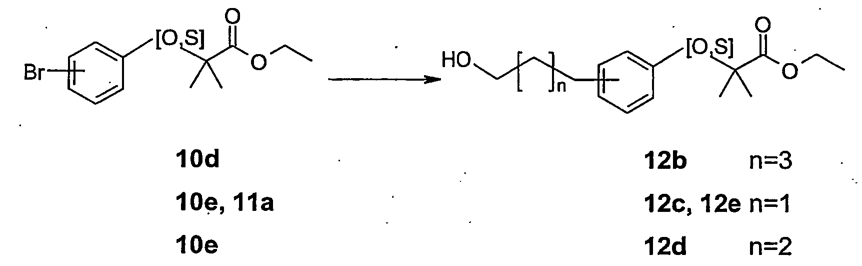

La synthèse des intermédiaires **12b-12e** est réalisée à partir des composés bromés de départ listés dans le tableau **13** suivant le mode opératoire décrit pour la synthèse de **12a** en utilisant différents alkynols. (rq : lorsqu'il s'agit d'un dérivé soufré, on utilise pour l'étape d'hydrogénation le catalyseur de Wilkinson).

**Tableau 13: intermédiaires 12b-12e**

| **Synthons de départ** | **alkynol** | Rdt | CCM | Etat | **Intermédiaires 12b-12d** |
|---|---|---|---|---|---|
| **10d** | 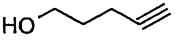 | 76% | Heptane-AcOEt: 60-40 Rf=0,37 | huile | **12b**: 2-[3-(5-Hydroxy-pentyl)-phénoxy]-2-méthyl-propionate d'éthyle |
| **10e** | 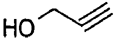 | 5% | Heptane-AcOEt: 60-40 Rf=0,33 | huile | **12c**: 2-[4-(3-Hydroxy-propyl)-phénoxy]-2-méthyl-propionate d'éthyle |
| **10e** | 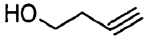 | 55% | EP-AcOEt:80-20 Rf=0,14 | huile | **12d**: 2-[4-(4-Hydroxy-butyl) -phénoxy]-2-méthyl-propionate d'éthyle |
| **11a** | 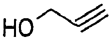 | 73% | EP-AcOEt:70-30 Rf=0,26 | huile | **12e**: 2-[3-(3-Hydroxy-propyl)-phénylsulfanyl]-2-méthyl-propionate d'éthyle |

| | | | | | |
|---|---|---|---|---|---|
| CCM : gel de silice 60 F 254 Merck, EP=éther de pétrole. | | | | | |

### Intermédiaires 13 :

### a) 2-[4-(2-Amino-éthyl)-phénoxy]-2-méthyl-propionate d'éthyle (13a)

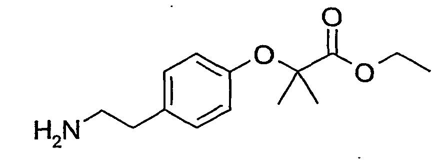

10,1g, (73,6 mmol) de tyramine sont placés en présence de bicarbonate de sodium (6,1 g, 72,6 mmol) dans un mélange de 100ml d'eau et 50ml d'acétone à 0°C. 11,6ml (81,2mmol) de chloroformiate de benzyle sont coulés goutte à goutte à cette température puis le milieu réactionnel est agité 4h à température ambiante. Après concentration à sec, le résidu obtenu est repris par l'eau puis extrait à l'AcOEt. Après séchage sur MgSO₄, les phases organiques sont concentrées à sec et le solide obtenu est recristallisé dans l'éther diéthylique : 17,2g de solide sont ainsi obtenus (rendement 86%). Ils sont ensuite placés dans 37ml de bromoisobutyrate d'éthyle en présence de 8,8g (63,7mmol) de carbonate de potassium à 130°C pendant 5h. Après filtration, le milieu réactionnel est concentré à sec et le résidu obtenu est purifié par chromatographie flash sur silice (éther de pétrole-AcOEt :70-30). 22,7g d'huile claire sont obtenus (rendement=93%). Cette huile est ensuite placée dans 200ml d'EtOH en présence de palladium sur charbon sous 3bars d'hydrogène puis cette solution est agitée pendant 3h à température ambiante. Après filtration sur célite, le milieu réactionnel est concentré à sec et 14,7g d'intermédiaire **13a** sont ainsi isolés sous forme d'huile (rendement quantitatif). CCM gel de silice 60 F 254 Merck, CH₂Cl₂-MeOH :90-10, Rf=0,11.

### b) 2-[3-(2-Amino-éthyl)-phénoxy]-2-méthyl-propionate d'éthyle (13b)

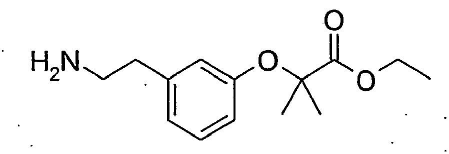

**10d** (14g, 49 mmol) est placé en présence de N-vinylphtalimide (11 g, 63 mmol) et de 27ml (194mmol) de triéthylamine dans 160 ml de DMF. Pd(OAc)₂ (0,3 g) et P(oTol)₃ (0,4g) sont ajoutés et le milieu réactionnel est agité à 110°C pendant 10 h. Le milieu réactionnel est concentré à sec et le résidu obtenu est repris par l'eau et extrait à l'AcOEt. Après séchage sur MgSO₄, les phases organiques sont concentrées à sec et l'huile isolée est purifiée par chromatographie flash sur silice (heptane-AcOEt :90-10). 11,5g d'huile sont obtenus (rendement 62%) puis placés dans une solution de 70ml de THF et 70ml d'EtOH en présence de Pd/C sous 6bar d'hydrogène. Ce mélange est agité 72h à température ambiante. Après filtration sur célite, le milieu réactionnel est concentré à sec et le résidu obtenu est purifié par chromatographie flash sur silice (éther de pétrole-AcOEt :80-20). 10,9g d'huile claire sont obtenus (rendement=94%). Cette huile est ensuite placée dans 140ml d'EtOH en présence de 3,5ml d'hydrate d'hydrazine puis cette solution est chauffée à reflux pendant 4h. Après filtration des insolubles, le milieu réactionnel est concentré à sec puis le résidu obtenu est purifié par chromatographie flash sur silice (CH₂Cl₂-MeOH-NH₄OH :90-9-1). 5,7g d'intermédiaire **13b** sont ainsi isolés sous forme d'huile (rendement=80%). CCM gel de silice 60 F 254 Merck, CH₂Cl₂-MeOH-NH₄OH :90-9-1, Rf=0,28.

### c) Intermédiaires 13c-13i

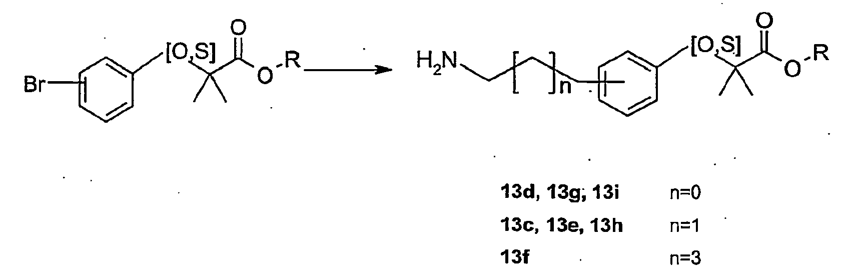

La synthèse des intermédiaires **13c-13i** est réalisée à partir des composés bromés de départ listés dans le tableau **14** suivant le mode opératoire décrit pour la synthèse de **13b** en utilisant des phtalimides diversement N-alkylés (rq : lorsqu'il s'agit d'un dérivé soufré, on utilise pour l'étape d'hydrogénation le catalyseur de Wilkinson).

**Tableau 14: intermédiaires 13b:13i**

| **Synthons de départ** | **Phtalimides N-substitués** | Rdt total | CCM Forme | Etat | **Intermédiaires 13c-13h** |
|---|---|---|---|---|---|
| **10d** | N-allylphtalimide | 80% | CH₂Cl₂-MeOH:90-10 Rf=0,12 | huile | **13c**:2-[3-(3-Amino-propyl)-phénoxy]-2-méthyl-propionate d'éthyle |
| **11a** | N-vinylphtalimide | 66% | CH₂Cl₂-MeOH-NH₄OH : 90-9-1 Rf=0,20 | huile | **13d** : 2-[3-(2-Amino-éthyl)-phénylsulfanyl]-2-méthyl-propionate d'éthyle |
| **11a** | N-allylphtalimide | 70% | CH₂Cl₂-MeOH-NH₄OH : 90-9-1 Rf=0,30 | huile | **13e**:2-[3-(3-Amino-propyl)-phénylsulfanyl]-2-méthyl-propionate d'éthyle |
| **11a** | N-but-3-enyl-phtalimide | 38% | CH₂Cl₂-MeOH-NH₄OH :90-9-1 Rf=0,27 | huile | **13f** : 2-[3-(4-Amino-butyl)-phénylsulfanyl]-2-méhyl-propionate d'éthyle |
| **11b** | N-vinylphtalimide | 41% | CH₂Cl₂-MeOH-NH₄OH :90-9-1 Rf=0,28 | huile | **13g**:2-[4-(2-Amino-éthyl)-phénylsulfanyl]-2-méthyl-propionate d'éthyle |
| **11b** | N-allylphtalimide | 38% | CH₂Cl₂-MeOH-NH₄OH :90-9-1 Rf=0,23 | huile | 13h: 2-[4-(3-Amino-propyl)-phénylsulfanyl]-2-méthyl-propionate d'éthyle |
| **11e** | N-vinylphtalimide | 63% | CH₂Cl₂-MeOH:90-10 Rf=0,18 | huile | 13i:2-[4-(2-Amino-éthyl)-phénylsulfanyl]-2-méthyl-propionate de tert-butyle |

| | | | | | |
|---|---|---|---|---|---|
| CCM : gel de silice 60 F 254 Merck | | | | | |

### b) Intermédiaires 13i-13n

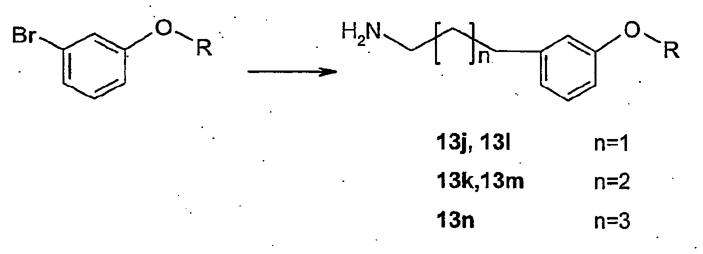

La synthèse des intermédiaires **13j-13n** est réalisée à partir des composés bromés de départ listés dans le tableau **15** suivant les modes opératoires décrits pour la synthèse de **13b** en utilisant des phtalimides diversement N-alkylés.

**Tableau 15: intermédiaires 13j-13n**

| **Synthons de départ** | **Phtalimides** N-**substitués** | Rdt | CCM | Etat | **Intermédiaires 13i-13n** |
|---|---|---|---|---|---|
| 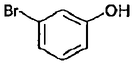 | N-allylphtalimide | 47% | CH₂Cl₂-MeOH-NH₄OH :80-18-2 Rf=0,25 | solide | 13j : 3-(3-Amino-propyl)-phénol |
| 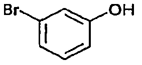 | N-but-3-ènylphtalimide | 49% | CH₂Cl₂-MeOH-NH₄OH :80-18-2 Rf=0,24 | huile | 13k: 3-(4-Amino-butyl)-phénol |
| 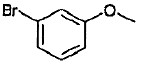 | N-allylphtalimide | 59% | CH₂Cl₂-MeOH-NH₄OH :80-18-2 Rf=0,20 | huile | 13l: 3-(3-Méthoxy-phényl)-propylamine |
| 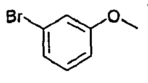 | N-but-3-ènylphtalimide | 77% | CH₂Cl₂-MeOH-NH₄OH :80-18-2 Rf=0,28 | huile | **13m** : 4-(3-Méthoxy-phényl)-butylamine |
| 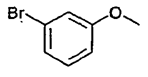 | N-pent-4-ènyl-phtalimide | 24% | CH₂Cl₂-MeOH-NH₄OH :90-9-1 Rf=0,45 | huile | **13n:** 5-(3-Méthoxy-phényl)-pentylamine |

| | | | | | |
|---|---|---|---|---|---|
| CCM : gel de silice 60 F 254 Merck | | | | | |

### Intermédiaires 14:

### a) 2-[4-(2-Heptylamino-éthyl)-phénoxyl-]-2-méthyl-propionate d'éthyle (14a)

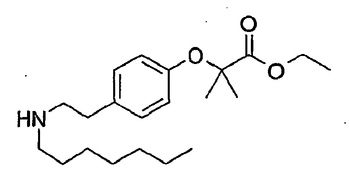

**13a** (4,1g, 5mmol) est placé en présence d'acide heptanoique (2,3ml, 16,5mmol) dans 42ml de dichlorométhane. 2,3ml (16,5mmol) de triéthylamine sont additionnés suivis de 2,8ml (18,2mmol) de diéthylcyanophosphonate. Ce mélange est agité 24h à température ambiante puis le milieu réactionnel est concentré à sec. Le résidu obtenu est repris par H₂O puis extrait à l'AcOEt. Après séchage sur MgSO₄, les phases organiques sont concentrées à sec et l'huile isolée est purifiée par chromatographie flash sur silice (CH₂Cl₂-MeOH :90-10). 4,7g d'huile sont obtenus (rendement=78%). 13ml d'une solution BH₃/THF (1M) sont placés à O°C sous azote puis l'huile précédemment obtenue, diluée dans 20ml de THF, est coulée goutte à goutte. Ce mélange est placé à reflux pendant 2h puis neutralisé par 10ml d'EtOH/HCl (1,5N). La solution est à nouveau placée à reflux pendant 1 h puis concentrée à sec. Le résidu obtenu est repris par une solution saturée de bicarbonate de sodium puis extrait au dichlorométhane. Après séchage sur MgSO₄, les phases organiques sont concentrées à sec et l'huile isolée est purifiée par chromatographie flash sur silice (CH₂Cl₂-MeOH :90-10). 1,6g d'intermédiaire **14a** sont ainsi isolés sous forme d'huile (rendement=73%). CCM gel de silice 60 F 254 Merck, CH₂Cl₂-MeOH-NH₄OH :90-9-1, Rf=0,50.

### b) Intermédiaires 14b-14c

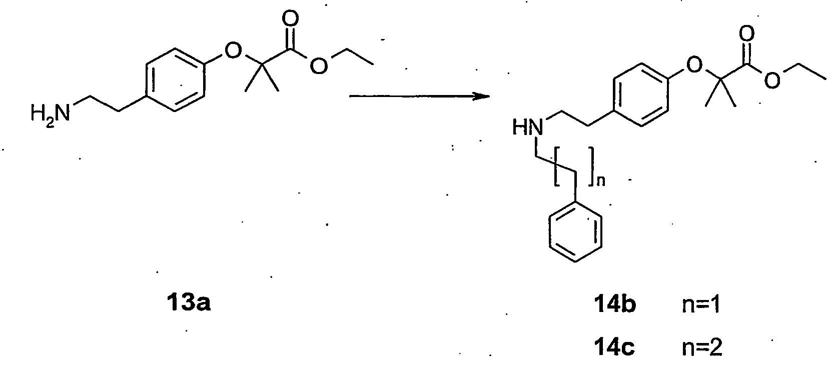

La synthèse des intermédiaires **14b-14c** est réalisée à partir de l'intermédiaire **13a** suivant le mode opératoire décrit pour la synthèse de **14a** en utilisant différents acides carboxyliques.

**Tableau 16: intermédiaires 14b-14c**

| **Acides carboxyliques** | Rdt total | Eluant | Forme | **Intermédiaires 14b-14c** |
|---|---|---|---|---|
| 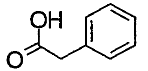 | 78% | CH₂Cl₂-MeOH : 90-10 Rf=0,37 | Huile | **14b**: 2-Méthyl-2-[4-(2-phénéthylamino-éthyl)-phénoxy]-propionate d'éthyle |
| 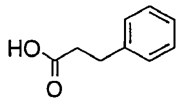 | 65% | CH₂Cl₂-MeOH : 90-10 Rf=0,44 | Huile | **14c**: 2-Méthyl-2-{4-[2-(3-phényl-propylamino)-éthyl]-phénoxy}-propionate d'éthyle |

| | | | | |
|---|---|---|---|---|
| CCM : gel de silice 60 F 254 Merck | | | | |

### EXEMPLES

### Exemple 1 : 2-{2-[2-(4-Butyl-2-méthyl-3,5-dioxo-2,3,4,5-tétrahydro-[1,2,4] triazin-6-yloxy)-éthyl]-phénoxy}-2-méthyl-propionate d'éthyle (1)

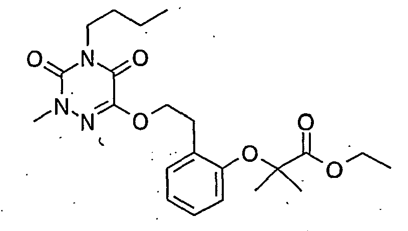

Le composé **1** est préparé selon la méthode de synthèse **1** : 1 g (4 mmol) de dérivé **10g** et 1g (3,8mmol) de triazine **5a** sont placés dans 3 ml de DMF en présence de 0,5g (3,7mmol) de K₂CO₃. Ce mélange est agité à 120°C pendant 7 h. Après filtration et concentration à sec du milieu réactionnel, le résidu obtenu est purifié par chromatographie flash sur silice (CH₂Cl₂-AcOEt :98-2). On isole 1,2g de cristaux blancs (rendement :74%).
CCM gel de silice 60 F 254 Merck, CH₂Cl₂-AcOEt :95-5, Rf=0,40. F=76°C
RMN ¹H (CDCl₃) : 0,94ppm (t, 3H, J=7,4Hz), 1,20ppm (t, 3H, J=7,2Hz), 1,39ppm (m, 2H, J=7,5ppm), 1,63ppm (m, 8H), 3,16ppm (t, 2H, J=7,6Hz), 3,50ppm (s, 3H), 3,95ppm (t, 2H, J=7,6Hz), 4,22ppm (q, 2H, J=7,0Hz), 4,37ppm (t, 2H, J=7,6Hz), 7,00ppm (m, 4H).

### Exemple 2 : 2-{3-[2-(2-Heptyl-4-méthyl-3,5-dioxo-2,3,4,5-tétrahydro-[1,2,4]triazin-6-yloxy)-éthyl]-phénoxy}-2-méthyl-propionate d'éthyle (2)

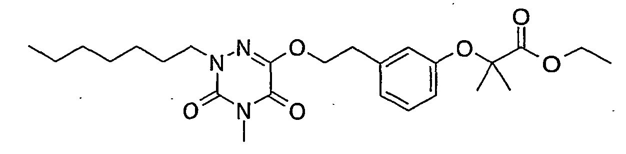

Le composé **2** (huile) est préparé à partir de la triazine 4b et de l'intermédiaire **10h** selon la méthode de synthèse **1**.
CCM gel de silice 60 F 254 Merck, CH₂Cl₂-AcOEt :90-10, Rf=0,62.

### Exemple 3 : 2-Méthyl-2-(3-{2-[2-méthyl-3,5-dioxo-4-(4,4,4-trifluoro-butyl)-2,3,4,5-tétrahydro-[1,2,4]triazin-6-yloxy]-éthyl}-phénoxy)-propionate d'éthyle (3)

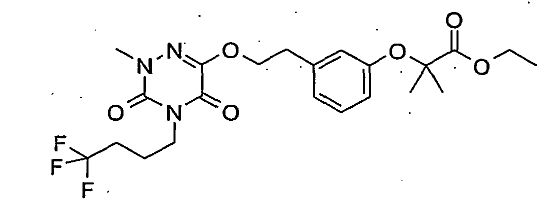

Le composé **3** (huile) est préparé à partir de la triazine **6a** et de l'intermédiaire **10h** selon la méthode de synthèse **1**.
CCM gel de silice 60 F 254 Merck, CH₂Cl₂-AcOEt :90-10, Rf=0,57.

### Exemple 4 : 2-Méthyl-2-(3-{3-[4-méthyl-3,5-dioxo-2-(4,4,4-trifluoro-butyl)-2,3,4,5-tétrahydro-[1,2,4]triazin-6-yloxy]-propyl}-phénoxy)-propionate d'éthyle (4)

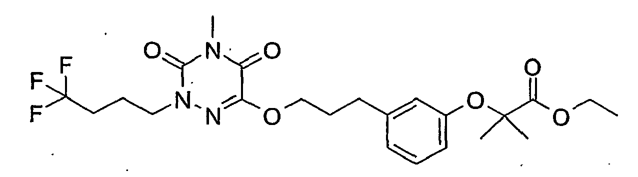

Le composé **4** (huile) est préparé à partir de la triazine **4b** et de l'intermédiaire **12a** selon la méthode de synthèse **1**.
CCM gel de silice 60 F 254 Merck, éther de pétrole-AcOEt :70-30, Rf=0,30.

### Exemple 5 : 2-{3-[3-(2-Heptyl-4-méthyl-3,5-dioxo-2,3,4,5-tétrahydro-[1,2,4]triazin-6-yloxy)-propyl]-phénoxy}-2-méthyl-propionate d'éthyle(5).

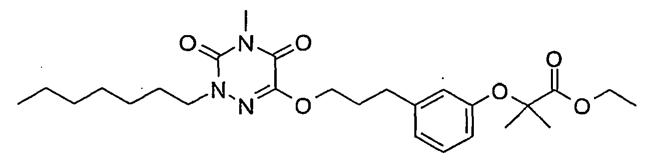

Le composé **5** (huile) est préparé à partir de la triazine **4b** et de l'intermédiaire **12a** selon la méthode de synthèse **1**.
CCM gel de silice 60 F 254 Merck, éther de pétrole-AcOEt :70-30, Rf=0,52.

### Exemple 6 : 2-Méthyl-2-(3-{3-[2-méthyl-3,5-dioxo-4-(4,4,4-trifluoro-butyl)-2,3,4,5-tétrahydro-[1,2,4]triazin-6-yloxy]-propyl}-phénoxy)-propionate d'éthyle (6)

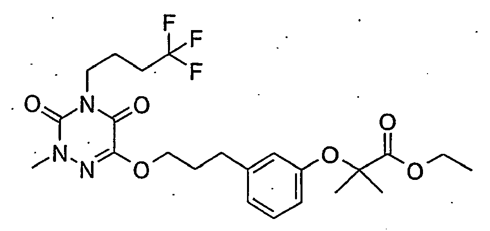

Le composé **6** (huile) est préparé à partir de la triazine **6a** et de l'intermédiaire **12a** selon la méthode de synthèse **1**.
CCM gel de silice 60 F 254 Merck, éther de pétrole-AcOEt :70-30, Rf=0,46.

### Exemple 7 : 2-{3-[3-(4-Heptyl-2-méthyl-3,5-dioxo-2,3,4,5-tétrahydro-[1,2,4]triazin-6-yloxy)-propyl]-phénoxy}-2-méthyl-propionate d'éthyle (7)

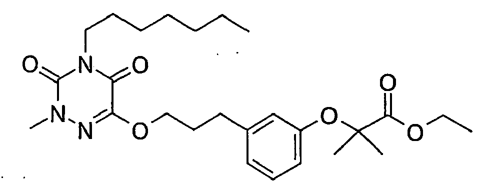

Le composé **7** (huile) est préparé à partir de la triazine **7a** et de l'intermédiaire **12a** selon la méthode de synthèse **1**.
CCM gel de silice 60 F 254 Merck, éther de pétrole-AcOEt :70-30, Rf=0,61.

### Exemple 8 : 2-(3-{3-[4-Heptyl-3,5-dioxo-2-(4,4,4-trifluoro-butyl)-2,3,4,5-tétrahydro-[1,2,4]triazin-6-yloxy]-propyl}-phénoxy)-2-méthyl-propionate d'éthyle (8)

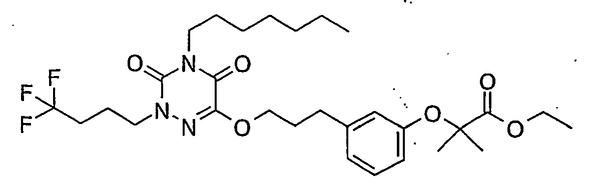

Le composé **8** (huile) est préparé à partir de la triazine **7b** et de l'intermédiaire **12a** selon la méthode de synthèse **1**.
CCM gel de silice 60 F 254 Merck, éther de pétrole-AcOEt :70-30, Rf=0,53.

### Exemple 9 : 2-Méthyl-2-(3-{5-[4-méthyl-3,5-dioxo-2-(4,4,4-trifluoro-butyl)-2,3,4,5-tétrahydro-[1,2,4]triazin-6-yloxy]-pentyl}-phénoxy)-propionate d'éthyle (9)

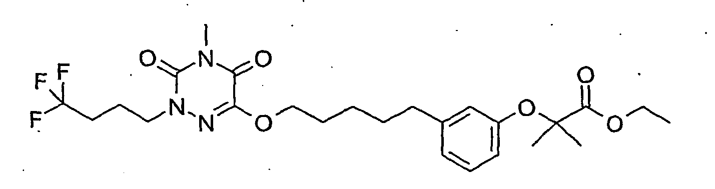

Le composé **9** (huile) est préparé à partir de la triazine **4a** et de l'intermédiaire **12b** selon la méthode de synthèse **1**.
CCM gel de silice 60 F 254 Merck, éther de pétrole-AcOEt :80-20, Rf=0,34.

### Exemple 10 : 2-{3-[5-(2-Heptyl-4-méthyl-3,5-dioxo-2,3,4,5-tétrahydro-[1,2,4]triazin-6-yloxy)-pentyl]-phénoxy}-2-méthyl-propionate d'éthyle (10)

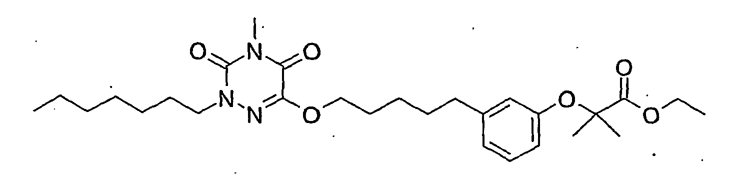

Le composé **10** (huile) est préparé à partir de la triazine **4b** et de l'intermédiaire **12b** selon la méthode de synthèse **1**.
CCM gel de silice 60 F 254 Merck, éther de pétrole-AcOEt :70-30, Rf=0,54.

### Exemple 11 : 2-{3-[5-(4-Butyl-2-méthyl-3,5-dioxo-2,3,4,5-tétrahydro-[1,2,4]triazin-6-yloxy)-pentyl]-phénoxy}-2-méthyl-propionate d'éthyle (11)

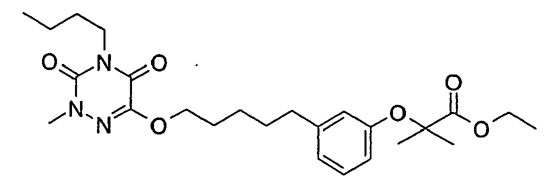

Le composé 11 (huile) est préparé à partir de la triazine **5a** et de l'intermédiaire **12b** selon la méthode de synthèse **1**.
CCM gel de silice 60 F 254 Merck, éther de pétrole-AcOEt :80-20, Rf=0,29.

### Exemple 12 : Acide 2-{3-[5-(4-butyl-2-méthyl-3,5-dioxo-2,3,4,5-tétrahydro-[1,2,4]triazin-6-yloxy)-pentyl]-phénoxy}-2-méthyl-propionique (12)

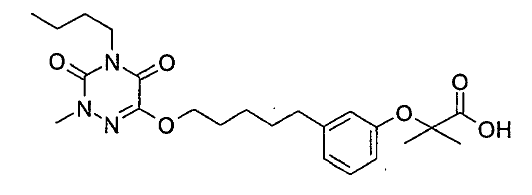

Le composé **12** (huile) est préparé par hydrolyse du composé **11** (HCl 12N, reflux, 16h, 62%).
CCM gel de silice 60 F 254 Merck, CH₂Cl₂-MeOH:90-10, Rf=0,43.

### Exemple 13 : 2-{3-[5-(2,4-Dibutyl-3,5-dioxo-2,3,4,5-tétrahydro-[1,2,4] triazin-6-yloxy)-pentyl]-phénoxy}-2-méthyl-propionate d'éthyle (13)

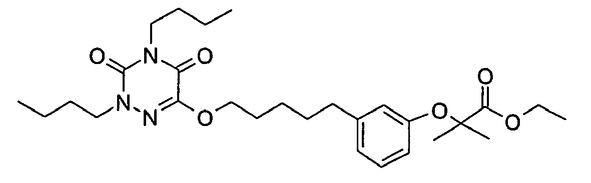

Le composé **13** (huile) est préparé à partir de la triazine **1c** et de l'intermédiaire **12b** selon la méthode de synthèse **1**.
CCM gel de silice 60 F 254 Merck, éther de pétrole-AcOEt :80-20, Rf=0,63.

### Exemple 14 : 2-(3-{5-[4-Butyl-3,5-dioxo-2-(4,4,4-trifluoro-butyl)-2,3,4,5-tétrahydro-[1,2,4]triazin-6-yloxy]-pentyl}-phénoxy)-2-méthyl-propionate d'éthyle (14)

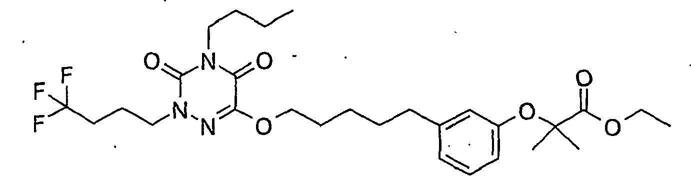

Le composé **14** (huile) est préparé à partir de la triazine 5b et de l'intermédiaire **12b** selon la méthode de synthèse **1**.
CCM gel de silice 60 F 254 Merck, éther de pétrole-AcOEt :80-20, Rf=0,35.

### Exemple 15 : 2-{3-[5-(4-Butyl-2-heptyl-3,5-dioxo-2,3,4,5-tétrahydro-[1,2,4]triazin-6-yloxy)-pentyl]-phénoxy}-2-méthyl-propionate d'éthyle (15)

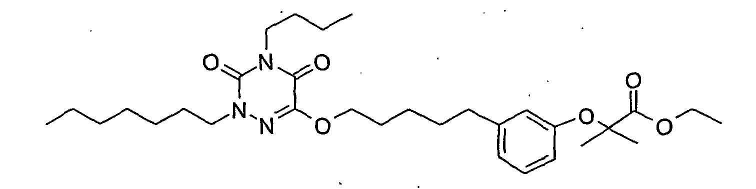

Le composé **15** (huile) est préparé à partir de la triazine **5c** et de l'intermédiaire **12b** selon la méthode de synthèse **1**.
CCM gel de silice 60 F 254 Merck, éther de pétrole-AcOEt :80-20, Rf=0,53.

### Exemple 16 : 2-Méthyl-2-(3-{5-[2-méthyl-3,5-dioxo-4-(4,4,4-trifluoro-butyl)-2,3,4,5-tétrahydro-[1,2,4]triazin-6-yloxy]-pentyl}-phénoxy)-propionate d'éthyle (16)

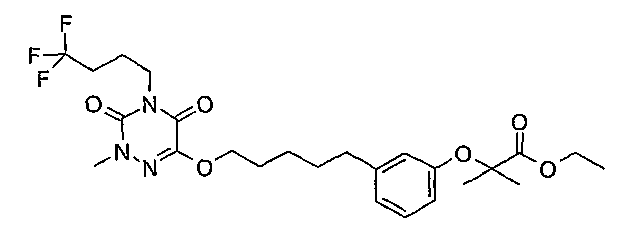

Le composé **16** (huile) est préparé à partir de la triazine **6a** et de l'intermédiaire **12b** selon la méthode de synthèse 1.
CCM gel de silice 60 F 254 Merck, éther de pétrole-AcOEt :80-20, Rf=0,33.

### Exemple 17 : 2-{3-[5-(4-Heptyl-2-méthyl-3,5-dioxo-2,3,4,5-tétrahydro-[1,2,4]triazin-6-yloxy)-pentyl]-phénoxy}-2-méthyl propionate d'éthyle (17)

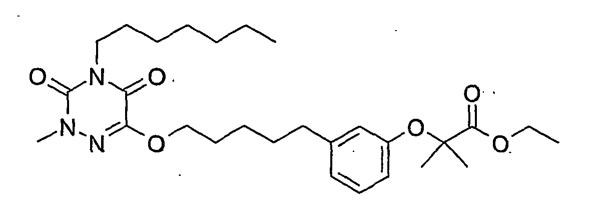

Le composé **17** (huile) est préparé à partir de la triazine **7a** et de l'intermédiaire **12b** selon la méthode de synthèse **1.**
CCM gel de silice 60 F 254 Merck, éther de pétrole-AcOEt :80-20, Rf=0,36.

### Exemple 18 : 2-(3-{5-[4-Heptyl-3,5-dioxo-2-(4,4,4-trifluoro-butyl)-2,3,4,5-tétrahydro-[1,2,4]triazin-6-ytoxy]-pentyl}-phénoxy}-2-méthyl-propionate d'éthyle (18)

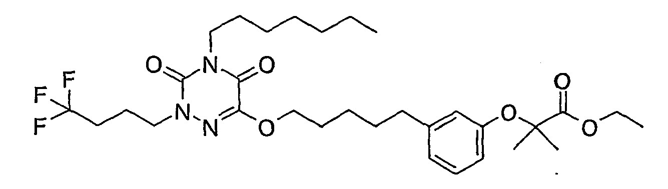

Le composé **18** (huile) est préparé à partir de la triazine **7b** et de l'intermédiaire **12b** selon la méthode de synthèse **1.**

CCM gel de silice 60 F 254 Merck, heptane-AcOEt :70-30, Rf=0,35.

### Exemple 19 : 2-{3-[5-(2-Benzyl-4-heptyl-3,5-dioxo-2,3,4,5-tétrahydro-[1,2,4]triazin-6-yloxy)-pentyl]-phénoxy}-2-méthyl-propionate d'éthyle (19)

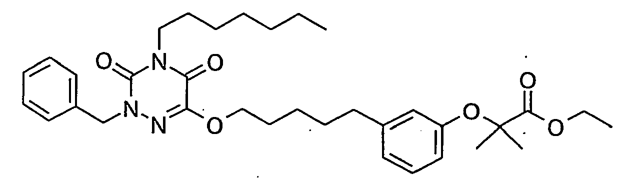

Le composé **19** (huile) est préparé à partir de la triazine **7c** et de l'intermédiaire **12b** selon la méthode de synthèse **1.**
CCM gel de silice 60 F 254 Merck, éther de pétrole-AcOEt :80-20, Rf=0,51.

### Exemple 20 : 2-[4-(2-Heptyl-4-méthyl-3,5-dioxo-2,3,4,5-tétrahydro-[1,2,4] triazin-6-yloxymethyl)-phénoxy]-2-méthyl-propionate d'éthyle (20)

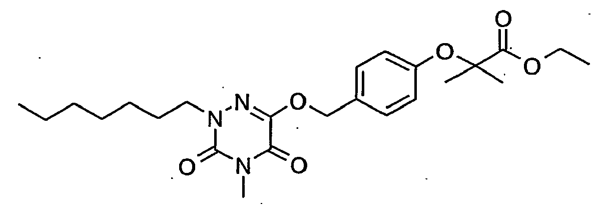

Le composé **20** (huile) est préparé à partir de la triazine **4b** et de l'intermédiaire **10f** selon la méthode de synthèse **1.**
CCM gel de silice 60 F 254 Merck, éther de pétrole-AcOEt :80-20, Rf=0,28.

### Exemple 21 : 2-Méthyl-2-{4-[2-méthyl-3,5-dioxo-4-(4,4,4-trifluoro-butyl)-2,3,4,5-tétrahydro-[1,2,4]triazin-6-yloxyméthyl]-phénoxy}-propionate d'éthyle (21)

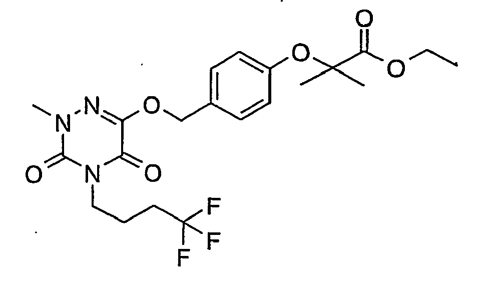

Le composé **21** (huile) est préparé à partir de la triazine **6a** et de l'intermédiaire **10f** selon la méthode de synthèse **1.**
CCM gel de silice 60 F 254 Merck, éther de pétrole-AcOEt :60-40, Rf=0,46.

### Exemple 22 : 2-[4-(4-Heptyl-2-méthyl-3,5-dioxo-2,3,4,5-tétrahydro-[1,2,4]triazin-6-yloxyméthyl)-phénoxy]-2-méthyl-propionate d'éthyle (22)

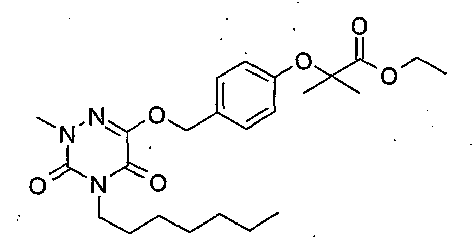

Le composé **22** (huile) est préparé à partir de la triazine **7a** et de l'intermédiaire **10f** selon la méthode de synthèse **1.**
CCM gel de silice 60 F 254 Merck, éther de pétrole-AcOEt :80-20, Rf=0,34.

### Exemple 23 : 2-{4-[4-Heptyl-3,5-dioxo-2-(4,4,4-trifluoro-butyl)-2,3,4,5-tétrahydro-[1,2,4]triazin-6-yloxyméthyl]-phénoxy}-2-méthyl-propionate d'éthyle (23)

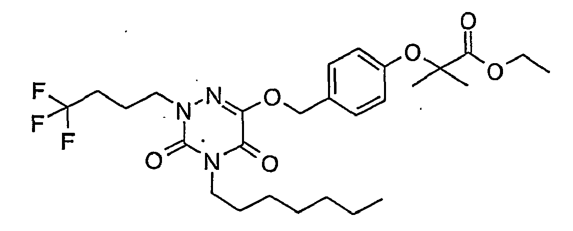

Le composé **23** (huile) est préparé à partir de la triazine **7b** et de l'intermédiaire **10f** selon la méthode de synthèse **1**.
CCM gel de silice 60 F 254 Merck, éther de pétrole-AcOEt :90-10, Rf=0,61.

### Exemple 24 : 2-Méthyl-2-(4-{2-[4-méthyl-3,5-dioxo-2-(4,4,4-trifluoro-butyl)-2,3,4,5-tétrahydro-[1,2,4]triazin-6-yloxy]-éthyl}-phénoxy)-propionate d'éthyle (24)

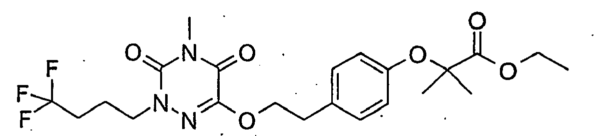

Le composé **24** (huile) est préparé à partir de la triazine **4a** et de l'intermédiaire **10j** selon la méthode de synthèse **1.**
CCM gel de silice 60 F 254 Merck, CH₂Cl₂-AcOEt :90-10, Rf=0,45.

### Exemple 25 : 2-{4-[2-(2-Heptyl-4-méthyl-3,5-dioxo-2,3,4,5-tétrahydro-[1,2,4]triazin-6-yloxy)-éthyl]-phénoxy}-2-méthyl-propionate d'éthyle (25)

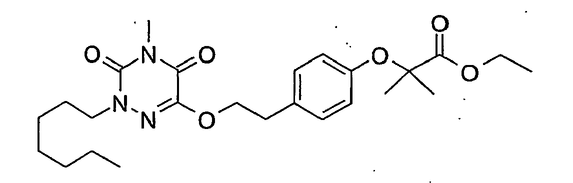

Le composé **25** (huile) est préparé à partir de la triazine **4b** et de l'intermédiaire **10j** selon la méthode de synthèse **1.**
CCM gel de silice 60 F 254 Merck, éther de pétrole-ACOEt :80-20, Rf=0,45.

### Exemple 26 : 2-{4-[2-(4-Heptyl-2-méthyl-3,5-dioxo-2,3,4,5-tétrahydro-[1,2,4]triazin-6-yloxy)-éthyl]-phénoxy}-2-méthyl-propionate d'éthyle (26)

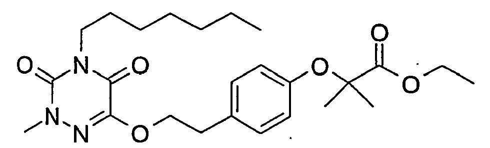

Le composé **26** (huile) est préparé à partir de la triazine **7a** et de l'intermédiaire **10j** selon la méthode de synthèse **1.**
CCM gel de silice 60 F 254 Merck, éther de pétrole-AcOEt :70-30, Rf=0,46.

### Exemple 27 : 2-{4-[3-(2-Heptyl-4-méthyl-3,5-dioxo-2,3,4,5-tétrahydro-[1,2,4]triazin-6-yloxy)-propyl]-phénoxy}-2-méthyl-propionate d'éthyle (27)

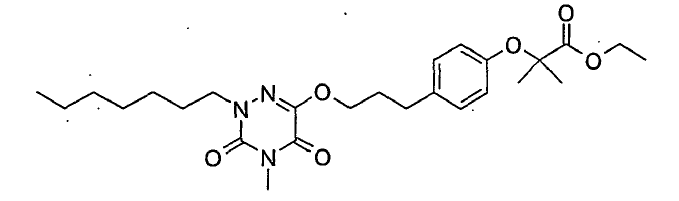

Le composé **27** (huile) est préparé à partir de la triazine **4b** et de l'intermédiaire **12c** selon la méthode de synthèse **1.**
CCM gel de silice 60 F 254 Merck, éther de pétrole-AcOEt :70-30, Rf=0,33.

### Exemple 28 : 2-Méthyl-2-(4-{4-[4-méthyl-3,5-dioxo-2-(4,4,4-trifluoro-butyl)-2,3,4,5-tétrahydro-[1,2,4]triazin-6-yloxy]-butyl}-phénoxy)-propionate d'éthyle (28)

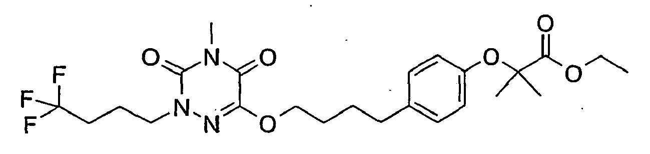

Le composé **28** (huile) est préparé à partir de la triazine **4a** et de l'intermédiaire **12d** selon la méthode de synthèse **1.**
CCM gel de silice 60 F 254 Merck, éther de pétrole-AcOEt :70-30, Rf=0,31.

### Exemple 29 : 2-{4-[4-(2-Heptyl-4-méthyl-3,5-dioxo-2,3,4,5-tétrahydro-[1,2,4]triazin-6-yloxy)-butyl]-phénoxy}-2-méthyl-propionate d'éthyle (29)

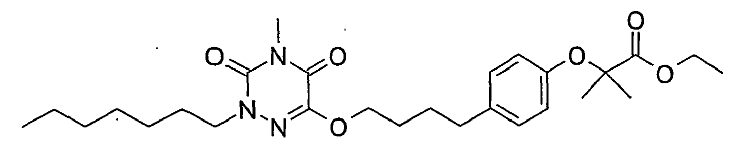

Le composé **29** (huile) est préparé à partir de la triazine **4b** et de l'intermédiaire **12d** selon la méthode de synthèse **1.**
CCM gel de silice 60 F 254 Merck, éther de pétrole-AcOEt :70-30, Rf=0,49.

### Exemple 30 : 2-Méthyl-2-(4-{4-[2-méthyl-3,5-dioxo-4-(4,4,4-trifluoro-butyl)-2,3,4,5-tétrahydro-[1,2,4]triazin-6-yloxy]-butyl}-phénoxy)-propionate d'éthyle (30)

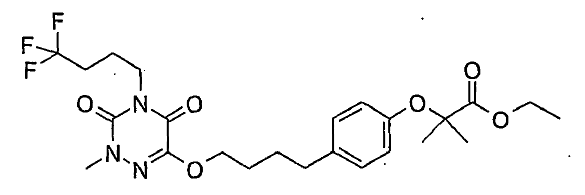

Le composé **30** (huile) est préparé à partir de la triazine **6a** et de l'intermédiaire **12d** selon la méthode de synthèse **1.**
CCM gel de silice 60 F 254 Merck, éther de pétrole-AcOEt :70-30, Rf=0,35.

### Exemple 31 : 2-(4-{4-[4-Heptyl-3,5-dioxo-2-(4,4,4-trifluoro-butyl)-2,3,4,5-tétrahydro-[1,2,4]triazin-6-yloxy]-butyl}-phénoxy)-2-méthyl-propionate d'éthyle (31)

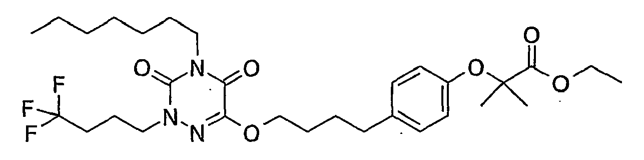

Le composé **31** (huile) est préparé à partir de la triazine **7b** et de l'intermédiaire **12d** selon la méthode de synthèse **1.**
CCM gel de silice 60 F 254 Merck, éther de pétrole-AcOEt :80-20, Rf=0,51.

### Exemple 32 : 2-{3-[3-(2-Heptyl-4-méthyl-3,5-dioxo-2,3,4,5-tétrahydro-[1,2,4] triazin-6-yloxy)-propyl]-phénylsulfanyl}-2-méthyl-propionate d'éthyle (32)

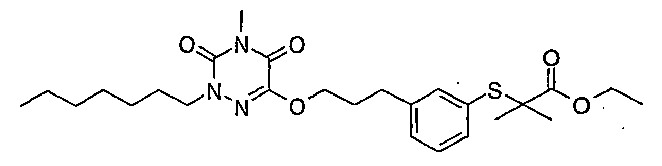

Le composé **32** (huile) est préparé à partir de la triazine **4b** et de l'intermédiaire **12e** selon la méthode de synthèse **1.**
CCM gel de silice 60 F 254 Merck, éther de pétrole-AcOEt :80-20, Rf=0,37.

### Exemple 33 : 2-{3-[3-(2-Heptyl-4-méthyl-3,5-dioxo-2,3,4,5-tétrahydro-[1,2, 4]triazin-6-yloxy)-propoxy]-phénoxy}-2-méthyl-propionate d'éthyle (33)

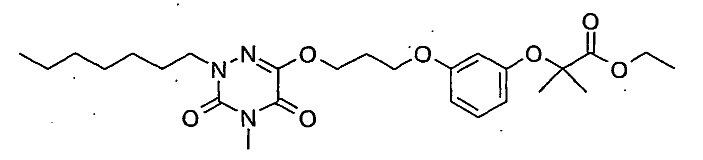

Le composé **33** est préparé selon la méthode de synthèse **2:** 1,1 g (3,7mmol) de triazine **9q,** 0,83g (3,7mmol) d'ester **10b** et 1,25g (4,7mmol) de PPh₃ sont placés dans 30ml de THF à 40°C. 0,74ml (4,7mmol) de DEAD dilué dans 10ml de THF sont coulés goutte à goutte et le mélange est agité 1 h à 40°C. Après, concentration à sec du milieu réactionnel, le résidu obtenu est purifié par chromatographie flash sur alumine neutre (heptane-AcOEt :80-20). On isole 0,8g de composé **33** sous forme d'huile claire (rendement :43%).
CCM gel de silice 60 F 254 Merck, heptane-AcOEt :50-50, Rf=0,30.

### Exemple 34 : 2-{3-[3-(2-Heptyl-4-méthyl-3,5-dioxo-2,3,4,5-tétrahydro-[1,2,4]triazin-6-yloxy)-propoxy]-phénylsulfanyl}-2-méthyl-propionate d'éthyle (34)

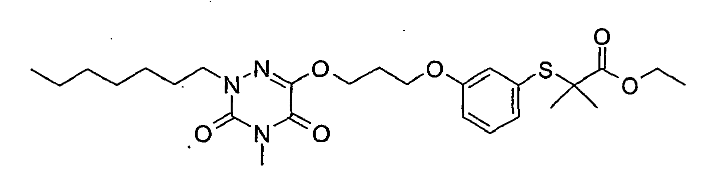

Le composé **34** (huile) est préparé à partir de la triazine **9q** et de l'intermédiaire **11c** selon la méthode de synthèse **2.**
CCM gel de silice 60 F 254 Merck, heptane-AcOEt :50-50, Rf=0,33.

### Exemple 35 : 2-(3-{3-[3,5-Dioxo-2,4-bis-(4,4,4-trifluoro-butyl)-2,3,4,5-tétrahydro-[1,2,4]triazin-6-yloxy]-propoxy}-phénoxy)-2-méthyl-propionate d'éthyle (35)

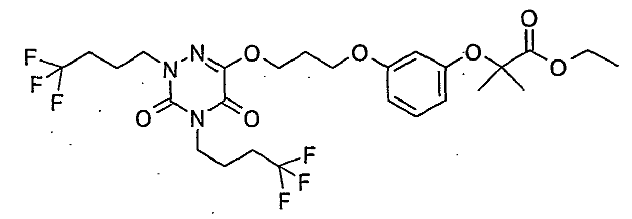

Le composé **35** (huile) est préparé à partir de la triazine **9r** et de l'intermédiaire **10b** selon la méthode de synthèse **2.**
CCM gel de silice 60 F 254 Merck, heptane-AcOEt :50-50, Rf=0,50.

### Exemple 36 : -2-{3-[4-(2-Heptyl-4-méthyl-3,5-dioxo-2,3,4,5-tétrahydro [1,2,4]triazin-6-yloxy)-butoxy]-phénoxy}-2-méthyl-propionate d'éthyle (36)

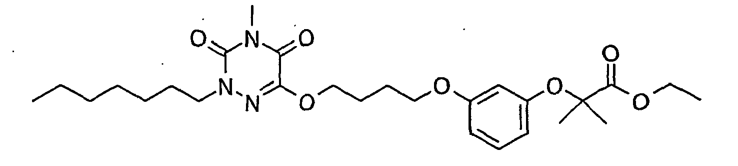

Le composé **36** (huile) est préparé à partir de la triazine **9s** et de l'intermédiaire **10b** selon la méthode de synthèse **2.**
CCM gel de silice 60 F 254 Merck, heptane-AcOEt :50-50, Rf=0,34.

### Exemple 37 : 2-{3-[4-(2-Heptyl-4-méthyl-3,5-dioxo-2,3,4,5-tétrahydro-[1,2,4] triazin-6-yloxy)-butoxy]-phénylsulfanyl}-2-méthyl-propionate d'éthyle (37)

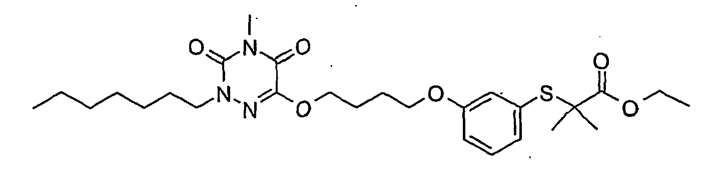

Le composé **37** (huile) est préparé à partir de la triazine 9s et de l'intermédiaire **11c** selon la méthode de synthèse **2.**
CCM gel de silice 60 F 254 Merck, heptane-AcOEt :50-50, Rf=0,37.

### Exemple 38 : 2-{4-[2-(2-Heptyl-4-méthyl-3,5-dioxo-2,3,4,5-tétrahydro-[1,2,4] triazin-6-yloxy)-éthoxy]-phénylsulfanyl}-2-méthyl-propionate d'éthyle (38)

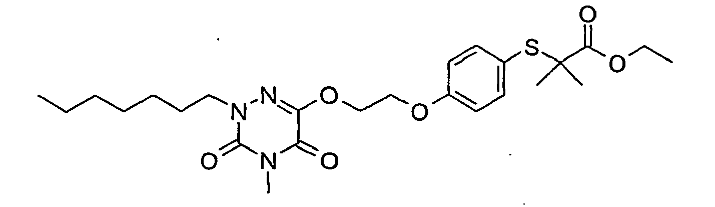

Le composé **38** (huile) est préparé à partir de la triazine **9p** et de l'intermédiaire **11 d** selon la méthode de synthèse **2.**
CCM gel de silice 60 F 254 Merck, heptane-AcOEt :50-50, Rf=0,33.

### Exemple 39 : 2-(4-{3-[3,5-Dioxo-2,4-bis-(4,4,4-trifluoro-butyl)-2,3,4,5-tétrahydro-[1,2,4]triazin-6-yloxy]-propoxy}-phénoxy)-2-méthyl-propionate d'éthyle (39)

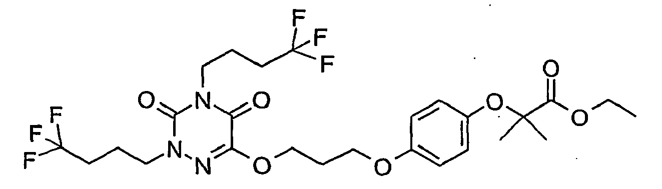

Le composé **39** (huile) est préparé à partir de la triazine **9r** et de l'intermédiaire **10c** selon la méthode de synthèse **2.**
CCM gel de silice 60 F 254 Merck, heptane-AcOEt :50-50, Rf=0,50.

### Exemple 40 : 2-{4-[2-(2-Heptyl-4-méthyl-3,5-dioxo-2,3,4,5-tétrahydro-[1,2, 4]triazin-6-ylamino)-éthoxy]-phénoxy}-2-méthyl-propionate d'éthyle (40)

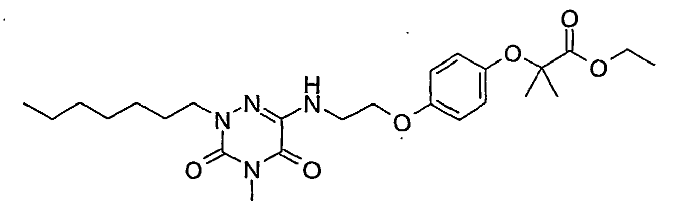

Le composé **40** (huile) est préparé à partir de la triazine **9b** et de l'intermédiaire **10c** selon la méthode de synthèse **2.**
CCM gel de silice 60 F 254 Merck, éther de pétrole-AcOEt :80-20, Rf=0,29.

### Exemple 41 : 2-(4-{2-[3,5-Dioxo-2,4-bis-(4,4,4-trifluoro-butyl)-2,3,4,5-tétrahydro-[1,2,4]triazin-6-ylamino]-éthoxy}-phénoxy)-2-méthyl-propionate d'éthyle (41)

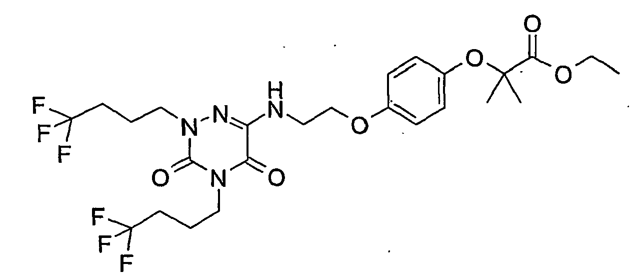

Le composé **41** (huile) est préparé à partir de la triazine **9a** et de l'intermédiaire 10c selon la méthode de synthèse **2.**
CCM gel de silice 60 F 254 Merck, éther de pétrole-AcOEt :80-20, Rf=0,50.

### Exemple 42 : 2-{4-[2-(2,4-Diheptyl-3,5-dioxo-2,3,4,5-tétrahydro-[1,2,4] triazin-6-ylamino)-éthoxy]-phénoxy}-2-méthyl-propionate d'éthyle (42)

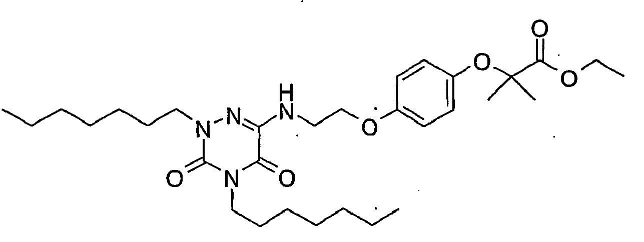

Le composé **42** (huile) est préparé à partir de la triazine 9c et de l'intermédiaire **10c** selon la méthode de synthèse **2.**
CCM gel de silice 60 F 254 Merck, éther de pétrole-AcOEt :80-20, Rf=0,85.

### Exemple 43: 2-(4-{2-[2,4-Bis-(3-cyclohexyl-propyl)-3,5-dioxo-2,3,4,5-tétrahydro-[1,2,4]triazin-6-ylamino]-éthoxy}-phénoxy)-2-méthyl-propionate d'éthyle (43)

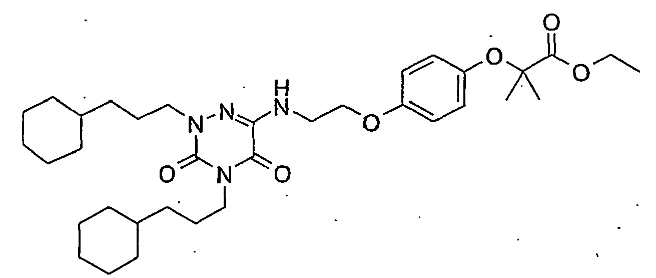

Le composé **43** (huile) est préparé à partir de la triazine **9d** et de l'intermédiaire **10c** selon la méthode de synthèse **2.**
CCM gel de silice 60 F 254 Merck, heptane-AcOEt :80-20, Rf=0,85.

### Exemple 44 : 2-Méthyl-2-(4-{3-[4-méthyl-3,5-dioxo-2-(4,4,4-trifluoro-butyl)-2,3,4,5-tétrahydro-[1,2,4]triazin-6-ylamino]-propoxy}-phénoxy)-propionate d'éthyle (44)

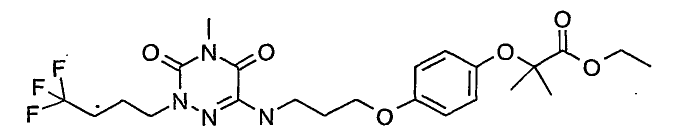

Le composé **44** (huile) est préparé à partir de la triazine **9f** et de l'intermédiaire **10c** selon la méthode de synthèse **2.**
CCM gel de silice 60 F 254 Merck, éther de pétrole-AcOEt :70-30, Rf=0,36.

### Exemple 45 : 2-{4-[3-(2-Heptyl-4-méthyl-3,5-dioxo-2,3,4,5-tétrahydro-[1,2,4]triazin-6-ylamino)-propoxyl-phénoxy}-2-méthyl-propionate d'éthyle (45)

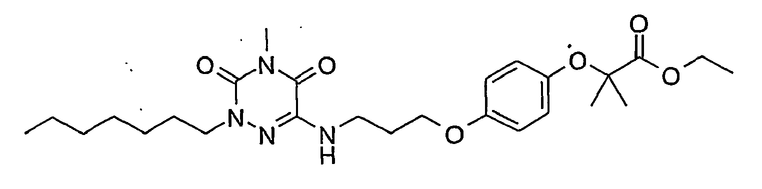

Le composé **45** (huile) est préparé à partir de la triazine **9g** et de l'intermédiaire **10c** selon la méthode de synthèse **2.**
CCM gel de silice 60 F 254 Merck, éther de pétrole-AcOEt :70-30, Rf=0,48.

### Exemple 46 : 2-Méthyl-2-(4-{3-[2-méthyl-3,5-dioxo-4-(4,4,4-trifluoro-butyl)-2,3,4,5-tétrahydro-[1,2,4]triazin-6-ylamino]-propoxy}-phénoxy)-propionate d'éthyle (46)

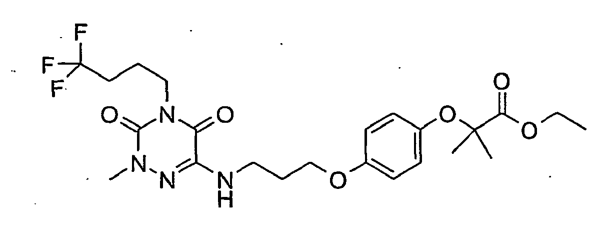

Le composé **46** (solide) est préparé à partir de la triazine **9h** et de l'intermédiaire **10c** selon la méthode de synthèse **2.**
CCM gel de silice 60 F 254 Merck, éther de pétrole-AcOEt :70-30, Rf=0,37. F=116°C

### Exempte 47 : 2-{4-[3-(4-Heptyl-2-méthyl-3,5-dioxo-2,3,4,5-tétrahydro-[1,2,4] triazin-6-ylamino)-propoxy]-phénoxy}-2-méthyl-propionate d'éthyle (47)

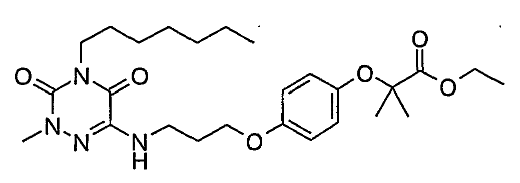

Le composé **47** (huile) est préparé à partir de la triazine **9i** et de l'intermédiaire **10c** selon la méthode de synthèse **2.**
CCM gel de silice 60 F 254 Merck, éther de pétrole-AcOEt :80-20, Rf=0,30.

### Exemple 48 : 2-(4-{3-[4-Heptyl-3,5-dioxo-2-(4,4,4-trifluoro-butyl)-2,3,4,5-tétrahydro-[1,2,4]triazin-6-ylamino]-propoxy}-phénoxy)-2-méthyl-propionate d'éthyle (48)

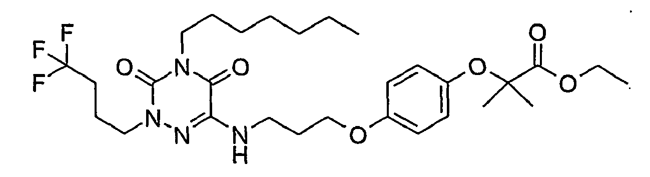

Le composé **48** (huile) est préparé à partir de la triazine **9j** et de l'intermédiaire **10c** selon la méthode de synthèse **2.**
CCM gel de silice 60 F 254 Merck, éther de pétrole-AcOEt :80-20, Rf=0,50.

### Exemple 49 : 2-{4-[3-(4-Heptyl-2-méthyl-3,5-dioxo-2,3,4,5-tétrahydro-[1,2,4]triazin-6-ylamino)-propoxy]-phénylsulfanyl}-2-méthyl-propionate d'éthyle (49)

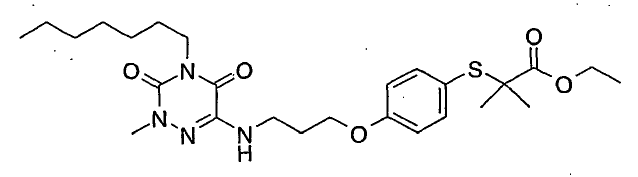

Le composé **49** (huile) est préparé à partir de la triazine **9i** et de l'intermédiaire **11 d** selon la méthode de synthèse **2.**
CCM gel de silice 60 F 254 Merck, éther de pétrole-AcOEt :80-20, Rf=0,46.

### Exemple 50 : 2-{4-[4-(4-Heptyl-2-méthyl-3,5-dioxo-2,3,4,5-tétrahydro-[1,2, 4]triazin-6-ylamino)-butoxy]-phénoxy}-2-méthyl-propionate d'éthyle (50)

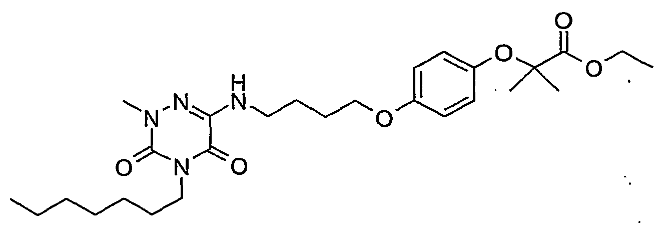

Le composé **50** (huile) est préparé à partir de la triazine **9I** et de l'intermédiaire **10c** selon la méthode de synthèse **2.**
CCM gel de silice 60 F 254 Merck, éther de pétrole-AcOEt :80-20, Rf=0,50.

### Exemple 51 : 2-(3-{3-[(2-Heptyl-4-méthyl-3,5-dioxo-2,3,4,5-tétrahydro-[1,2,4]triazin-6-yl)-(4,4,4-trifluoro-butyl)-amino]-propoxy}-phénoxy)-2-méthyl-propionate d'éthyle (51)

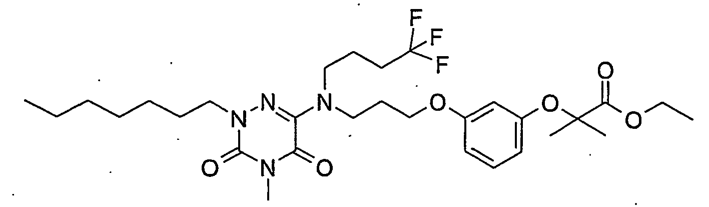

Le composé **51** (huile) est préparé à partir de la triazine **9m** et de l'intermédiaire **10b** selon la méthode de synthèse **3** en utilisant des conditions de couplage de Mitsunobu telles que celles décrites pour l'exemple **33.** CCM gel de silice 60 F 254 Merck, éther de pétrole-AcOEt :90-10, Rf=0,56.

### Exemple 52 : 2-(3-{3-[(2,4-Diméthyl-3,5-dioxo-2,3,4,5-tétrahydro-[1,2,4] triazin-6-yl)-heptyl-amino]-propoxy}-phénylsulfanyl)-2-méthyl-propionate d'éthyle (52)

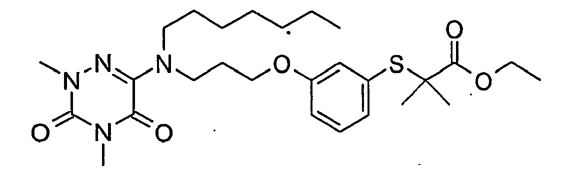

Le composé **52** (huile) est préparé à partir de la triazine **9n** et de l'intermédiaire **11c** selon la méthode de synthèse **3** en utilisant des conditions de couplage telles que celles décrites pour l'exemple **33.**
CCM gel de silice 60 F 254 Merck, heptane-AcOEt :70-30, Rf=0,22.

### Exemple 53 : 2-(4-{3-[(2,4-Diméthyl-3,5-dioxo-2,3,4,5-tétrahydro-[1,2,4] triazin-6-yl)-heptyl-amino]-propoxy}-phénylsulfanyl)-2-méthyl-propionate d'éthyle (53)

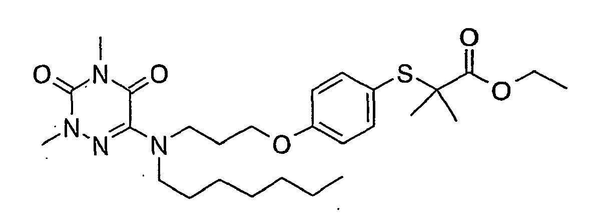

Le composé **53** (huile) est préparé à partir de la triazine **9n** et de l'intermédiaire **11d** selon la méthode de synthèse **3** en utilisant des conditions de couplage telles que celles décrites pour l'exemple **33.**
CCM gel de silice 60 F 254 Merck, heptane-AcOEt :80-20, Rf=0,45.

### Exemple 54 : 2-(3-{4-[(2,4-Diméthyl-3,5-dioxo-2,3,4,5-tétrahydro-[1,2,4] triazin-6-yl)-heptyl-amino]-butoxy)-phénylsulfanyl)-2-méthyl-propionate d'éthyle (54)

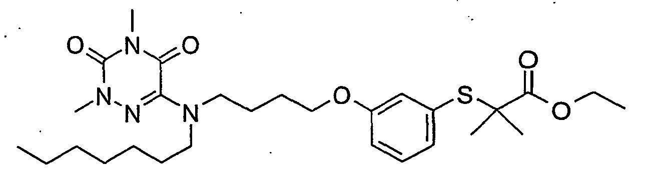

Le composé **54** (huile) est préparé à partir de la triazine **9o** et de l'intermédiaire **11c** selon la méthode de synthèse **3** en utilisant des conditions de couplage telles que celles décrites pour l'exemple **33.**
CCM gel de silice 60 F 254 Merck, heptane-AcOEt :70-30, Rf=0,25.

### Exemple 55 : 2-(2-{2-[3,5-Dioxo-2,4-bis-(4,4,4-trifluoro-butyl)-2,3,4,5-tétrahydro-[1,2,4]triazin-6-ylamino]-éthyl}-phénoxy)-2-méthyl-propionate d'éthyle (55)

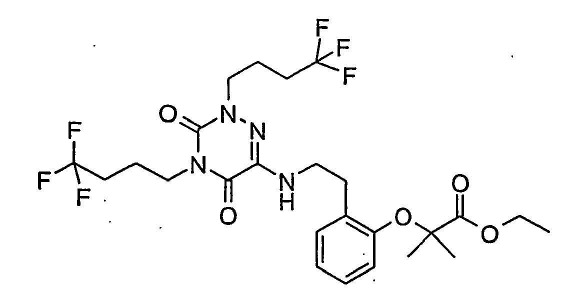

Le composé **55** est préparé selon la méthode de synthèse **4** : 6,6g (16,1mmol) de triazine **1d**, 2g (13,4mmol) de 2-(2-méthoxy-phényl)-éthylamine et 4,7ml (33,9mmol) de triéthylamine sont placés dans 20ml de nbutanol à 120°C pendant 28h. Après concentration à sec du milieu réactionnel, le résidu obtenu est repris par H₂O et extrait à l'AcOEt. Les phases organiques sont séchées sur MgSO₄ puis concentrées à sec. L'huile obtenue est purifiée par chromatographie flash sur silice (éther de pétrole-AcOEt :90-10). On isole 3,1g d'intermédiaire sous forme d'huile (rendement :48%) qui sont ensuite placés dans 30ml de CH₂Cl₂ à 0°C sous azote. Une solution de BBr₃ (12,8ml à 1 M dans CH₂Cl₂) est coulée goutte à goutte et le milieu réactionnel est agité 3,5h à température ambiante. Il est ensuite placé à 0°C et acidifié par une solution d'HCl 0,1 N jusqu'à pH=1. La phase organique est décantée puis lavée avec 100ml d'eau. Après séchage sur MgSO₄, elle est concentrée à sec et le résidu obtenu est purifié par chromatographie flash sur silice (CH₂Cl₂-AcOEt :95-5).

On isole 1,9g de phénol correspondant (rendement=65%) qui est ensuite placé dans 2ml de DMF en présence de 1,9ml (12,5mmol) de bromoisobutyrate d'éthyle et 0,6g (4,3mmol) de K₂CO₃. Le milieu réactionnel est chauffé à 130°C pendant 22h puis filtré et concentré à sec. L'huile obtenue est purifiée par chromatographie flash sur silice (CH₂Cl₂-AcOEt :98-2). 0,8g de composé **55** (rendement=34%) sous forme d'huile est isolé.
CCM gel de silice 60 F 254 Merck, CH₂Cl₂-AcOEt :95-5, Rf=0,66.

### Exemple 56 : 2-Méthyl-2-(3-{2-[4-méthyl-3,5-dioxo-2-(4,4,4-trifluoro-butyl)-2,3,4,5-tétrahydro-[1,2,4]triazin-6-ylamino]-éthyl}-phénoxy)-propionate d'éthyle (56)

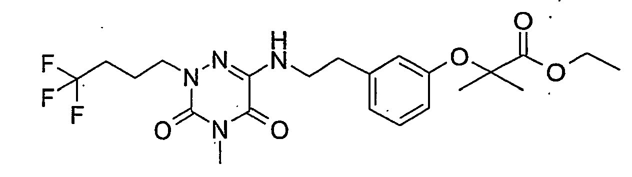

Le composé **56** est préparé selon la méthode de synthèse **1** : 1,1 g (4,4 mmol) de dérivé **13b** et 1g (3,7mmol) de triazine **4a** sont placés dans 10 ml de nBuOH en présence de 1,3ml (9,3mmol) de triéthylamine. Ce mélange est agité à 120°C pendant 24 h. Après concentration à sec du milieu réactionnel, le résidu obtenu est repris par H₂O et extrait à l'AcOEt. Les phases organiques sont séchées sur MgSO₄ puis concentrées à sec. L'huile obtenue est purifiée par chromatographie flash sur silice (éther de pétrole-AcOEt :90-10). On isole 0,4g de composé **56** sous forme d'huile (rendement :27%).
CCM gel de silice 60 F 254 Merck, éther de pétrole-AcOEt :80-20, Rf=0,19.

### Exemple 57 : 2-{3-[2-(2-Heptyl-4-méthyl-3,5-dioxo-2,3,4,5-tétrahydro-[1,2, 4]triazin-6-ylamino)-éthyl]-phénoxy}-2-méthyl-propionate d'éthyle (57)

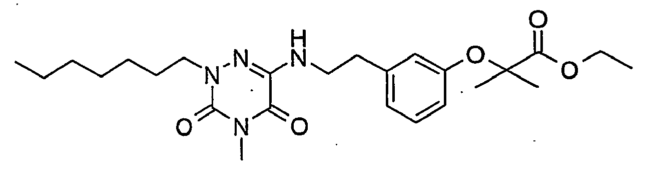

Le composé **57** (huile) est préparé à partir de la triazine **4b** et de l'intermédiaire **13b** selon la méthode de synthèse **1**.
CCM gel de silice 60 F 254 Merck, éther de pétrole-AcOEt : 70-30, Rf=0,44.

### Exemple 58 : 2-Méthyl-2-(3-{2-[2-méthyl-3,5-dioxo-4-(4,4,4-trifluoro-butyl)-2,3,4,5-tétrahydro-[1,2,4]triazin-6-ylamino]-éthyl}-phénoxy)-propionate d'éthyle (58)

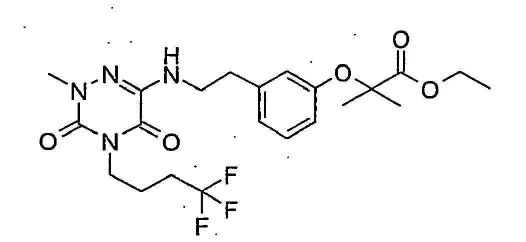

Le composé **58** (huile) est préparé à partir de la triazine 6a et de l'intermédiaire **13b** selon la méthode de synthèse **1.**
CCM gel de silice 60 F 254 Merck, éther de pétrole-AcOEt :80-20, Rf=0,33.

### Exemple 59 : 2-{3-[2-(4-Heptyl-2-méthyl-3,5-dioxo-2,3,4,5-tétrahydro-[1,2, 4]triazin-6-ylamino)-éthyl]-phénoxy)-2-méthyl-propionate d'éthyle (59)

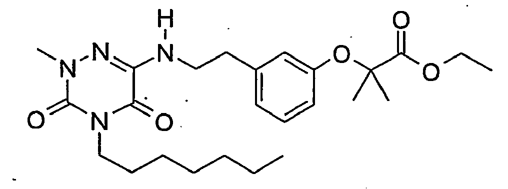

Le composé **59** (huile) est préparé à partir de la triazine **7a** et de l'intermédiaire **13b** selon la méthode de synthèse **1.**
CCM gel de silice 60 F 254 Merck, éther de pétrole-AcOEt :80-20, Rf=0,50.

### Exemple 60 : 2-(3-{2-[4-Heptyl-3,5-dioxo-2-(4,4,4-trifluoro-butyl)-2,3,4,5-tétrahydro-[1,2,4]triazin-6-ylamino]-éthyl}-phénoxy)-2-méthyl-propionate d'éthyle (60)

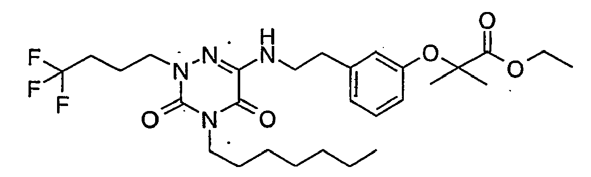

Le composé **60** (huile) est préparé à partir de la triazine **7b** et de l'intermédiaire **13b** selon la méthode de synthèse **1.**
CCM gel de silice 60 F 254 Merck, éther de pétrole-AcOEt :90-10, Rf=0,22.

### Exemple 61 : 2-{3-[2-(4-Heptyl-2-méthyl-3,5-dioxo-2,3,4,5-tétrahydro-[1,2, 4]triazin-6-ylamino)-éthyl]-phénylsulfanyl}-2-méthyl-propionate d'éthyle (61)

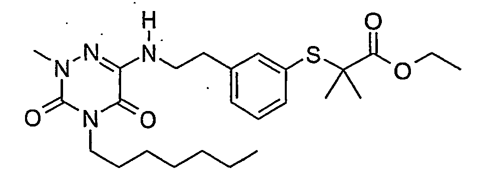

Le composé **61** (huile) est préparé à partir de la triazine **7a** et de l'intermédiaire **13d** selon la méthode de synthèse **1.**
CCM gel de silice 60 F 254 Merck, éther de pétrole-AcOEt :70-30, Rf=0,61.

### Exemple 62 : 2-Méthyl-2-(3-{3-[4-méthyl-3,5-dioxo-2-(4,4,4-trifluoro-butyl)-2,3,4,5-tétrahydro-[1,2,4]triazin-6-ylamino]-propyl}-phénoxy)-propionate d'éthyle (62)

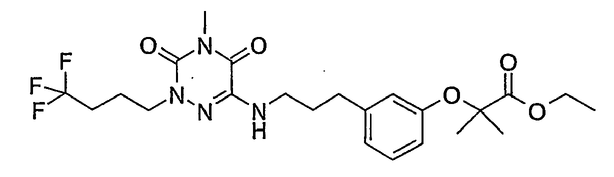

Le composé **62** (huile) est préparé à partir de la triazine **4a** et de l'intermédiaire **13j** selon la méthode de synthèse **4.**
CCM gel de silice 60 F 254 Merck, CH₂Cl₂-AcOEt :90-10, Rf=0,60.

### Exemple 63 : 2-(3-{3-[2-(2-Cyano-éthyl)-4-méthyl-3,5-dioxo-2,3,4,5-tétrahydro-[1,2,4]triazin-6-ylamino]-propyl}-phénoxy)-2-méthyl-propionate d'éthyle (63)

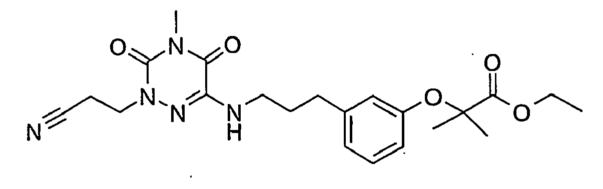

Le composé **63** (huile) est préparé à partir de la triazine **3a** et de l'intermédiaire **13j** selon la méthode de synthèse **4.**
CCM gel de silice 60 F 254 Merck, éther de pétrole-AcOEt :80-20, Rf=0,44.

### Exemple 64: 2-{3-[3-(2-Heptyl-4-méthyl-3,5-dioxo-2,3,4,5-tétrahydro-[1,2, 4]triazin-6-ylamino)-propyl]-phénoxy)-2-méthyl-propionate d'éthyle (64)

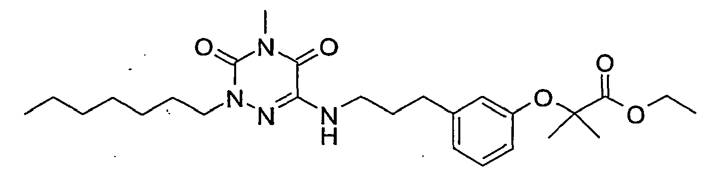

Le composé **64** (huile) est préparé à partir de la triazine **4b** et de l'intermédiaire **13j** selon la méthode de synthèse **4.**
CCM gel de silice 60 F 254 Merck, éther de pétrole-AcOEt :80-20, Rf=0,20.

### Exemple 65 : 2-Méthyl-2-(3-{3-[2-méthyl-3,5-dioxo-4-(4,4,4-trifluoro-butyl)-2,3,4,5-tétrahydro-[1,2,4]triazin-6-ylamino]-propyl}-phénoxy)-propionate d'éthyle (65)

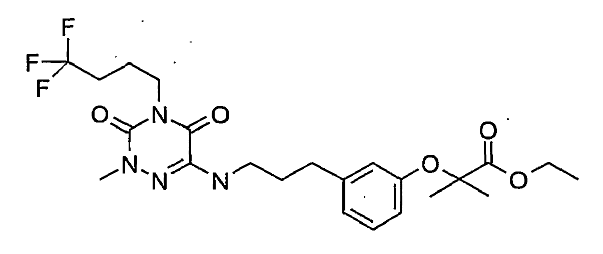

Le composé **65** (huile) est préparé à partir de la triazine **6a** et de l'intermédiaire **13j** selon la méthode de synthèse **4.**
CCM gel de silice 60 F 254 Merck, CH₂Cl₂-AcOEt :90-10, Rf=0,78.

### Exemple 66 : 2-(3-{3-[3,5-Dioxo-2,4-bis-(4,4,4-trifluoro-butyl)-2,3,4,5-tétrahydro-[1,2,4]triazin-6-ylamino]-propyl}-phénoxy)-2-méthyl-propionate d'éthyle (66)

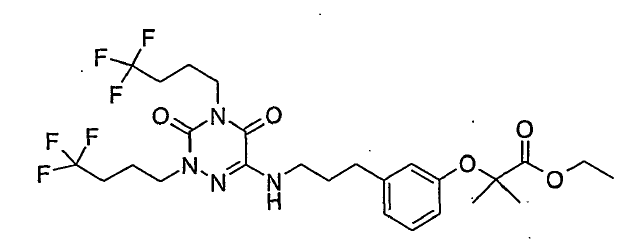

Le composé **66** (huile) est préparé à partir de la triazine **1d** et de l'intermédiaire **13l** selon la méthode de synthèse **4**.
CCM gel de silice 60 F 254 Merck, éther de pétrole-AcOEt :70-30, Rf=0,49.

### Exemple 67 : 2-{3-[3-(4-Heptyl-2-méthyl-3,5-dioxo-2,3,4,5-tétrahydro-[1,2, 4]triazin-6-ylamino)-propyl]-phénoxy}-2-méthyl-propionate d'éthyle (67)

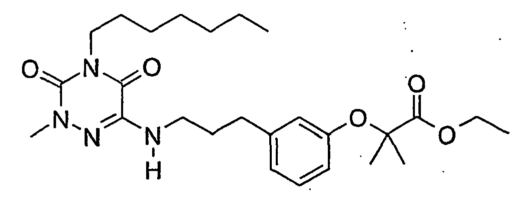

Le composé **67** (huile) est préparé à partir de la triazine **7a** et de l'intermédiaire **13c** selon la méthode de synthèse **1**.
CCM gel de silice 60 F 254 Merck, éther de pétrole-AcOEt :80-20, Rf=0,50.

### Exemple 68 : 2-(3-{3-[4-Heptyl-3,5-dioxo-2-(4,4,4-trifluoro-butyl)-2,3,4,5-tétrahydro-[1,2,4]triazin-6-ylamino]-propyl}-phénoxy)-2-méthyl-propionate d'éthyle (68)

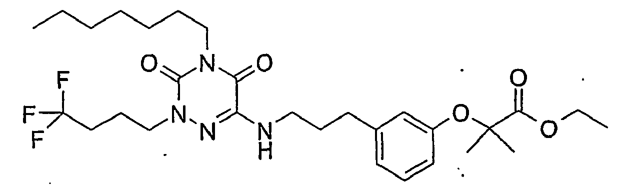

Le composé **68** (huile) est préparé à partir de la triazine **7b** et de l'intermédiaire **13j** selon la méthode de synthèse **4**.
CCM gel de silice 60 F 254 Merck, éther de pétrole-AcOEt :80-20, Rf=0,63.

### Exemple 69 : 2-{3-[3-(2,4-Diheptyl-3,5-dioxo-2,3,4,5-tétrahydro-[1,2,4]triazin-6-ylamino)-propyl]-phénoxyl-2-méthyl-propionate d'éthyle (69)

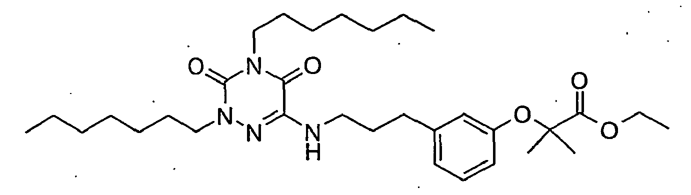

Le composé **69** (huile) est préparé à partir de la triazine **1e** et de l'intermédiaire **13j** selon la méthode de synthèse **4**.
CCM gel de silice 60 F 254 Merck, éther de pétrole-AcOEt :80-20, Rf=0,53.

### Exemple 70 : 2-{3-[3-(2-Benzyl-4-heptyl-3,5-dioxo-2,3,4,5-tétrahydro-[1,2, 4]triazin-6-ylamino)-propyl]-phénoxy}-2-méthyl-propionate d'éthyle (70)

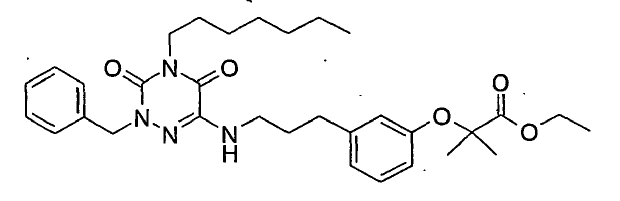

Le composé **70** (huile) est préparé à partir de la triazine **7c** et de l'intermédiaire **13j** selon la méthode de synthèse **4**.
CCM gel de silice 60 F 254 Merck, éther de pétrole-AcOEt :80-20, Rf=0,47.

### Exemple 71 : 2-(3-{3-[4-Benzyl-3,5-dioxo-2-(4,4,4-trifluoro-butyl)-2,3,4,5-tétrahydro-[1,2,4]triazin-6-ylamino]-propyl}-phénoxy)-2-méthyl-propionate d'éthyle (71)

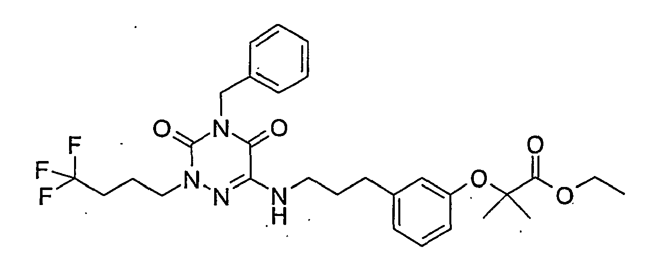

Le composé **71** (huile) est préparé à partir de la triazine **8a** et de l'intermédiaire **13j** selon la méthode de synthèse **4**.
CCM gel de silice 60 F 254 Merck, éther de pétrole-AcOEt :80-20, Rf=0,30.

### Exemple 72 : 2-{3-[3-(4-Benzyl-2-heptyl-3,5-dioxo-2,3,4,5-tétrahydro-[1,2, 4] triazin-6-ylamino)-propyl]-phénoxy}-2-méthyl-propionate d'éthyle (72)

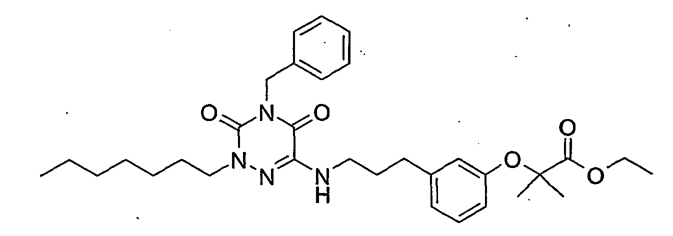

Le composé **72** (huile) est préparé à partir de la triazine **8b** et de l'intermédiaire **13j** selon la méthode de synthèse **4**.
CCM gel de silice 60 F 254 Merck, éther de pétrole-AcOEt :80-20, Rf=0,42.

### Exemple 73 : 2-{3-[3-(4-Heptyl-2-méthyl-3,5-dioxo-2,3,4,5-tétrahydro-[1,2,4]triazin-6-ylamino)-propyl]-phénylsulfanyl}-2-méthyl-propionate d'éthyle (73)

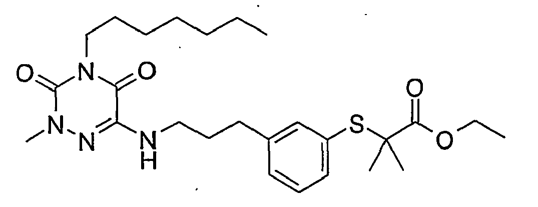

Le composé **73** (huile) est préparé à partir de la triazine **7a** et de l'intermédiaire **13e** selon la méthode de synthèse **1**.
CCM gel de silice 60 F 254 Merck, éther de pétrole-AcOEt :80-20, Rf=0,58.

### Exemple 74 : 2-Méthyl-2-(3-{4-[4-méthyl-3,5-dioxo-2-(4,4,4-trifluoro-butyl)-2,3,4,5-tétrahydro [1,2,4]triazin-6-ylamino]-butyl}-phénoxy)-propionate d'éthyle (74)

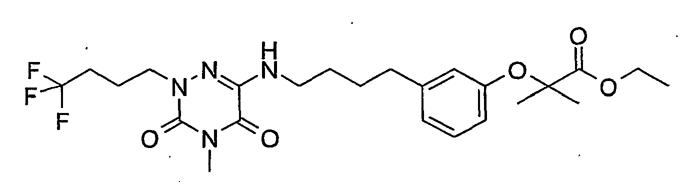

Le composé **74** (huile) est préparé à partir de la triazine **4a** et de l'intermédiaire **13m** selon la méthode de synthèse **4.**
CCM gel de silice 60 F 254 Merck, éther de pétrole-AcOEt :80-20, Rf=0,21.

### Exemple 75 : 2-{3-[4-(2-Heptyl-4-méthyl-3,5-dioxo-2,3,4,5-tétrahydro-[1,2, 4]triazin-6-ylamino)-butyl]-phénoxy}-2-méthyl-propionate d'éthyle (75)

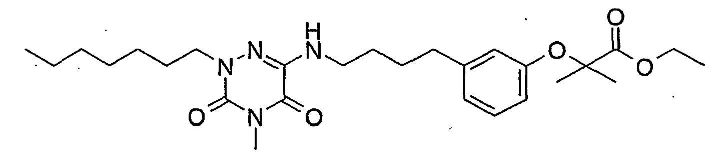

Le composé **75** (huile) est préparé à partir de la triazine **4b** et de l'intermédiaire **13m** selon la méthode de synthèse **4.**
CCM gel de silice 60 F 254 Merck, éther de pétrole-AcOEt :80-20, Rf=0,44.

### Exemple 76 : 2-Méthyl-2-(3-{4-[4-(3-méthyl-but-2-ènyl)-3,5-dioxo-2-(4,4,4-trifluoro-butyl)-2,3,4,5-tétrahydro-[1,2,4]triazin-6-ylamino]-butyl}-phénoxy)-propionate d'éthyle (76)

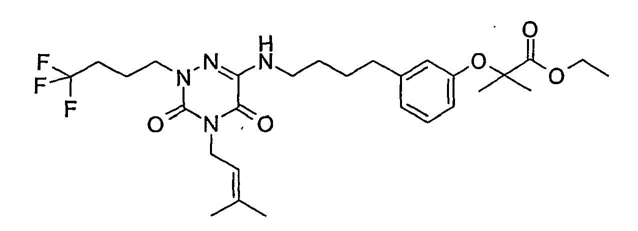

Le composé **76** (huile) est préparé à partir de la triazine **5d** et de l'intermédiaire **13m** selon la méthode de synthèse **4.**
CCM gel de silice 60 F 254 Merck, éther de pétrole-AcOEt :80-20, Rf=0,50.

### Exemple 77 : 2-Méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénoxy)-propionate d'éthyle (77)

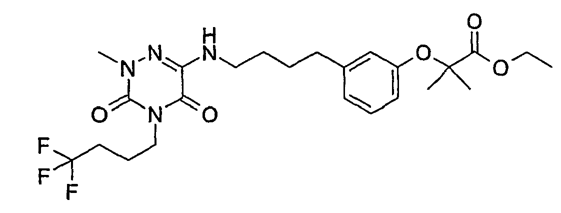

Le composé **77** (huile) est préparé à partir de la triazine **6a** et de l'intermédiaire **13k** selon la méthode de synthèse **4.**
CCM gel de silice 60 F 254 Merck, éther de pétrole-AcOEt :80-20, Rf=0,35.

### Exemple 78 : 2-Méthyl-2-{3-(4-[2-méthyl-3,5-dioxo-4-(4,4,4-trifluoro-butyl)-2,3,4,5-tétrahydro-[1,2,4]triazin-6-ylamino]-butyl}-phénoxy)-propionate de tert-butyle (78)

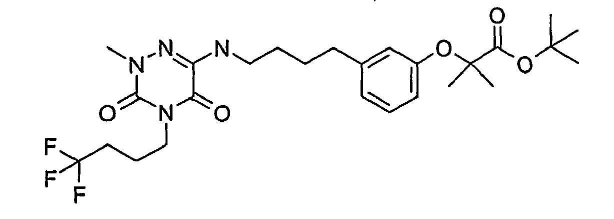

Le composé 78 (huile) est préparé à partir de la triazine **6a** et de l'intermédiaire **13m** selon la méthode de synthèse **4** en utilisant à la dernière étape le bromoisobutyrate de tert-butyle.
CCM gel de silice 60 F 254 Merck, heptane-AcOEt :60-40, Rf=0,35.

### Exemple 79 : Acide 2-méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénoxy)-propionique (79)

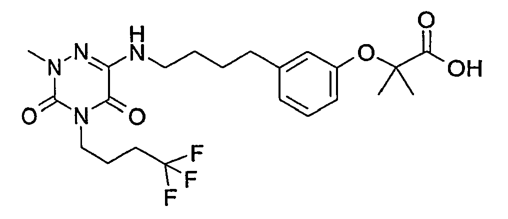

Après hydrolyse du composé **78** (acide trifluoroacétique/CH₂Cl₂, rendement=61%), le composé **79** est isolé sous forme de solide.
CCM gel de silice 60 F 254 Merck, CH₂Cl₂-MeOH:90-10, Rf=0,73. F=116°C.

### Exemple 80 : 2-(3-{4-[3,5-Dioxo-2,4-bis-(4,4,4-trifluoro-butyl)-2,3,4,5-tétrahydro-[1,2,4]triazin-6-ylamino]-butyl}-phénoxy)-2-méthyl-propionate d'éthyle (80)

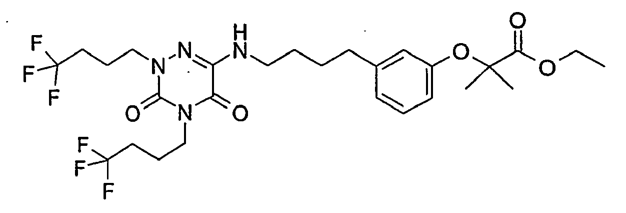

Le composé 80 (huile) est préparé à partir de la triazine **1d** et de l'intermédiaire **13m** selon la méthode de synthèse **4.**
CCM gel de silice 60 F 254 Merck, éther de pétrole-AcOEt :80-20, Rf=0,32.

### Exemple 81 : 2-(3-{4-[2-(2-Cyano-éthyl)-3,5-dioxo-4-(4,4,4-trifluoro-butyl)-2,3,4,5-tétrahydro-[1,2,4]triazin-6-ylamino]-butyl}-phénoxy)-2-méthyl-propionate d'éthyle (81)

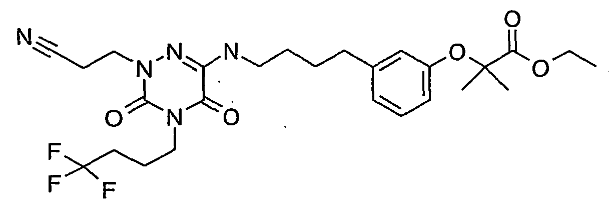

Le composé **81** (huile) est préparé à partir de la triazine **3b** et de l'intermédiaire **13k** selon la méthode de synthèse **4.**
CCM gel de silice 60 F 254 Merck, éther de pétrole-AcOEt :70-30, Rf=0,20.

### Exemple 82 : 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionate d'éthyle (82)

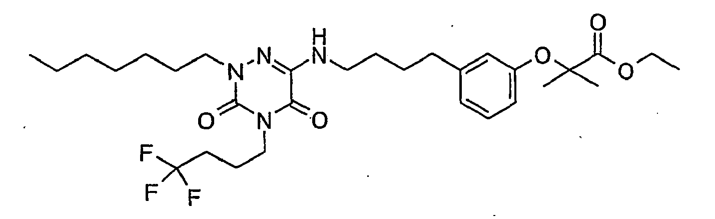

Le composé **82** (huile) est préparé à partir de la triazine **6b** et de l'intermédiaire **13k** selon la méthode de synthèse **4.**
CCM gel de silice 60 F 254 Merck, éther de pétrole-AcOEt :80-20, Rf=0,37..

### Exemple 83 : 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionate de tert-butyle (83)

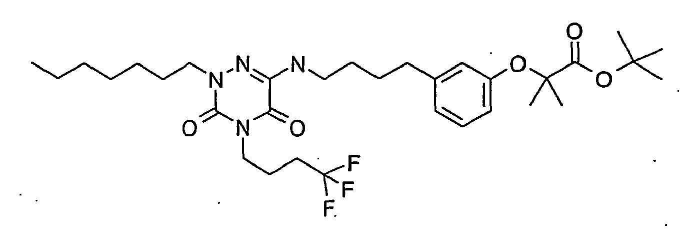

Le composé **83** (huile) est préparé à partir de la triazine **6b** et de l'intermédiaire **13m** selon la méthode de synthèse **4** en utilisant à la dernière étape le bromoisobutyrate de tert-butyle.
CCM gel de silice 60 F 254 Merck, heptane-AcOEt :60-40, Rf=0,43.

### Exemple 84 : Acide 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionique (84)

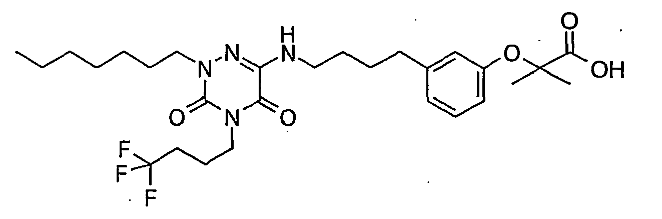

Après hydrolyse du composé **83** (acide trifluoroacétique/CH₂Cl₂, rendement=76%), le composé **84** est isolé sous forme d'huile.
CCM gel de silice 60 F 254 Merck, CH₂Cl₂-MeOH:95-5, Rf=0,39.

### Exemple 85 : 2-(3-{4-[4-Heptyl-3,5-dioxo-2-(4,4,4-trifluoro-butyl)-2,3,4,5-tétrahydro-[1,2,4]triazin-6-ylamino]-butyl}-phénoxy)-2-méthyl-propionate d'éthyle (85)

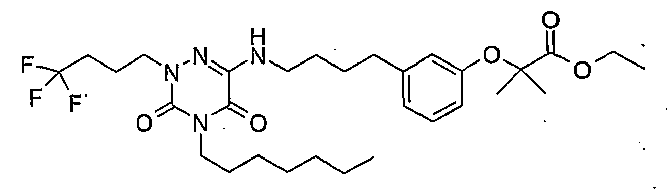

Le composé **85** (huile) est préparé à partir de la triazine **7b** et de l'intermédiaire **13m** selon la méthode de synthèse **4.**
CCM gel de silice 60 F 254 Merck, éther de pétrole-AcOEt :80-20, Rf=0,75.

### Exemple 86 : 2-(3-{4-[2-(2-Cyano-éthyl)-4-heptyl-3,5-dioxo-2,3,4,5-tétrahydro-[1,2,4]triazin-6-ylamino]-butyl}-phénoxy)-2-méthyl-propionate d'éthyle (86)

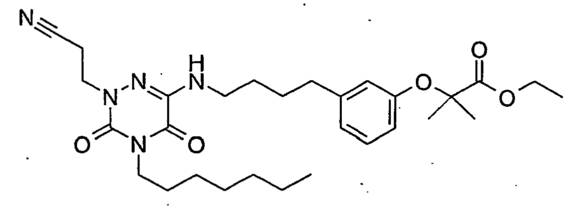

Le composé **86** (huile) est préparé à partir de la triazine **3c** et de l'intermédiaire **13m** selon la méthode de synthèse **4.**
CCM gel de silice 60 F 254 Merck, éther de pétrole-AcOEt :70-30, Rf=0,51.

### Exemple 87 : 4-(6-{4-[3-(1-Ethoxycarbonyl-1-méthyl-éthoxy)-phényl]-butylamino}-4-heptyl-3,5-dioxo-4,5-dihydro-3H-[1,2,4]triazin-2-yl)-but-2-enonate d'éthyle (87)

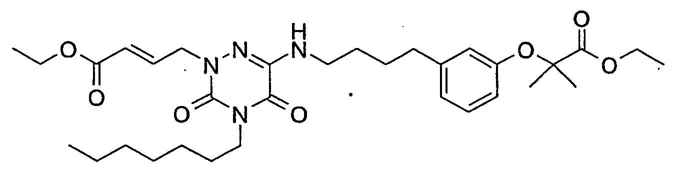

Le composé **87** (huile) est préparé selon la méthode de synthèse **6:** 0,2g (5mmol) de NaH (60% dans la paraffine) sont placés en suspension dans 10ml de DMF à 0°C sous azote. 1,4g (2,6mmol) de composé **86** dilué dans 4ml de DMF sont coulés goutte à goutte. Le mélange est agité 4,5h à température ambiante puis concentré à sec. Le résidu obtenu est repris par H₂O et extrait à l'AcOEt. Les phases organiques sont séchées sur MgSO₄ puis concentrées à sec. L'huile obtenue est purifiée par chromatographie flash sur silice (CH₂Cl₂-AcOEt :90-10) et 0,8g de solide sont isolés (rendement :63%). 78mg (1,9mmol) de NaH (60% dans la paraffine) sont placés en suspension dans 15ml de DMF à 0°C sous azote. Le solide isolé précédemment (0,8g, 1,6mmol) dilué dans 5ml de DMF est coulé goutte à goutte puis ce mélange est agité 1 h à température ambiante. 0,29ml (2,1mmol) de 4-bromo-but-2-ènoate d'éthyle est ajouté puis l'agitation est poursuivie pendant 9h. Après concentration à sec, le résidu obtenu est repris par H₂O et extrait à l'AcOEt. Les phases organiques sont séchées sur MgSO₄ puis concentrées à sec. L'huile obtenue est purifiée par chromatographie flash sur silice (éther de pétrole-AcOEt :80-20). On isole 0,6g de composé **87** sous forme d'huile (rendement=56%).
CCM gel de silice 60 F 254 Merck, éther de pétrole-AcOEt :80-20, Rf=0,33.

### Exemple 88 : 2-{3-[4-(2,4-Diheptyl-3,5-dioxo-2,3,4,5-tétrahydro-[1,2,4] triazin-6-ylamino)-butyl]-phénoxy)-2-méthyl-propionate d'éthyle (88)

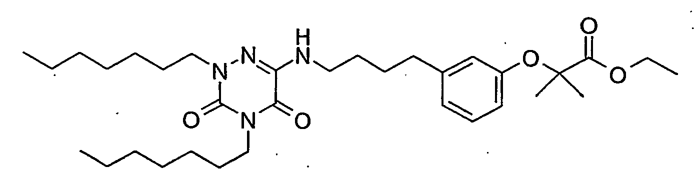

Le composé **88** (huile) est préparé à partir de la triazine **1e** et de l'intermédiaire **13k** selon la méthode de synthèse **4.**
CCM gel de silice 60 F 254 Merck, éther de pétrole-AcOEt :80-20, Rf=0,53.

### Exemple 89 : 2-{3-[4-(2-Benzyl-4-heptyl-3,5-dioxo-2,3,4,5-tétrahydro-[1,2, 4]triazin-6-ylamino)-butyl]-phénoxy)-2-méthyl-propionate d'éthyle (89)

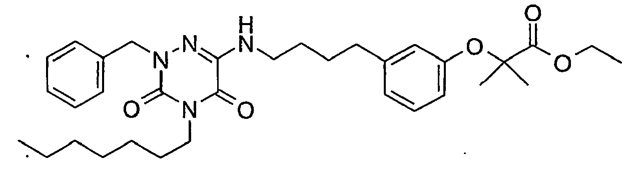

Le composé **89** (solide) est préparé à partir de la triazine **7c** et de l'intermédiaire **13k** selon la méthode de synthèse **4.**
CCM gel de silice 60 F 254 Merck, éther de pétrole-AcOEt :80-20, Rf=0,37. F=58°C.

### Exemple 90 : 2-(3-{4-[4-Benzyl-3,5-dioxo-2-(4,4,4-trifluoro-butyl)-2,3,4;5-tétrahydro-[1,2,4]triazin-6-ylamino]-butyl}-phénoxy)-2-méthyl-propionate d'éthyle (90)

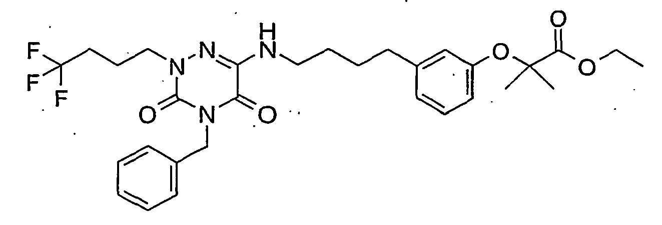

Le composé **90** (huile) est préparé à partir de la triazine **8a** et de l'intermédiaires **13k** selon la méthode de synthèse **4.**
CCM gel de silice 60 F 254 Merck, éther de pétrole-AcOEt :80-20, Rf=0,46.

### Exemple 91 : 2-{3-[4-(4-Benzyl-2-heptyl-3,5-dioxo-2,3,4,5-tetrahydro-[1,2, 4]triazin-6-ylamino)-butyl]-phénoxy}-2-méthyl-propionate d'éthyle (91)

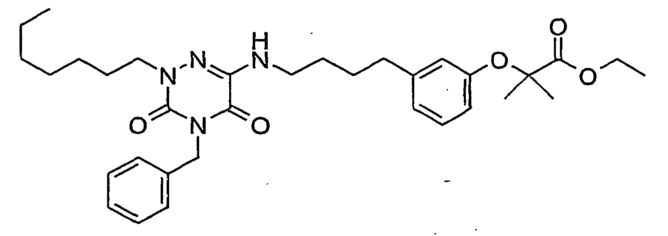

Le composé **91** (huile) est préparé à partir de la triazine **8b** et de l'intermédiaire **13k** selon la méthode de synthèse **4.**
CCM gel de silice 60 F 254 Merck, éther de pétrole-AcOEt :80-20, Rf=0,60.

### Exemple 92 : 2-(3-{5-[3,5-Dioxo-2,4-bis-(4,4,4-trifluoro-butyl)-2,3,4,5-tétrahydro-[1,2,4]triazin-6-ylamino]-pentyl}-phénoxy)-2-méthyl-propionate d'éthyle (92)

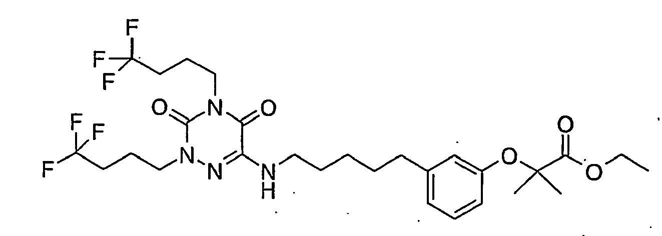

Le composé **92** (huile) est préparé à partir de la triazine **1d** et de l'intermédiaire **13n** selon la méthode de synthèse **4.**
CCM gel de silice 60 F 254 Merck, éther de pétrole-AcOEt :80-20, Rf=0,45.

### Exemple 93 : 2-Méthyl-2-(3-{4-[4-méthyl-3,5-dioxo-2-(4,4,4-trifluoro-butyl)-2,3,4,5-tetrahydro-[1,2,4]triazin-6-ylamino]-butyl}-phénylsulfanyl)-propionate d'éthyle (93)

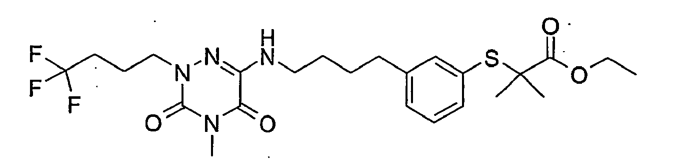

Le composé **93** (huile) est préparé à partir de la triazine **4a** et de l'intermédiaire **13f** selon la méthode de synthèse **1.**
CCM gel de silice 60 F 254 Merck, éther de pétrole-AcOEt :80-20, Rf=0,45.

### Exemple 94 : 2-{3-[4-(2-Heptyl-4-méthyl-3,5-dioxo-2,3,4,5-tétrahydro-[1,2,4]triazin-6-ylamino)-butyl]-phénylsulfanyl}-2-méthyl-propionate d'éthyle (94)

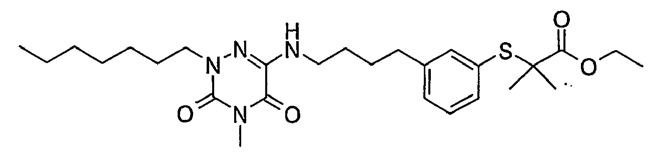

Le composé **94** (huile) est préparé à partir de la triazine **4b** et de l'intermédiaire **13f** selon la méthode de synthèse **1.**
CCM gel de silice 60 F 254 Merck, éther de pétrole-AcOEt :80-20, Rf=0,26.

### Exemple 95 : 2-Méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénylsulfanyl)-propionate d'éthyle (95)

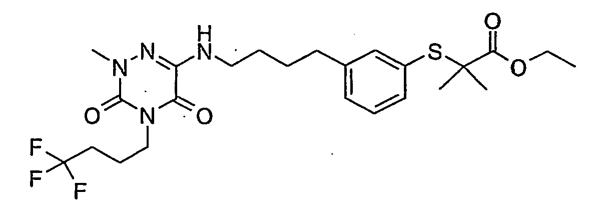

Le composé **95** (huile) est préparé à partir de la triazine **6a** et de l'intermédiaire **13f** selon la méthode de synthèse **1.**
CCM gel de silice 60 F 254 Merck, éther de pétrole-AcOEt :80-20, Rf=0,34.

### Exemple 96 : Acide 2-méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénylsulfanyl)-propionique (96)

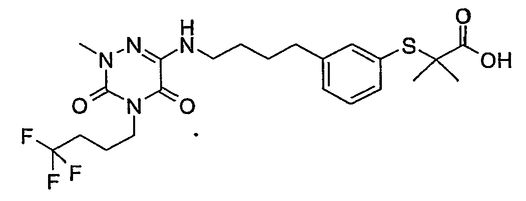

Après hydrolyse du composé **95** (BBr₃/CH₂Cl₂, rendement=49%), le composé **96** est isolé sous forme de solide.
CCM gel de silice 60 F 254 Merck, CH₂Cl₂-AcOEt :80-20, Rf=0,24. F=106°C

### Exemple 97 : 2-(3-{4-[4-Heptyl-3,5-dioxo-2-(4,4,4-trifluoro-butyl)-2,3,4,5-tétrahydro-[1,2,4]triazin-6-ylamino]-butyl}-phénylsulfanyl)-2-méthyl-propionate d'éthyle (97)

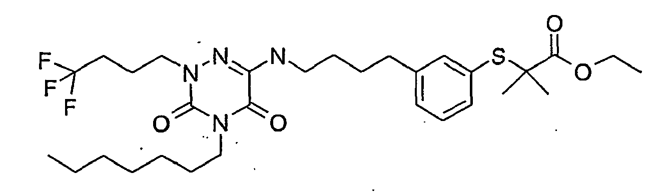

Le composé **97** (huile) est préparé à partir de la triazine **7b** et de l'intermédiaire **13f** selon la méthode de synthèse **1.**
CCM gel de silice 60 F 254 Merck, éther de pétrole-AcOEt :80-20, Rf=0,74.

### Exemple 98 : 2-{3-[4-(2,4-Diheptyl-3,5-dioxo-2,3,4,5-tetrahydro-[1,2,4] triazin-6-ylamino)-butyl]-phénylsulfanyl}-2-méthyl-propionate d'éthyle (98)

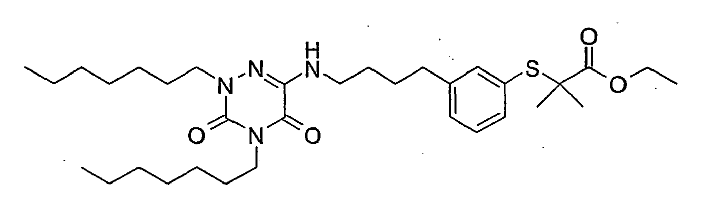

Le composé **98** (huile) est préparé à partir de la triazine **1e** et de l'intermédiaire **13f** selon la méthode de synthèse **1.**
CCM gel de silice 60 **F** 254 Merck, éther de pétrole-AcOEt :80-20, Rf=0,53.

### Exemple 99 : 2-{3-[4-(2-Benzyl-4-heptyl-3,5-dioxo-2,3,4,5-tétrahydro-[1,2, 4]triazin-6-ylamino)-butyl]-phénylsulfanyl}-2-méthyl-propionate d'éthyle (99)

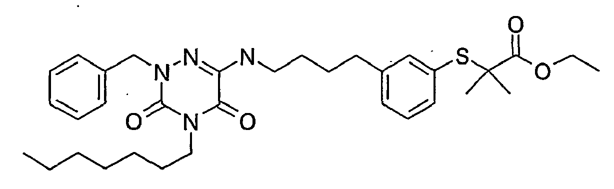

Le composé **99** (huile) est préparé à partir de la triazine **7c** et de l'intermédiaire **13f** selon la méthode de synthèse **1.**
CCM gel de silice 60 F 254 Merck, éther de pétrole-AcOEt :80-20, Rf=0,43.

### Exemple 100 : 2-(3-{4-[4-Benzyl-3,5-dioxo-2-(4,4,4-trifluoro-butyl)-2,3,4,5-tétrahydro-[1,2,4]triazin-6-ylamino]-butyl}-phénylsulfanyl)-2-méthyl-propionate d'éthyle (100)

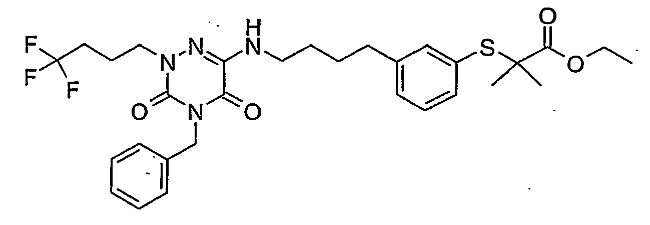

Le composé **100** (huile) est préparé à partir de la triazine **8a** et de l'intermédiaire **13f** selon la méthode de synthèse **1.**
CCM gel de silice 60 F 254 Merck, éther de pétrole-AcOEt :80-20, Rf=0,27.

### Exemple 101 : 2-{3-[4-(4-Benzyl-2-heptyl-3,5-dioxo-2,3,4,5-tétrahydro-[1,2,4]triazin-6-ylamino)-butyl]-phénylsulfanyl}-2-méthyl-propionate d'éthyle (101)

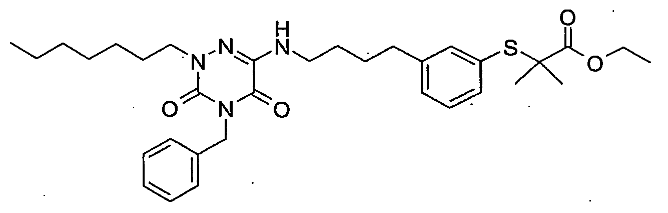

Le composé **101** (huile) est préparé à partir de la triazine **8b** et de l'intermédiaire **13f** selon la méthode de synthèse **1.**
CCM gel de silice 60 F 254 Merck, éther de pétrole-AcOEt :80-20, Rf=0,39.

### Exemple 102 : 2-Méthyl-2-(4-{2-[4-méthyl-3,5-dioxo-2-(4,4,4-trifluoro-butyl)-2,3,4,5-tétrahydro-[1,2,4]triazin-6-ylamino]-éthyl}-phénoxy)-propionate d'éthyle (102)

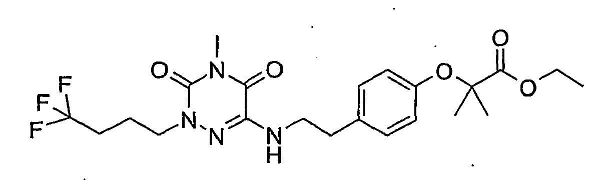

Le composé **102** (huile) est préparé à partir de la triazine **4a** et de l'intermédiaire **13a** selon la méthode de synthèse **1.**
CCM gel de silice 60 F 254 Merck, éther de pétrole-AcOEt :80-20, Rf=0,22.

### Exemple 103 : 2-{4-[2-(2-Heptyl-4-méthyl-3,5-dioxo-2,3,4,5-tétrahydro-[1,2,4] triazin-6-ylamino)-éthyl]-phénoxy}-2-méthyl-propionate d'éthyle (103)

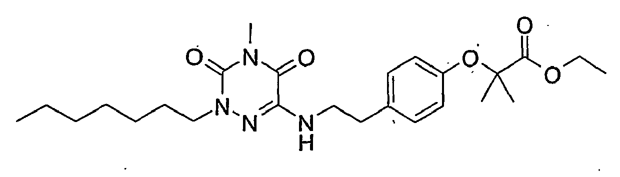

Le composé **103** (huile) est préparé à partir de la triazine **4b** et de l'intermédiaire **13a** selon la méthode de synthèse **1.**
CCM gel de silice 60 F 254 Merck, CH₂Cl₂-A_{C}OEt :90-10, Rf=0,54.

### Exemple 104 : 4-[6-{2-[4-(1-Ethoxycarbonyl-1-méthyl-éthoxy)-phényl]-éthylamino}-3,5-dioxo-4-(4,4,4-trifluoro-butyl)-4,5-dihydro-3H-[1,2,4] triazin-2-yl]-but-2-ènoate d'éthyle (104)

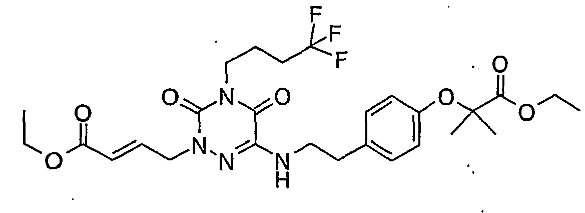

Le composé **104** (huile) est préparé à partir de la triazine **3b** et de l'intermédiaire **13a** selon la méthode de synthèse **6** en réalisant l'étape d'alkylation avec le 4-bromo-but-2-ènoate d'éthyle.
CCM gel de silice 60 F 254 Merck, éther de pétrole-AcOEt :80-20, Rf=0,34.

### Exemple 105 : 2-(4-{2-[2-(3-Cyclohexyl-propyl)-3,5-dioxo-4-(4,4,4-trifluorobutyl)-2,3,4,5-tétrahydro-[1,2,4]triazin-6-ylamino]-éthyl}-phénoxy)-2-méthyl-propionate d'éthyle (105)

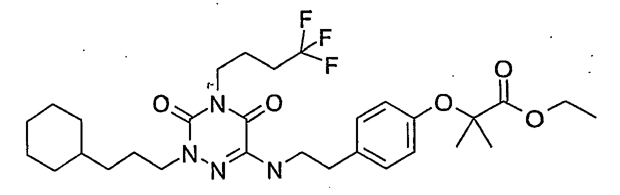

Le composé **105** (huile) est préparé à partir de la triazine **6c** et de l'intermédiaire **13a** selon la méthode de synthèse **1.**
CCM gel de silice 60 F 254 Merck, éther de pétrole-AcOEt :80-20, Rf=0,44.

### Exemple 106 : 2-{4-[2-(4-Heptyl-2-méthyl-3,5-dioxo-2,3,4,5-tétrahydro-[1, 2,4]triazin-6-ylamino)-éthyl]-phénoxy}-2-méthyl-propionate d'éthyle (106)

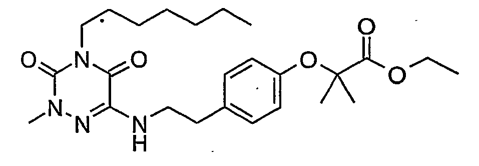

Le composé **106** (solide) est préparé à partir de la triazine **7a** et de l'intermédiaire **13a** selon la méthode de synthèse **1.**
CCM gel de silice 60 F 254 Merck, éther de pétrole-AcOEt :70-30, Rf=0,60. F=54°C..

### Exemple 107 : 2-(4-{2-[4-Heptyl-3,5-dioxo-2-(4,4,4-trifluoro-butyl)-2,3,4,5-tétrahydro-[1,2,4]triazin-6-ylamino]-éthyl}-phénoxy)-2-méthyl-propionate d'éthyle (107)

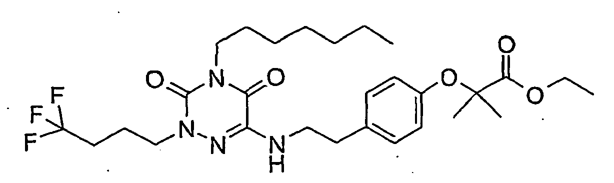

Le composé **107** (solide) est préparé à partir de la triazine **7b** et de l'intermédiaire **13a** selon la méthode de synthèse **1.**
CCM gel de silice 60 F 254 Merck, éther de pétrole-AcOEt :80-20, Rf=0,47. F=63°C.

### Exemple 108 : 2-{4-[2-(2,4-Diheptyl-3,5-dioxo-2,3,4,5-tétrahydro-[1,2,4] triazin-6-ylamino)-éthyl]-phénoxy}-2-méthyl-propionate d'éthyle (108)

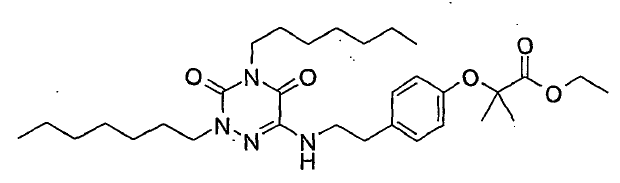

Le composé **108** (huile) est préparé à partir de la triazine **1e** et de l'intermédiaire **13a** selon la méthode de synthèse **1.**
CCM gel de silice 60 F 254 Merck, éther de pétrole-AcOEt :80-20, Rf=0,57.

### Exemple 109 : 2-{4-[2-(4-Benzyl-2-heptyl-3,5-dioxo-2,3,4,5-tétrahydro-[1,2,4] triazin-6-ylamino)-éthyl]-phénoxy}-2-méthyl-propionate d'éthyle (109)

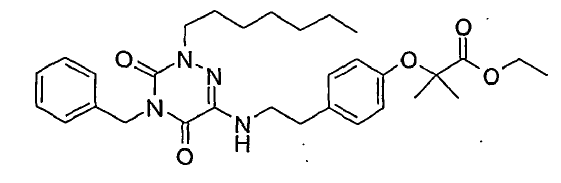

Le composé **109** (solide) est préparé à partir de la triazine **8b** et de l'intermédiaire **13a** selon la méthode de synthèse **1.**
CCM gel de silice 60 F 254 Merck, éther de pétrole-AcOEt :80-20, Rf=0,60. F=65°C.

### Exemple 110 : 2-{4-[2-(2,4-Bis-benzyloxyméthyl-3,5-dioxo-2,3,4,5-tétra-hydro-[1,2,4]triazin-6-ylamino)-éthyl]-phénoxy}-2-méthyl-propionate d'éthyle (110)

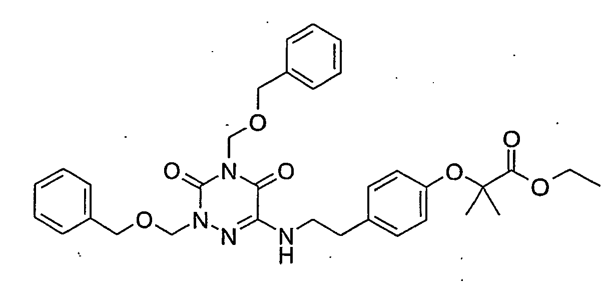

Le composé **110** (huile) est préparé à partir de la triazine **1g** et de l'intermédiaire **13a** selon la méthode de synthèse **1.**
CCM gel de silice 60 F 254 Merck, éther de pétrole-AcOEt :80-20, Rf=O,34.

### Exemple 111 : 2-(4-{2-[(2,4-Dirnéthyl-3,5-dioxo-2,3,4,5-tétrahydro-[1,2,4] triazin-6-yl)-(4,4,4-trifluoro-butyl)-amino]-éthyl}-phénoxy)-2-méthyl-propionate d'éthyle (111)

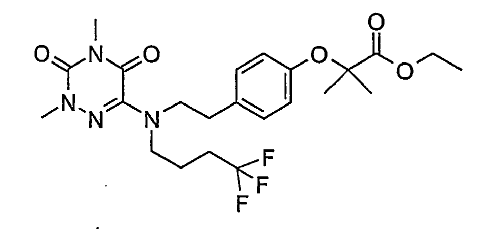

Le composé **111** est préparé selon la méthode de synthèse **5 :** 3,2g (14,7mmol) de triazine **1 b** et 5,4ml (36,8mmol) de 2-(4-méthoxy-phényl)-éthyla mine sont placés dans 30ml de nBuOH en présence de 5,1ml (36,8mmol) de triéthylamine à 130°C pendant 10,5h. Après concentration à sec, le résidu obtenu est repris par H₂O et extrait à l'AcOEt. Les phases organiques sont séchées sur MgSO₄ puis concentrées à sec. L'huile obtenue est purifiée par chromatographie flash sur silice (heptane-AcOEt :50-50) et on isole 2,3g de solide (rendement=54%). 0,48g (12mmol) de NaH (60% dans la paraffine) sont placés en suspension dans 15ml de DMF à 0°C sous azote. 2,3g (7,9mmol) de solide précédemment isolé dilué dans 5ml de DMF sont coulés goutte à goutte. Le mélange est agité 0,5h à température ambiante puis 4,7g (19,8mmol) de 1,1,1-triffluoro-4-iodo-butane sont ajoutés et l'agitation est poursuivie pendant 6,5h. Après concentration à sec, le résidu obtenu est repris par H₂O et extrait à l'AcOEt. Les phases organiques sont séchées sur MgSO₄ puis concentrées à sec. L'huile obtenue est purifiée par chromatographie flash sur silice (CH₂Cl₂-AcOEt :95-5) et 0,82g d'huile sont isolés (rendement :26%). 0,4g de cette dernière sont ensuite placés dans 15ml de CH₂Cl₂ à -60°C sous azote, une solution de BBr₃ (1 M dans CH₂Cl₂) (0,24ml dilué dans 2ml de CH₂Cl₂) est coulée goutte à goutte et le milieu réactionnel est agité 2h à température ambiante. Il est ensuite placé à 0°C et neutralisé par une solution d'HCl 1 N. La phase organique est décantée puis lavée avec 1 00ml d'eau. Après séchage sur MgSO₄, elle est concentrée à sec et on isole 0,38g de phénol correspondant (rendement quantitatif). Il est ensuite placé dans 0,5ml de bromoisobutyrate d'éthyle en présence de 0,14g (1mmol) de K₂CO₃ à 150°C pendant 5h. Après filtration et concentration à sec, le résidu obtenu est purifié par chromatographie flash sur silice (CH₂Cl₂-AcOEt :95-5) et 0,25g de composé **111** sous forme d'huile est isolé (rendement=51 %).
CCM gel de silice 60 F 254 Merck, CH₂Cl₂-AcOEt :90-10, Rf=0,65.

### Exemple 112 : 2-(4-{2-[(2,4-Diméthyl-3,5-dioxo-2,3,4,5-tétrahydro-[1,2,4] triazin-6-yl)-heptyl-amino]-éthyl}-phénoxy)-2-méthyl-propionate d'éthyle (112)

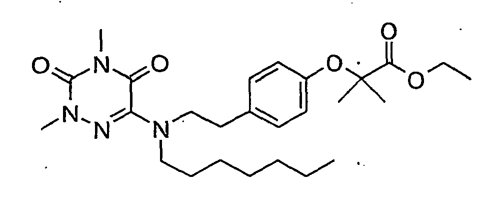

Le composé **112** (huile) est préparé à partir de la triazine **1b** et de l'intermédiaire **14a** selon la méthode de synthèse **1.**
CCM gel de silice 60 F 254 Merck, CH₂Cl₂-AcOEt :70-30, Rf=0,70.

### Exemple 113 : Acide 2-(4-{2-[(2,4-diméthyl-3,5-dioxo-2,3,4,5-tétrahydro-[1,2,4] triazin-6-yl)-heptyl-amino]-éthyl}-phénoxy)-2-méthyl-propionique (113)

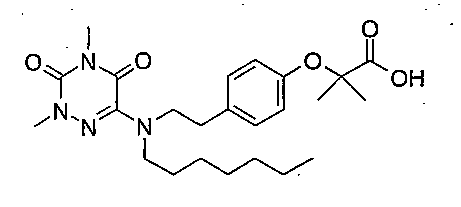

Le composé **113** est préparé à partir de la triazine **1b** et de la 2-(4-méthoxy-phényl)-éthylamine selon la méthode de synthèse **5** en alkylant l'azote avec le 1-bromoheptane et en utilisant le bromoisobutyrate de tert-butyle. Après hydrolyse avec l'acide trifluoroacétique dans CH₂Cl₂, le composé **113** est isolé sous forme d' huile.
CCM gel de silice 60 F 254 Merck, CH₂Cl₂-MeOH:90-10, Rf=0,43.

### Exemple 114 : 2-(4-{2-[(2,4-Diméthyl-3,5-dioxo-2,3,4,5-tétrahydro-[1,2,4] triazin-6-yl)-phénéthyl-amino]-éthyl}-phénoxy)-2-méthyl-propionate d'éthyle (114)

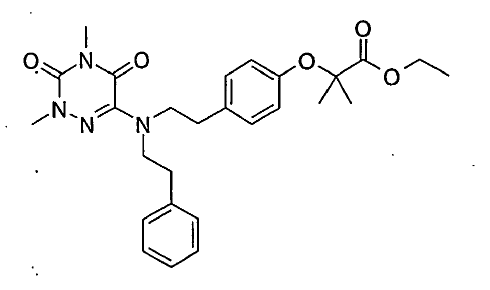

Le composé **114** (huile) est préparé à partir de la triazine **1 b** et de l'intermédiaire **14b** selon la méthode de synthèse **1.**
CCM gel de silice 60 F 254 Merck, CH₂Cl₂-AcOEt :90-10, Rf=0,74.

### Exemple 115 : 2-(4-{2-[(2,4-Diméthyl-3,5-dioxo-2,3,4,5-tétrahydro-[1,2,4] triazin-6-yl)-(3-phényl-propyl)-amino]-éthyl}-phénoxy)-2-méthyl-propionate d'éthyle (115)

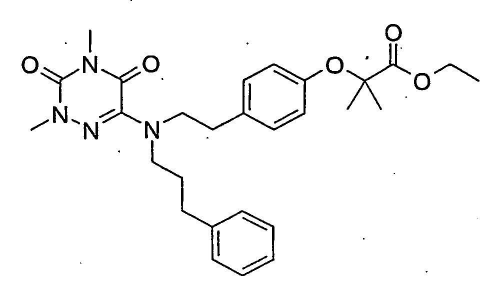

Le composé **115** (huile) est préparé à partir de la triazine **1b** et de l'intermédiaire **14c** selon la méthode de synthèse **1.**
CCM gel de silice 60 F 254 Merck, CH₂Cl₂-A_{C}OEt :90-10, Rf=0,63.

### Exemple 116 : 2-(4-{2-[(4-Heptyl-2-méthyl-3,5-dioxo-2,3,4,5-tétrahydro-[1,2,4]triazin-6-yl)-phénéthyl-amino]-éthyl}-phénoxy)-2-méthyl-propionate d'éthyle (116)

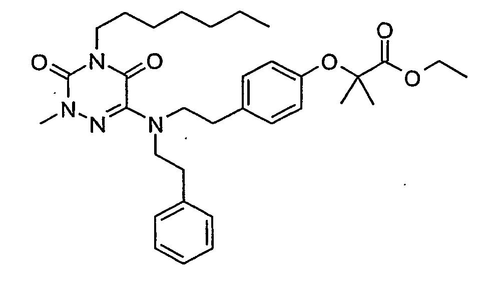

Le composé **116** (huile) est préparé à partir de la triazine **7a** et de l'intermédiaire **14b** selon la méthode de synthèse **1.**
CCM gel de silice 60 F 254 Merck, CH₂Cl₂, Rf=0,52.

### Exemple 117 : 2-(4-{2-[(4-Heptyl-2-méthyl-3,5-dioxo-2,3,4,5-tétrahydro-[1,2,4]triazin-6-yl)-(3-phényl-propyl)-amino]-éthyl}-phénoxy)-2-méthyl-propionate d'éthyle (117)

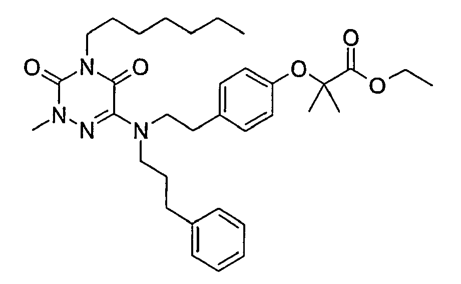

Le composé **117** (huile) est préparé à partir de la triazine **7a** et de l'intermédiaire **14c** selon la méthode de synthèse **1.**
CCM gel de silice 60 F 254 Merck, CH₂Cl₂, Rf=0,46.

### Exemple 118 : Acide 2-méthyl-2-(4-{2-[4-méthyl-3,5-dioxo-2-(4,4,4-trifluorobutyl)-2,3,4,5-tétrahydro-[1,2,4]triazin-6-ylamino]-éthyl}-phénylsulfanyl)-propionique (118)

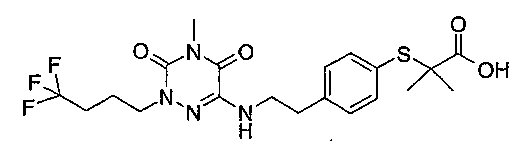

Le composé **118** est préparé à partir de la triazine **4a** et de l'intermédiaire **13i** selon la méthode de synthèse **1.** Après hydrolyse avec l'acide trifluoroacétique dans CH₂Cl₂, le composé **118** est isolé sous forme de solide.
CCM gel de silice 60 F 254 Merck, AcOEt, Rf=0,42. F=128°C_{.}

### Exemple 119 : 2-{4-[2-(2-Heptyl-4-méthyl-3,5-dioxo-2,3,4,5-tétrahydro-[1,2,4]triazin-6-ylamino)-éthyl]-phénylsulfanyl}-2-méthyl-propionate d'éthyle (119)

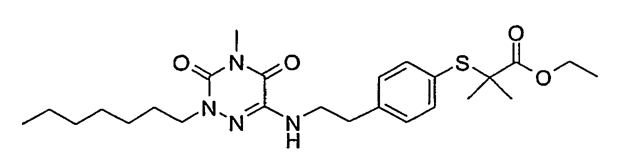

Le composé **119** (huile) est préparé à partir de la triazine **4b** et de l'intermédiaire **13g** selon la méthode de synthèse **1.**
CCM gel de silice 60 F 254 Merck, éther de pétrole-AcOEt :80-20, Rf=0,60.

### Exemple 120 : Acide 2-{4-[2-(2-Heptyl-4-méthyl-3,5-dioxo-2,3,4,5-tétrahydro-[1,2,4]triazin-6-ylamino)-éthyl]-phénylsulfanyl}-2-méthyl-propionique (120)

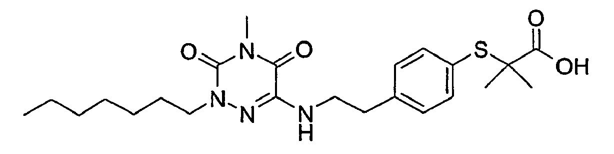

Le composé **120** est préparé à partir de la triazine **4b** et de l'intermédiaire **13i** selon la méthode de synthèse **1.** Après hydrolyse avec l'acide trifluoroacétique dans CH₂Cl₂, le composé **120** est isolé sous forme de solide.
CCM gel de silice 60 F 254 Merck, CH₂Cl₂-MeOH :90-10, Rf=0,34. F=70°C.

### Exemple 121 : Acide 2-{4-[2-(4-butyl-2-heptyl-3,5-dioxo-2,3,4,5-tétrahydro-[1,2,4]triazin-6-ylamino)-éthyl]-phénylsulfanyl}-2-méthyl-propionique (121)

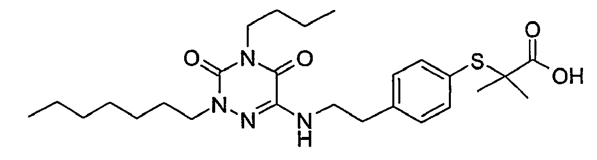

Le composé **121** est préparé à partir de la triazine **5c** et de l'intermédiaire **13i** selon la méthode de synthèse **1.** Après hydrolyse avec l'acide trifluoroacétique dans CH₂Cl₂, le composé **121** est isolé sous forme d'huile.
CCM gel de silice 60 F 254 Merck, CH₂Cl₂-MeOH :98-2, Rf=0,46.

### Exemple 122 : 2-{4-[2-(4-Heptyl-2-méthyl-3,5-dioxo-2,3,4,5-tétrahydro-[1,2,4] triazin-6-ylamino)-éthyl]-phénylsulfanyl}-2-méthyl-propionate d'éthyle (122)

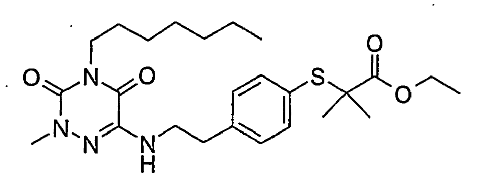

Le composé **122** (solide) est préparé à partir de la triazine **7a** et de l'intermédiaire **13g** selon la méthode de synthèse **1.**
CCM gel de silice 60 F 254 Merck, éther de pétrole-AcOEt :80-20, Rf=0,67. F=49°C.

### Exemple 123 : Acide 2-(4-{2-[4-Heptyl-3,5-dioxo-2-(4,4,4-trifluoro-butyl)-2,3,4,5-tétrahydro-[1,2,4]triazin-6-ylamino]-éthyl}-phénylsulfanyl)-2-méthyl-propionique (123)

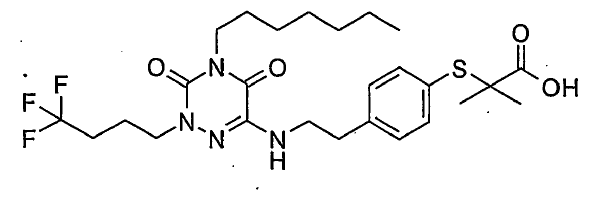

Le composé **123** est préparé à partir de la triazine **7b** et de l'intermédiaire **13i** selon la méthode de synthèse **1.** Après hydrolyse avec l'acide trifluoroacétique dans CH₂Cl₂, le composé **123** est isolé sous forme de solide.
CCM gel de silice 60 F 254 Merck, éther de pétrole-AcOEt :80-20, Rf=0,19. F=86°C.

### Exemple 124 : 2-{4-[3-(2-Heptyl-4-méthyl-3,5-dioxo-2,3,4,5-tétrahydro-[1,2,4]triazin-6-ylamino)-propyl]-phénylsulfanyl}-2-méthyl-propionate d'éthyle (124)

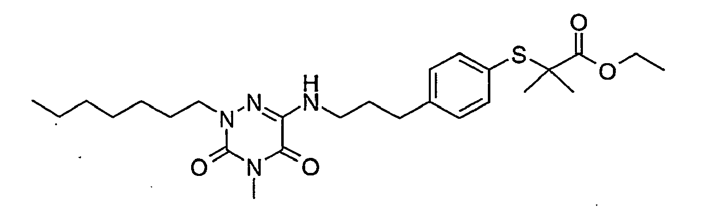

Le composé **125** (huile) est préparé à partir de la triazine **4b** et de l'intermédiaire **13h** selon la méthode de synthèse **1.**
CCM gel de silice 60 F 254 Merck, éther de pétrole-AcOEt :80-20, Rf=0,20.

### EVALUATION PHARMACOLOGIQUE

### In vitro

Activation de la transcription (transactivation) du gène rapporteur contrôlé par des éléments de réponse spécifiques après liaison du ligand au récepteur (reporter gene assay)..

Ces expériences sont réalisées selon J.M. Lehmann et al. (J. Biol. Chem 1995; 270 :12953-12956) avec quelques modifications. Des cellules Cos-7 (ATCC, CRL-1651) sub-confluentes sont transfectées avec (i) les récepteurs chimériques contenant le domaine de liaison au ligand de PPARα ou PPARγ ou PPARδ humain fusionné au domaine de liaison à l'ADN de la galactosidase de levure (Gal-4) d'une part, (ii) le plasmide rapporteur contenant cinq copies de l'élément de réponse de Gal-4 en amont du promoteur de la thymidine kinase adjacent au gène de la luciférase (p5xUAS-tk-Luc) d'autre part. Après 24 heures, ces cellules sont traitées pendant les 24 heures suivantes par les composés ou leur véhicule et l'activité luciférase est évaluée après extraction cellulaire selon les recommandations du fournisseur (Promega).

Les résultats ont été reportés dans le tableau 17 ci-après, dans lequel la mention "hit" désigne un composé dont le niveau de transactivation est significatif sans pour autant permettre de définir une EC₅₀, la mention"nd" signifie que la mesure n'a pas été réalisée.

**Tableau 17 : transactivation de gène rapporteur avec les différents sous-types de PPAR d'origine humaine**

| **Exemples** | **hPPAR - GAL4 alpha EC₅₀ (µM)** | **hPPAR - GAL4 gamma EC₅₀ (µM)** | **hPPAR - GAL4 delta EC₅₀ (µM)** |
|---|---|---|---|
| **Acide fenofibrique** | 16,9 | 65,2 | >100 |
| **Rosiglitazone** | 0 | 1,12 | 0 |
| **Pioglitazone** | > 10 | 4,77 | 0 |
| 1 | 3-10 | 0 | 0 |
| 2 | >10 | ~10 | 0 |
| 3 | -3 | 0 | 0 |
| 4 | >10 | 0 | 0 |
| 5 | -3 | ~10 | 0 |
| 6 | ~0,3 | 0 | 0 |
| 7 | 0,3-1 | 1-3 | 0 |
| 8 | ~0,3 | 3-10. | 0 |
| 9 | 3-10 | ~10 | 0 |
| 10 | 0,3-1 | ~1 | 0 |
| 11 | 0,3-1 | 1-3 | 0 |
| 12 | ~0,03 | 1-3 | hit |
| 13 | 0,01-0,03 | 0,1-0, 3 | ~3 |
| 14 | 0,01-0,03 | ~1 | -10 |
| 15 | 0,001-0,003 | 0,1-0,3 | hit |
| 16 | -0,1 | 3-10 | 0 |
| 17 | 0,3-1 | -0,3 | 0 |
| 18 | 0,03-0,1 | ~1 | 0 |
| 19 | ~3 | 3-10 | 0 |
| 20 | hit | hit | 0 |
| 21 | 3-10 | 0 | 0 |
| 22 | 10 | hit | 0 |
| 23 | 1 | hit | 0 |
| 24 | >10 | 0 | 0 |
| 25 | hit | >10 | hit |
| 26 | >10 | 0 | 0 |
| 27 | -3 | 3-10 | 3-10 |
| 28 | ~10 | hit | 0 |
| 29 | 3-10 | 3-10 | 3-10 |
| 30 | 0,03-0,1 | >10 | hit |
| 31 | ~0,1 | ~-1 | 3-10 |
| 32 | 1-3 | hit | 0 |
| 33 | -3 | -3 | 0 |
| 34 | >10 | >10 | 0 |
| 35 | 0,03-0,1 | 3-10 | ~10 |
| 36 | 0,3-1 | 3-10 | hit |
| 37 | >10 | ~10 | 0 |
| 38 | >10 | >10 | 0 |
| 39 | 0,1-0,3 | 3-10 | ~3 |
| 40 | 3-10 | -10 | >10 |
| 41 | ~0,1 | ~10 | 1-3 |
| 42 | ~1 | >10 | 1-3 |
| 43 | ~3 | hit | 0 |
| 44 | >10 | 3-10 | 0 |
| 45 | 1-3 | -3 | hit |
| 46 | 0,3-1 | 0 | 0 |
| 47 | ~0,3 | ~0,3 | hit |
| 48 | 0,3-1 | 0,3-1 | ~3 |
| 49 | >10 | >10 | 0 |
| 50 | >10 | -10 | 0 |
| 51 | 3-10 | >10 | 0 |
| 52 | hit | 0 | 0 |
| 53 | 1-3 | 0 | 0 |
| 54 | >10 | 0 | 0 |
| 55 | >10 | 0 | 0 |
| 56 | hit | hit | 0 |
| 57 | ~3 | 1-3 | hit |
| 58 | >10 | 0 | 0 |
| 59 | 1-3 | nd | 0 |
| 60 | >10 | hit | 0 |
| 61 | ~3 | nd | 0 |
| 62 | >10 | 0 | 0 |
| 63 | 0 | hit | 0 |
| 64 | >10 | 3-10 | hit |
| 65 | 0,1-0,3 | >10 | 0 |
| 66 | 0,03-0,1 | >10 | -10 |
| 67 | ~1 | nd | 0 |
| 68 | 0,1-0,3 | 3-10 | 0 |
| 69 . | 1-3 | -10 | 0 |
| 70 | ~1 | ~10 | 0 |
| 71 | ~1 | hit | 0 |
| 72 | 3-10 | 3-10 | 0 |
| 73 | 0,1-0,3 | nd | 0 |
| 74 | ~ 1 | 3-10 | 0 |
| 75 | 3-10 | 3-10 | 0 |
| 76 | 0,01-0,03 | 1-3 | 3-10 |
| 77. | ~0,3 | 3-10 | 0 |
| 78 | >10 | 0 | 0 |
| 79 | ~0,03 | 3-10 | 0 |
| 80 | 0,01-0,03 | 0,3-1 | >10 |
| 81 | 0,3-1 | >10 | 0 |
| 82 | -0,01 | 0,1-0,3 | hit |
| 83 | >10 | 0 | 0 |
| 84 | 0,003-0,01 | ~0,3 | 1-3 |
| 85 | ~0,03 | 1-3 | hit |
| 86 | >10 | ~1 | hit |
| 87 | ~0,3 | 0,1-0,3 | hit |
| 88 | ~0,3 | ~10 | 0 |
| 89 | >10 | 1-3 | 3-10 |
| 90 | ~0,1 | ~10 | 0 |
| 91 | 0,3-1 | 3-10 | 0 |
| 92 | 0,003-0,01 | 3-10 | 0 |
| 93 | >10 | hit | 0 |
| 94 | 3-10 | 3-10 | 0 |
| 95 | 1-3 | >10 | 0 |
| 96 | 0,03-0,1 | -10 | >10 |
| 97 | 1-3 | hit | 0 |
| 98 | >10 | 0 | 0 |
| 99 | >10 | 3-10 | 0 |
| 100 | 1-3 | >10 | 0 |
| 101 | 1-3 | 0 | 0 |
| 102 | >10 | hit | 0 |
| 104 | 1-3 | > 10 | 0 |
| 105 | 1-3 | ~10 | 0 |
| 106 | ~1 | nd | >10 |
| 107 | ~1 | hit | 0 |
| 108 | >10 | hit | 0 |
| 109 | ~1 | nd | 3-10 |
| 110 | 1-3 | hit | >10 |
| 111 | 0 | hit | 0 |
| 112 | 1-3 | ~10 | 1-3 |
| 113 | 0,3-1 | 3-10 | 0,3-1 |
| 114 | ~10 | nd | 0 |
| 115 | ~3 | nd | >10 |
| 116 | ~10 | nd | 0 |
| 117 | ~10 | nd | 0 |
| 118 | 1-3 | ~10 | 0 |
| 119 | 0,3 | 0,3 | hit |
| 120 | 0,3-1 | 1-3 | hit |
| 121 | 0,03-0,1 | 1-3 | hit |
| 122 | ~0,3 | nd | >10 |
| 123 | 0,1-0,3 | ~10 | 0 |
| 124 | 0,3-1 | >10 | 0 |

### In vivo

Normalisation des paramètres métaboliques (cholestérol, triglycérides plasmatiques) chez le rat mâle insulino-résistant (Ico : ZUCKER - fa/fa) à jeun (depuis 16-18 heures) après traitement par voie orale, une fois par jour pendant quatre jours, avec les composés à évaluer ou leur véhicule d'administration.

Ces paramètres métaboliques sont mesurés par spectrophotométrie en début et en fin de traitement sur chaque animal.

**Tableau 18 : Normalisation des paramètres métaboliques**

| **Exemples** | **Plasma triglycérides** | **Plasma cholestérol** |
|---|---|---|
| 11 | inactif à 2,5 mg/kg -37% à 10 mg/kg | -25% à 2,5 mg/kg -25% à 10 mg/kg |
| 13 | inactif à 2,5 mg/kg -28% à 10 mg/kg | -8% à 2,5 mg/kg -12% à 10 mg/kg |
| 15 | -22% à 2,5 mg/kg -63% à 10 mg/kg | -4% à 2,5 mg/kg -8% à 10 mg/kg |
| 16 | inactif à 2,5 mg/kg -15% à 10 mg/kg | -14% à 2,5 mg/kg -25% à 10 mg/kg |
| 17 | -37% à 10 mg/kg | actif à 10 mg/kg |
| 41 | inactif à 10 mg/kg | -28% à 10 mg/kg |
| 45 | inactif à 10 mg/kg | -16% à 10 mg/kg |
| 46 | -32% à 10 mg/kg | -13% à 10 mg/kg |
| 47 | -45% à 10 mg/kg | -25% à 10 mg/kg |
| 53 | inactif à 10 mg/kg | -7% à 2,5 mg/kg -28% à 10 mg/kg |
| 59 | -13% à 2,5 mg/kg | -37% à 2,5 mg/kg |
| 65 | inactif à 2,5 mg/kg -13% à 10 mg/kg | -13% à 2,5 mg/kg -23% à 10 mg/kg |
| 66 | inactif à 10 mg/kg | -9% à 10 mg/kg |
| 67 | -23% à 10 mg/kg | -14% à 10 mg/kg |
| 68 | actif à 10 mg/kg | -21 % à 10 mg/kg |
| 72 | inactif à 10 mg/kg | -21 % à 10 mg/kg |
| 73 | inactif à 10 mg/kg | -14% à 10 mg/kg |
| 74 | inactif à 2,5 mg/kg -26% à 10 mg/kg | -18% à 2,5 mg/kg -29% à 10 mg/kg |
| 75 | actif à 10 mg/kg | -19% à 2,5 mg/kg -13% à 10 mg/kg |
| 76 | -7% à 10 mg/kg | -16% à 2,5 mg/kg -22% à 10 mg/kg |
| 77 | actif à 10 mg/kg | -29% à 2,5 mg/kg -35% à 10 mg/kg |
| 79 | -55% à 2,5 mg/kg | -32% à 2,5 mg/kg |
| 80 | inactif à 10 mg/kg | -16% à 10 mg/kg |
| 81 | -17% à 10 mg/kg | -12% à 10 mg/kg |
| 82 | actif à 10 mg/kg | -13% à 10 mg/kg |
| 84 | inactif à 2,5 mg/kg -7% à 10 mg/kg | inactif à 2,5 mg/kg -13% à 10 mg/kg |
| 85 | actif à 10 mg/kg | -21 % à 10 mg/kg |
| 92 | inactif à 10 mg/kg | -18% à 10 mg/kg |
| 95 | inactif à 10 mg/kg | -18% à 2,5 mg/kg -40% à 10 mg/kg |
| 96 | -26% à 2,5 mg/kg -60% à 10 mg/kg | -28% à 2,5 mg/kg -21 % à 10 mg/kg |
| 112 | -31 % à 10 mg/kg | -43% à 10 mg/kg |
| 113 | inactif à 2,5 mg/kg -26% à 10 mg/kg | -30% à 2,5 mg/kg -40% à 10 mg/kg |
| 119 | actif à 2,5 mg/kg -29% à 10 mg/kg | -21 % à 2,5 mg/kg -32% à 10 mg/kg |
| 120 | -27% à 2,5 mg/kg -73% à 10 mg/kg | -32% à 2,5 mg/kg -33 % à 10 mg/kg |
| 121 | -24% at 2.5 mg/kg | -7% at 2.5 mg/kg |
| 122 | -35% à 10 mg/kg | -22% à 10 mg/kg |
| 123 | -6% à 2,5 mg/kg -20% à 10 mg/kg | -8% à 2,5 mg/kg -14% à 10 mg/kg |

Ainsi, la présente invention concerne, à titre de médicaments nouveaux utilisables dans le traitement des maladies nécessitant des agonistes des récepteurs PPAR alpha et/ou PPAR gamma, les composés de formule **I** précédemment définis. Ces composés sont utiles dans la prévention et le traitement des maladies telles que les dyslipidémies diabétiques, l'hypertriglycéridémie, l'hypercholestérolémie, l'hyper-insulinémie, l'hyperglycémie, le syndrome métabolique, l'obésité, l'athérosclérose, ou encore en dermatologie, dans des pathologies à composante inflammatoire ou résultant d'une différentiation cellulaire anormale, ainsi que dans le traitement des maladies telles que le psoriasis, l'acné, les dermatites atopiques, le vieillissement cutané, le photovieillissement.

Enfin, l'invention concerne des compositions pharmaceutiques contenant à titre de principe actif au moins un composé de formule **I** précédemment définis, de préférence en association avec tout excipient approprié.

## Revendications

1. Dérivés de la 3,5-dioxo-(2H,4H)-1,2,4-triazine de formule générale **I** 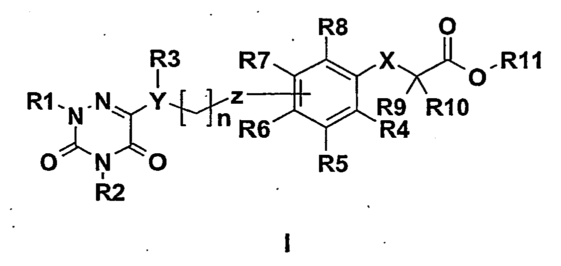 dans laquelle
- **R₁ et R₂** peuvent être identiques ou différents et représentent un radical alkyle ou alkènyle linéaire ou branché en C₁-C₇, un radical alkyle en C₁-C₆ substitué par des groupements tels que, trifluorométhyle, cycloalkyle en C₅-C₆, nitrile, alkoxycarbonylvinyle en C₁-C₄, hydroxycarbonylvinyle, alkoxycarbonyle en C₁-C₄, carboxylate, benzyloxy ou phényle (pour lesquels le noyau phényle est éventuellement substitué par un ou plusieurs groupements tels que alcoyle en C₁-C₄, alkoxy en C₁-C₄, nitro, halogène, trifluorométhyle).
- **YR₃** représente l'oxygène ou **NR₃** pour lequel **R₃** représente l'hydrogène, un radical alcoyle ou alkènyle linéaire ou branché en C₁-C₇, un radical alkyle en C₁-C₆ substitué par des groupements tels que, trifluorométhyle ou phényle (pour lesquels le noyau phényle est éventuellement substitué par un ou plusieurs groupements tels que alcoyle en C₁-C₄, alkoxy en C₁-C₄, nitro, halogène, trifluorométhyle)
- **Z** représente un atome d'oxygène ou un atome de carbone pouvant être lié aux positions ortho, méta ou para du groupement phényle de la formule **I**
- **n** peut-être égal à 0 à 5 lorsque **Z**=C ou 2 à 4 lorsque **Z**=O,
- **X** représente l'oxygène ou le soufre
- **R₄, R₅, R₆, R₇** et **R₈** représentent l'hydrogène ou le fluor,
- **R₉, R₁₀** et **R₁₁** représentent l'hydrogène ou un groupement alkyle en C₁-C₅ linéaire ou branché
ainsi que les sels d'addition avec les bases pharmaceutiquement acceptables, et les différents énantiomères des composés possédant des carbones asymétriques, ainsi que leurs mélanges en toutes proportions incluant notamment les mélanges racémiques.

2. Dérivés de la 3,5-dioxo-(2H,4H)-1,2,4-triazine de formule générale **I** selon la revendication 1 dans laquelle :
- **R₁** et **R₂** représentent indépendamment, l'un de l'autre, un radical alkyle ou alkènyle linéaire ou branché en C₁-C₇, un radical alkyle en C₁-C₆ substitué par des groupements tels que, trifluorométhyle, cycloalkyle en C₆, nitrile, phényle (pour lesquels le noyau phényle est éventuellement substitué par un ou plusieurs groupements tels que alcoyle en C₁-C₄, alkoxy en C₁-C_{4,} nitro, halogène, trifluorométhyle),
- **YR₃** représente l'oxygène ou **NR₃** pour lequel **R₃** représente l'hydrogène, un radical alcoyle ou alkènyle linéaire ou branché en C₁-C₇, un radical alkyle en C₁-C₆ substitué par des groupements tels que, trifluorométhyle ou phényle,
- **Z** représente un atome d'oxygène ou un atome de carbone pouvant être lié aux positions ortho, méta ou para du groupement phényle de la formule **I**
- **n** peut-être égal à 0 à 5 **lorsque Z=**C ou 2 à 4 lorsque **Z=**O,
- **X** représente l'oxygène ou le soufre
- **R₄, R₅, R₆, R₇** et **R₈** représentent l'hydrogène ou le fluor,
- **R₉, R₁₀** et **R₁₁** représentent l'hydrogène ou un groupement alkyle en C₁-C₅ linéaire ou branché

3. Dérivés de la 3,5-dioxo-(2H,4H)-1,2,4-triazine de formule générale **I** selon l'une des revendications 1 et 2 dans laquelle :
- **R₁ et R₂** représentent indépendamment l'un de l'autre, un radical alkyle ou alkènyle linéaire ou branché en C₁-C₇, un radical alkyle en C₁-C₆ substitué par des groupements tels que, trifluorométhyle ou nitrile,
- **YR₃** représente l'oxygène ou **NR₃** pour lequel **R₃** représente l'hydrogène ou un radical alcoyle linéaire ou branché en C₁-C₇,
- **Z** représente un atome de carbone pouvant être lié aux positions ortho, méta ou para du groupement phényle de à formule **I**
- **n** peut-être égal à 0 à 5,
- **X** représente l'oxygène ou le soufre
- **R₄, R₅, R₆, R₇** et **R₈** représentent l'hydrogène ou le fluor,
- **R₉, R₁₀** et **R₁₁** représentent l'hydrogène ou un groupement alkyle en C₁-C₅ linéaire ou branché, en particulier **R₉** et **R₁₀** représentent le groupement méthyle et **R₁₁** l'hydrogène ou le groupement éthyle,

4. Dérivés de la 3,5-dioxo-(2H,4H)-1,2,4-triazine de formule générale 1 selon l'une des revendications 1 à 3 dans laquelle :
- **R₁** et **R₂** représentent indépendamment l'un de l'autre, un radical alkyle ou alkènyle linéaire ou ramifié en C₁-C₇ éventuellement substitué en bout de chaîne par un groupement trifluorométhyle,
- **YR₃** représente l'oxygène ou **NR₃** pour lequel **R₃** représente l'hydrogène ou un radical alcoyle linéaire ou branché en C₁-C₇,
- **Z** représente un atome de carbone pouvant être lié aux positions méta ou para du groupement phényle de la formule **I**
- **n** peut-être égal à 1 à 5,
- **X** représente l'oxygène ou le soufre
- **R₄** à **R₈** représentent l'hydrogène,
- **R₉** et **R₁₀** représentent un radical méthyl
- **R₁₁** représente l'hydrogène ou un radical éthyl

5. Composés de formule générale **I** selon la revendication 1) **caractérisés en ce qu'**ils sont choisis parmi :
1. 2-{2-[2-(4-Butyl-2-méthyl-3,5-dioxo-2,3,4,5-tétrahydro-[1,2,4]triazin-6-yloxy)-éthyl]-phénoxy}-2-méthyl-propionate d'éthyle
2. 2-{3-[2-(2-Heptyl-4-méthyl-3,5-dioxo-2,3,4,5-tétrahydro-[1,2,4]triazin-6-yloxy)-éthyl]-phénoxy}-2-méthyl-propionate d'éthyle
3. 2-Méthyl-2-(3-{2-[2-méthyl-3,5-dioxo-4-(4,4,4-trifluoro-butyl)-2,3,4,5-tétrahydro-[1,2,4]triazin-6-yloxy]-éthyl}-phénoxy)-propionate d'éthyle
4. 2-Méthyl-2-(3-{3-[4-m éthyl-3, 5-d ioxo-2-(4,4,4-trifluoro-butyl )-2,3,4,5-tétrahydro-[1,2,4]triazin-6-yloxy]-propyl}-phénoxy)-propionate d'éthyle
5. 2-{3-[3-(2-Heptyl-4-méthyl-3,5-dioxo-2,3,4,5-tétrahydro-[1,2,4]triazin-6-yloxy)-propyl]-phénoxy}-2-méthyl-propionate d'éthyle
6. 2-Méthyl-2-(3-{3-[2-méthyl-3,5-dioxo-4-(4,4,4-trifluoro-butyl)-2,3,4,5-tétrahydro-[1,2,4]triazin-6-yloxy]-propyl}-phénoxy)-propionate d'éthyle
7. 2-{3-[3-(4-Heptyl-2-méthyl-3,5-dioxo-2,3,4,5-tétrahydro-[1,2,4]triazin-6-yloxy)-propyl]-phénoxy}-2-méthyl-propionate d'éthyle
8. 2-(3-{3-[4-Heptyl-3,5-dioxo-2-(4,4,4-trifluoro-butyl)-2,3,4,5-tétrahydro-[1,2,4]triazin-6-yloxy]-propyl}-phénoxy)-2-méthyl-propionate d'éthyle ,
9. 2-Méthyl-2-(3-{5-[4-méthyl-3,5-dioxo-2-(4,4,4-trifluoro-butyl)-2,3,4,5-tétrahydro-[1,2,4]triazin-6-yloxy]-pentyl}-phénoxy)-propionate d'éthyle
10. 2-{3-[5-(2-Heptyl-4-méthyl-3,5-dioxo-2,3,4,5-tétrahydro-[1,2,4]triazin-6-yloxy)-pentyl]-phénoxy}-2-méthyl-propionate d'éthyle
11. 2-{3-[5-(4-Butyl-2-méthyl-3,5-dioxo-2,3,4,5-tétrahydro-[1,2,4]triazin-6-yloxy)-pentyl]-phénoxy}-2-méthyl-propionate d'éthyle
12. Acide 2-{3-[5-(4-butyl-2-méthyl-3,5-dioxo-2,3,4,5-tétrahydro-[1,2,4]triazin-6-yloxy)-pentyl]-phénoxy}-2-méthyl-propionique
13. 2-{3-[5-(2,4-Dibutyl-3,5-dioxo-2,3,4,5-tétrahydro-[1,2,4]triazin-6-yloxy)-pentyl]-phénoxy}-2-méthyl-propionate d'éthyle
14. 2-(3-{5-[4-Butyl-3,5-dioxo-2-(4,4,4-trifluoro-butyl)-2,3,4,5-tétrahydro-[1,2,4]triazin-6-yloxy]-pentyl}-phénoxy)-2-méthyl-propionate d'éthyle
15. 2-{3-[5-(4-Butyl-2-heptyl-3,5-dioxo-2,3,4,5-tétrahydro-[1,2,4]triazin-6-yloxy)-pentyl]-phénoxy}-2-méthyl-propionate d'éthyle
16. 2-Méthyl-2-(3-{5-[2-méthyl-3,5-dioxo-4-(4,4,4-trifluoro-butyl)-2,3,4,5-tétrahydro-[1,2,4]triazin-6-yloxy]-pentyl}-phénoxy)-propionate d'éthyle
17. 2-{3-[5-(4-Heptyl-2-méthyl-3,5-dioxo-2,3,4,5-tétrahydro-[1,2,4]triazin-6-yloxy)-pentyl]-phénoxy}-2-méthyl propionate d'éthyle
18. 2-(3-{5-[4-Heptyl-3,5-dioxo-2-(4,4,4-trifluoro-butyl)-2,3,4,5-tétrahydro-[1,2,4]triazin-6-yloxy]-pentyl}-phénoxy)-2-méthyl-propionate d'éthyle
19. 2-{3-[5-(2-Benzyl-4-heptyl-3,5-dioxo-2,3,4,5-tétrahydro-[1,2,4]triazin-6-yloxy)-pentyl]-phénoxy}-2-méthyl-propionate d'éthyle
20. 2-[4-(2-Heptyl-4-méthyl-3,5-dioxo-2,3,4,5-tétrahydro-[1,2,4]triazin-6-yloxymethyl)-phénoxy]-2-méthyl-propionate d'éthyle
21. 2-Méthyl-2-{4-[2-méthyl-3,5-dioxo-4-(4,4,4-triffluoro-butyl)-2,3,4,5-tétrahydro-[1,2,4]triazin-6-yloxyrnéthyl]-phénoxy}-propionate d'éthyle
22. 2-[4-(4-Heptyl-2-méthyl-3,5-dioxo-2,3,4,5-tétrahydro-[1,2,4]triazin-6-yloxyméthyl)-phénoxy]-2-méthyl-propionate d'éthyle
23. 2-{4-[4-Heptyl-3,5-dioxo-2-(4,4,4-trifluoro-butyl)-2,3,4,5-tétrahydro-[1,2,4]triazin-6-yloxyméthyl]-phénoxy}-2-méthyl-propionate d'éthyle
24. 2-Méthyl-2-(4-{2-[4-méthyl-3,5-dioxo-2-(4,4,4-trifluoro-butyl)-2,3,4,5-tétrahydro-[1,2,4]triazin-6-yloxy]-éthyl}-phénoxy)-propionate d'éthyle
25. 2-{4-[2-(2-Heptyl-4-méthyl-3,5-dioxo-2,3,4,5-tétrahydro-[1,2,4]triazin-6-yloxy)-éthyl]-phénoxy}-2-méthyl-propionate d'éthyle
26. 2-{4-[2-(4-Heptyl-2-méthyl-3,5-dioxo-2,3,4,5-tétrahydro-[1,2,4]triazin-6-yloxy)-éthyl]-phénoxy}-2-méthyl-propionate d'éthyle
27. 2-{4-[3-(2-Heptyl-4-méthyl-3,5-dioxo-2,3,4,5-tétrahydro-[1,2,4]triazin-6-yloxy)-propyl]-phénoxy}-2-méthyl-propionate d'éthyle
28. 2-Méthyl-2-(4-{4-[4-méthyl-3,5-dioxo-2-(4,4,4-trifluoro-butyl)-2,3,4,5-tétrahydro-[1,2,4]triazin-6-yloxy]-butyl}-phénoxy)-propionate d'éthyle
29. 2-{4-[4-(2-Heptyl-4-méthyl-3,5-dioxo-2,3,4,5-tétrahydro-[1,2,4]triazin-6-yloxy)-butyl]-phénoxy}-2-méthyl-propionate d'éthyle
30. 2-Méthyl-2-(4-{4-[2-méthyl-3,5-dioxo-4-(4,4,4-trifluoro-butyl)-2,3,4,5-tétrahydro-[1,2,4]triazin-6-yloxy]-butyl}-phénoxy)-propionate d'éthyle
31. 2-(4-{4-[4-Heptyl-3,5-dioxo-2-(4,4,4-trifluoro-butyl)-2,3,4,5-tétrahydro-[1,2,4]triazin-6-yloxy]-butyl}-phénoxy)-2-méthyl-propionate d'éthyle
32. 2-{3-[3-(2-Heptyl-4-méthyl-3,5-dioxo-2,3,4,5-tétrahydro-[1,2,4]triazin-6-yloxy)-propyl]-phénylsulfanyl}-2-méthyl-propionate d'éthyle
33. 2-{3-[3-(2-Heptyl-4-méthyl-3,5-dioxo-2,3,4,5-tétrahydro-[1,2,4]triazin-6-yloxy)-propoxy]-phénoxy}-2-méthyl-propionate d'éthyle
34. 2-{3-[3-(2-Heptyl-4-méthyl-3,5-dioxo-2,3,4,5-tétrahydro-[1,2,4]triazin-6-yloxy)-propoxy]-phénylsulfanyl}-2-méthyl-propionate d'éthyle
35. 2-(3-{3-[3,5-Dioxo-2,4-bis-(4,4,4-trifluoro-butyl)-2,3,4,5-tétrahydro-[1,2,4]triazin-6-yloxy]-propoxy}-phénoxy)-2-méthyl-propionate d'éthyle
36. 2-{3-[4-(2-Heptyl-4-méthyl-3,5-dioxo-2,3,4,5-tétrahydro-[1,2,4]triazin-6-yloxy)-butoxy]-phénoxy}-2-méthyl-propionate d'éthyle
37. 2-{3-[4-(2-Heptyl-4-méthyl-3,5-dioxo-2,3,4,5-tétrahydro-[1,2,4]triazin-6-yloxy)-butoxy]-phénylsulfanyl}-2-méthyl-propionate d'éthyle
38. 2-{4-[2-(2-Heptyl-4-méthyl-3,5-dioxo-2,3,4,5-tétrahydro-[1,2,4]triazin-6-yloxy)-éthoxy]-phénylsulfanyl}-2-méthyl-propionate d'éthyle
39. 2-(4-{3-[3,5-Dioxo-2,4-bis-(4,4,4-trifluoro-butyl)-2,3,4,5-tétrahydro-[1,2,4]triazin-6-yloxy]-propoxy}-phénoxy)-2-méthyl-propionate d'éthyle
40. 2-{4-[2-(2-Heptyl-4-méthyl-3,5-dioxo-2,3,4,5-tétrahydro-[1,2,4]triazin-6-ylamino)-éthoxy]-phénoxy}-2-méthyl-propionate d'éthyle
41. 2-(4-{2-[3,5-Dioxo-2,4-bis-(4,4,4-trifluoro-butyl)-2,3,4,5-tétrahydro-[1,2,4]triazin-6-ylamino]-éthoxy}-phénoxy)-2-méthyl-propionate d'éthyle
42. 2-{4-[2-(2,4-Diheptyl-3,5-dioxo-2,3,4,5-tétrahydro-[1,2,4]triazin-6-ylamino)-éthoxy]-phénoxy}-2-méthyl-propionate d'éthyle
43. 2-(4-{2-[2,4-Bis-(3-cyclohexyl-propyl)-3, 5-dioxo-2,3,4,5-tétrahydro-[1,2,4]triazin-6-ylamino]-éthoxy}-phénoxy)-2-méthyl-propionate d'éthyle
44. 2-Méthyl-2-(4-{3-[4-méthyl-3,5-dioxo-2-(4,4,4-trifluoro-butyl)-2,3,4,5-tétrahydro-[1,2,4]triazin-6-ylamino]-propoxy}-phénoxy)-propionate d'éthyle
45. 2-{4-[3-(2-Heptyl-4-méthyl-3,5-dioxo-2,3,4,5-tétrahydro-[1,2,4]triazin-6-ylamino)-propoxy]-phénoxy}-2-méthyl-propionate d'éthyle
46. 2-Méthyl-2-(4-{3-[2-méthyl-3,5-dioxo-4-(4,4,4-trifluoro-butyl)-2,3,4,5-tétrahydro-[1,2,4]triazin-6-ylamino]-propoxy}-phénoxy)-propionate d'éthyle
47. 2-{4-[3-(4-Heptyl-2-méthyl-3,5-dioxo-2,3,4,5-tétrahydro-[1,2,4]triazin-6-ylamino)-propoxy]-phénoxy}-2-méthyl-propionate d'éthyle
48. 2-(4-{3-[4-Heptyl-3,5-dioxo-2-(4,4,4-trifluoro-butyl)-2,3,4,5-tétrahydro-[1,2,4]triazin-6-ylamino]-propoxy}-phénoxy)-2-méthyl-propionate d'éthyle
49. 2-{4-[3-(4-Heptyl-2-méthyl-3,5-dioxo-2,3,4,5-tétrahydro-[1,2,4]triazin-6-ylamino)-propoxy]-phénylsulfanyl}-2-méthyl-propionate d'éthyle
50. 2-{4-[4-(4-Heptyl-2-méthyl-3,5-dioxo-2,3,4,5-tétrahydro-[1,2,4]triazin-6-ylamino)-butoxy]-phénoxy}-2-méthyl-propionate d'éthyle
51. 2-(3-{3-[(2-Heptyl-4-méthyl-3,5-dioxo-2,3,4,5-tétrahydro-[1,2,4]triazin-6-yl)-(4,4,4-trifluoro-butyl)-amino]-propoxy}-phénoxy)-2-méthyl-propionate d'éthyle
52. 2-(3-{3-[(2,4-Diméthyl-3,5-dioxo-2,3,4,5-tétrahydro-[1,2,4]triazin-6-yl)-heptyl-amino]-propoxy}-phénylsulfanyl)-2-méthyl-propionate d'éthyle
53. 2-(4-{3-[(2,4-Diméthyl-3,5-dioxo-2,3,4,5-tétrahydro-[1,2,4]triazin-6-yl)-heptyl-amino]-propoxy}-phénylsulfanyl)-2-méthyl-propionate d'éthyle
54. 2-(3-{4-[(2,4-Diméthyl-3,5-dioxo-2,3,4,5-tétrahydro-[1,2,4] triazin-6-yl)-heptyl-amino]-butoxy}-phénylsulfanyl)-2-méthyl-propionate d'éthyle
55. 2-(2-{2-[3,5-Dioxo-2,4-bis-(4,4,4-trifluro-butyl)-2,3,4,5-tétrahydro-[1,2,4]triazin-6-ylamino]-éthyl}-phénoxy)-2-méthyl-propionate d'éthyle
56. 2-Méthyl-2-(3-{2-[4-méthyl-3,5-dioxo-2-(4,4,4-trifluoro-butyl)-2,3,4,5-tétrahydro-[1,2,4]triazin-6-ylamino]-éthyl}-phénoxy)-propionate d'éthyle
57. 2-{3-[2-(2-Heptyl-4-méthyl-3,5-dioxo-2,3,4,5-tétrahydro-[1,2,4]triazin-6-ylamino)-éthyl]-phénoxy}-2-méthyl-propionate d'éthyle
58. 2-Méthyl-2-(3-{2-[2-méthyl-3,5-dioxo-4-(4,4,4-trifluoro-butyl)-2,3,4,5-tétrahydro-[1,2,4]triazin-6-ylamino]-éthyl)-phénoxy)-propionate d'éthyle
59. 2-{3-[2-(4-Heptyl-2-méthyl-3,5-dioxo-2,3,4,5-tétrahydro-[1,2,4]triazin-6-ylamino)-éthyl]-phénoxy}-2-méthyl-propionate d'éthyle
60. 2-(3-{2-[4-Heptyl-3,5-dioxo-2-(4,4,4-trifluoro-butyl)-2,3,4,5-tétrahydro-[1,2,4]triazin-6-ylamino]-éthyl}-phénoxy)-2-méthyl-propionate d'éthyle
61. 2-{3-[2-(4-Heptyl-2-méthyl-3,5-dioxo-2,3,4,5-tétrahydro-[1,2,4]triazin-6-ylamino)-éthyl]-phénylsulfanyl}-2-méthyl-propionate d'éthyle
62. 2-Méthyl-2-(3-{3-[4-méthyl-3,5-dioxo-2-(4,4,4-trifluoro-butyl)-2,3,4,5-tétrahydro-[1,2,4]triazin-6-ylamino]-propyl}-phénoxy)-propionate d'éthyle
63. 2-(3-{3-[2-(2-Cyano-éthyl)-4-méthyl-3,5-dioxo-2,3,4,5-tétrahydro-[1,2,4]triazin-6-ylamino]-propyl}-phénoxy)-2-méthyl-propionate d'éthyle -
64. 2-{3-[3-(2-Heptyl-4-méthyl-3,5-dioxo-2,3,4,5-tétrahydro-[1,2,4]triazin-6-ylamino)-propyl]-phénoxy}-2-méthyl-propionate d'éthyle
65. 2-Méthyl-2-(3-{3-[2-méthyl-3,5-dioxo-4-(4,4,4-trifluoro-butyl)-2,3,4,5-tétrahydro-[1,2,4]triazin-6-ylamino]-propyl}-phénoxy)-propionate d'éthyle
66. 2-(3-{3-[3,5-Dioxo-2,4-bis-(4,4,4-triftuoro-butyl)-2,3,4,5-tétrahydro-[1,2,4]triazin-6-ylamino]-propyl}-phénoxy)-2-méthyl-propionate d'éthyle
67. 2-{3-[3-(4-Heptyl-2-méthyl-3,5-dioxo-2,3,4,5-tétrahydro-[1,2,4]triazin-6-ylamino)-propyl]-phénoxy}-2-méthyl-propionate d'éthyle
68. 2-(3-{3-[4-Heptyl-3,5-dioxo-2-(4,4,4-trifluoro-butyl)-2,3,4,5-tétrahydro-[1,2,4]triazin-6-ylamino]-propyl}-phénoxy)-2-méthyl-propionate d'éthyle
69. 2-{3-[3-(2,4-Diheptyl-3,5-dioxo-2,3,4,5-tétrahydro-[1,2,4]triazin-6-ylamino)-propyl]-phénoxy}-2-méthyl-propionate d'éthyle
70. 2-{3-[3-(2-Benzyl-4-heptyl-3,5-dioxo-2,3,4,5-tétrahydro-[1,2,4]triazin-6-ylamino)-propyl]-phénoxy}-2-méthyl-propionate d'éthyle
71. 2-(3-{3-[4-Benzyl-3,5-dioxo-2-(4,4,4-trifluoro-butyl)-2,3,4,5-tétrahydro-[1,2,4]triazin-6-ylamino]-propyl}-phénoxy)-2-méthyl-propionate d'éthyle
72. 2-{3-[3-(4-Benzy)-2-heptyl-3,5-dioxo-2,3,4,5-tétrahydro-[1,2,4]triazin-6-ylamino)-propyl]-phénoxy}-2-méthyl-propionate d'éthyle
73. 2-{3-[3-(4-Hepiyl-2-méthyl-3,5-dioxo-2,3,4,5-tétrahydro-[1,2,4]triazin-6-ylamino)-propyl]-phénylsulfanyl}-2-méthyl-propionate d'éthyle
74. 2-Méthyl-2-(3-{4-[4-méthyl-3,5-dioxo-2-(4,4,4-trifluoro-butyl)-2,3,4,5-tétrahydro-[1,2,4]triazin-6-ylamino]-butyl}-phénoxy)-propionate d'éthyle 110
75. 2-{3-[4-(2-Heptyl-4-méthyl-3,5-dioxo-2,3,4,5-tétrahydro-[1,2,4]triazin-6-ylamino)-butyl]-phénoxy}-2-méthyl-propionate d'éthyle
76. 2-Méthyl-2-(3-{4-[4-(3-méthyl-but-2-ènyl)-3,5-dioxo-2-(4,4,4-trifluoro-butyl)-2,3,4,5-tétrahydro-[1,2,4]triazin-6-ylamino]-butyl}-phénoxy)-propionate d'éthyle
77. 2-Méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénoxy)-propionate d'éthyle
78. 2-Méthyl-2-(3-{4-[2-méthyl-3,5-dioxo-4-(4,4,4-trifluoro-butyl)-2,3,4,5-tétrahydro-[1,2,4]triazin-ylamino]-butyl}-phénoxy)-propionate de tert-butyle
79. Acide 2-méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénoxy)-propionique
80. 2-(3-{4-[3,5-Dioxo-2,4-bis-(4,4,4-trifluoro-butyl)-2,3,4,5-tétrahydro-[1,2,4] triazin-6-ylamino]-butyl}-phénoxy)-2-méthyl-propionate d'éthyle
81. 2-(3-{4-[2-(2-Cyano-éthyl)-3,5-dioxo-4-(4,4,4-trifluoro-butyl)-2,3,4,5-tétrahydro-[1,2,4]triazin-6-ylamino]-butyl}-phénoxy)-2-méthyl-propionate d'éthyle
82. 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionate d'éthyle
83. 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionate de tert-butyle
84. Acide 2-(3-{4-[2-Heptyl-3,5-dioxo-4-(4,4,4-trifluoro-butyl)-2,3,4,5-tétrahydro-[1,2,4]triazin-6-ylamino]-butyl}-phénoxy)-2-méthyl-propionique
85. 2-(3-{4-[4-Heptyl-3,5-dioxo-2-(4,4,4-trifluoro-butyl)-2,3,4,5-tétrahydro-[1,2,4]triazin-6-ylamino]-butyl}-phénoxy)-2-méthyl-propionate d'éthyle
86. 2-(3-{4-[2-(2-Cyano-éthyl)-4-heptyl-3,5-dioxo-2,3,4,5-tétrahydro-[1,2,4]triazin-6-ylamino]-butyl}-phénoxy)-2-méthyl-propionate d'éthyle
87. 4-(6-{4-[3-(1-Ethoxycarbonyl-1-méthyl-éthoxy)-phényl]-butylamino}-4-heptyl-3,5-dioxo-4,5-dihydro-3H-[1,2,4]triazin-2-yl)-but-2-enonate d'éthyle
88. 2-{3-[4-(2,4-Diheptyl-3,5-dioxo-2,3,4,5-tétrahydro-[1,2,4]triazin-6-ylamino)-butyl]-phénoxy}-2-méthyl-propionate d'éthyle
89. 2-{3-[4-(2-Benzyl-4-heptyl-3,5-dioxo-2,3,4,5-tétrahydro-[1,2,4]triazin-6-ylamino)-butyl]-phénoxy}-2-méthyl-propionate d'éthyle
90. 2-(3-{4-[4-Benzyl-3,5-dioxo-2-(4,4,4-trifluoro-butyl)-2,3,4,5-tétrahydro-[1,2,4]triazin-6-ylamino]-butyl}-phénoxy)-2-méthyl-propionate d'éthyle
91. 2-{3-[4-(4-Benzyl-2-heptyl-3,5-dioxo-2,3,4,5-tetrahydro-[1,2,4]triazin-6-ylamino)-butyl]-phénoxy}-2-méthyl-propionate d'éthyle
92. 2-(3-{5-[3,5-Dioxo-2,4-bis-(4,4,4-trifluoro-butyl)-2,3,4,5-tétrahydro-[1,2,4]triazin-6-ylamino]-pentyl}-phénoxy)-2-méthyl-propionate d'éthyle
93. 2-Méthyl-2-(3-{4-[4-méthyl-3,5-dioxo-2-(4,4,4-trifluoro-butyl)-2,3,4,5-tetrahydro-[1,2,4]triazin-6-ylamino]-butyl}-phénylsulfanyl)-propionate d'éthyle
94. 2-{3-[4-(2-Heptyl-4-méthyl-3,5-dioxo-2,3,4,5-tétrahydro-[1,2,4]triazin-6-ylamino)-butyl]-phénylsulfanyl}-2-méthyl-propionate d'éthyle
95. 2-Méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénylsulfanyl)-propionate d'éthyle
96. Acide 2-méthyl-2-(3-{4-[2-méthyl-3,5-dioxo-4-(4,4,4-trifluoro-butyl)-2,3,4,5-tétrahydro-[1,2,4]triazin-6-ylamino]-butyl}-phénylsulfanyl)-propionique
97. 2-(3-{4-[4-Heptyl-3,5-dioxo-2-(4,4,4-trifluoro-butyl)-2,3,4,5-tétrahydro-[1,2,4]triazin-6-ylamino]-butyl}-phénylsulfanyl)-2-méthyl-propionate d'éthyle
98. 2-{3-[4-(2,4-Diheptyl-3,5-dioxo-2,3,4,5-tetrahydro-[1,2,4]triazin-6-ylamino)-butyl]-phénylsulfanyl}-2-méthyl-propionate d'éthyle
99. 2-{3-[4-(2-Benzyl-4-heptyl-3,5-dioxo-2,3,4,5-tétrahydro-[1,2,4]triazin-6-ylamino)-butyl]-phénylsulfanyl}-2-méthyl-propionate d'éthyle
100. 2-(3-{4-[4-Benzyl-3,5-dioxo-2-(4,4,4-trifluoro-butyl)-2,3,4,5-tétrahydro-[1,2,4]triazin-6-ylamino]-butyl}-phénylsulfanyl)-2-méthyl-propionate d'éthyle
101. 2-{3-[4-(4-Benzyl-2-heptyl-3,5-dioxo-2,3,4,5-tétrahydro-[1,2,4]triazin-6-ylamino)-butyl]-phénylsulfanyl}-2-méthyl-propionate d'éthyle
102. 2-Méthyl-2-(4-{2-[4-méthyl-3,5-dioxo-2-(4,4,4-trifluoro-butyl)-2,3,4,5-tétrahydro-[1,2,4]triazin-6-ylamino]-éthyl}-phénoxy)-propionate d'éthyle
103. 2-{4-[2-(2-Heptyl-4-méthyl-3,5-dioxo-2,3,4,5-tétrahydro-[1,2,4]triazin-6-ylamino)-éthyl]-phénoxy}-2-méthyl-propionate d'éthyle
104. 4-[6-{2-[4-(1-Ethoxycarbonyl-1-méthyl-éthoxy)-phényl]-éthylamino}-3,5-dioxo-4-(4,4,4-trifluoro-butyl)-4,5-dihydro-3H-[1,2,4]triazin-2-yl]-but-2-ènoate d'éthyle
105. 2-(4-{2-[2-(3-Cyclohexyl-propyl)-3,5-dioxo-4-(4,4,4-trifluoro-butyl)-2,3,4,5-tétrahydro-[1,2,4]triazin-6-ylamino]-éthyl}-phénoxy)-2-méthyl-propionate d'éthyle
106. 2-{4-[2-(4-Heptyl-2-méthyl-3,5-dioxo-2,3,4,5-tétrahydro-[1,2,4]triazin-6-ylamino)-éthyl]-phénoxy}-2-méthyl-propionate d'éthyle
107. 2-(4-{2-[4-Heptyl-3,5-dioxo-2-(4,4,4-trifluoro-butyl)-2,3,4,5-tétrahydro-[1,2,4]triazin-6-ylamino]-éthyl}-phénoxy)-2-méthyl-propionate d'éthyle
108. 2-{4-[2-(2,4-Diheptyl-3,5-dioxo-2,3,4,5-tétrahydro-[1,2,4]triazin-6-ylamino)-éthyl]-phénoxy}-2-méthyl-propionate d'éthyle
109. 2-{4-[2-(4-Benzyl-2-heptyl-3,5-dioxo-2,3,4,5-tétrahydro-[1,2,4]triazin-6-ylamino)-éthyl]-phénoxy}-2-méthyl-propionate d'éthyle
110. 2-{4-[2-(2,4-Bis-benzyloxyméthyl-3,5-dioxo-2,3,4,5-tétrahydro-[1,2,4] triazin-6-ylamino)-éthyl]-phénoxy}-2-méthyl-propionate d'éthyle
111. 2-(4-{2-[(2,4-Diméthyl-3,5-dioxo-2,3,4,5-tétrahydro-[1,2,4]triazin-6-yl)-(4,4,4-trifluoro-butyl)-amino]-éthyl}phénoxy)-2-méthyl-propionate d'éthyle
112. 2-(4-{2-[(2,4-Diméthyl-3,5-dioxo-2,3,4,5-tétrahydro-[1,2,4]triazin-6-yl)-heptyl-amino]-éthyl}-phénoxy)-2-méthyl-propionate d'éthyle
113. Acide 2-(4-{2-[(2,4-diméthyl-3,5-dioxo-2,3,4,5-tétrahydro-[1,2,4]triazin-6-yl)-heptyl-amino]-éthyl}-phénoxy)-2-méthyl-propionique
114. 2-(4-{2-[(2,4-Diméthyl-3,5-dioxo-2,3,4,5-tétrahydro-[1,2,4]triazin-6-yl)-phénéthyl-amino]-éthyl}-phénoxy)-2-méthyl-propionate d'éthyle
115. 2-(4-{2-[(2,4-Diméthyl-3,5-dioxo-2,3,4,5-tétrahydro-[1,2,4]triazin-6-yl)-(3-phényl-propyl)-amino]-éthyl}-phénoxy)-2-méthyl-propionate d'éthyle
116. 2-(4-{2-[(4-Heptyl-2-méthyl-3,5-dioxo-2,3,4,5-tétrahydro-[1,2,4]triazin-6-yl)-phénéthyl-amino]-éthyl}-phénoxy)-2-méthyl-propionate d'éthyle
117. 2-(4-{2-[(4-Heptyl-2-méthyl-3,5-dioxo-2,3,4,5-tétrahydro-[1,2,4]triazin-6-yl)-(3-phényl-propyl)-amino]-éthyl}-phénoxy)-2-méthyl-propionate d'éthyle
118. Acide 2-méthyl-2-(4-{2-[4-méthyl-3,5-dioxo-2-(4,4,4-trifluoro-butyl)-2,3,4,5-tétrahydro-[1,2,4]triazin-6-ylamino]-éthyl}-phénylsulfanyl)-propionique
119. 2-{4-[2-(2-Heptyl-4-méthyl-3,5-dioxo-2,3,4,5-tétrahydro-[1,2,4]triazin-6-ylamino)-éthyl]-phénylsulfanyl}-2-méthyl-propionate d'éthyle
120. Acide 2-{4-[2-(2-Heptyl-4-méthyl-3,5-dioxo-2,3,4,5-tétrahydro-[1,2,4]triazin-6-ylamino)-éthyl]-phénylsulfanyl}-2-méthyl-propionique
121. Acide 2-{4-[2-(4-butyl-2-heptyl-3,5-dioxo-2,3,4,5-tétrahydro-[1,2,4]triazin-6-ylamino)-éthyl]-phénylsulfanyl}-2-méthyl-propionique
122. 2-{4-[2-(4-Heptyl-2-méthyl-3,5-dioxo-2,3,4,5-tétrahydro-[1,2,4]triazin-6-ylamino)-éthyl]-phénylsulfanyl}-2-méthyl-propionate d'éthyle
123. Acide 2-(4-{2-[4-Heptyl-3,5-dioxo-2-(4,4,4-trifluoro-butyl)-2,3,4,5-tétrahydro-[1,2,4]triazin-6-ylamino]-éthyl}-phénylsulfanyl)-2-méthyl-propionique
124. 2-{4-[3-(2-Heptyl-4-méthyl-3,5-dioxo-2,3,4,5-tétrahydro-[1,2,4]triazin-6-ylamino)-propyl]-phénylsulfanyl}-2-méthyl-propionate d'éthyle

6. Procédé de préparation des composés chimiques selon l'une des revendications **1)** à **5) caractérisé en ce que** :
l'on condense un dérivé de formule générale **II** 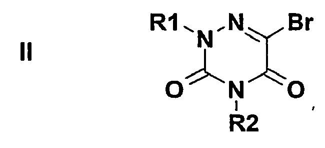
dans lequel R₁ et R₂ représentent les groupements tels que décrits précédemment dans la formule I avec un dérivé de formule générale III 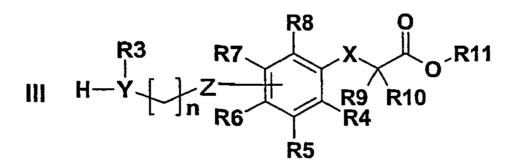 où **YR₃, n, Z, X, R₄, R₅, R₆, R₇, R₈, R₉, R₁₀ et R₁₁** sont tels que décrits précédemment dans la formule **I,** notamment en présence d'une base telle que la triéthylamine dans le nbutanol (lorsque Y=N) ou de carbonate de potassium dans le diméthylformamide (lorsque **YR₃**=O);

7. Procédé de préparation des composés chimiques selon l'une des revendications **1)** à **5)** de formule générale **I** pour lesquels **Z=O caractérisé en ce que** :
a) l'on condense un dérivé de formule générale **II** 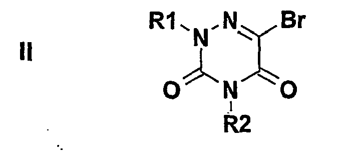 dans lequel **R₁** et **R₂** représentent les groupements tels que décrits précédemment dans la formule **I** avec un dérivé de formule générale **IV** 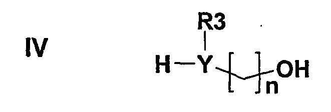 dans lequel **R₃Y** peut être égal à NH ou O et n est tel que décrit précédemment dans la formule **I,** notamment en l'absence de solvant sans ajouter de base (dans le cas où **R₃Y**=NH) ou en présence d'une base telle que K₂CO₃ (dans le cas où **R₃Y**=O).
b) l'on condense le dérivé obtenu **V** 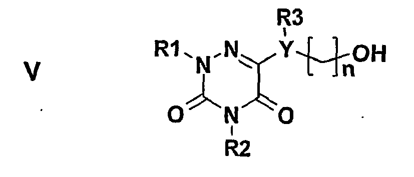 avec un composé de formule générale **VI** 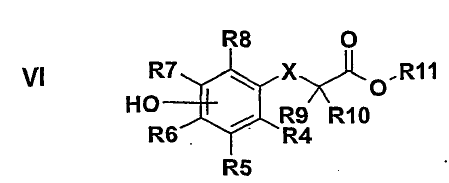
où **X, R₄, R₅, R₆, R₇, R₈, R₉, R₁₀ et R₁₁** sont tels que décrits précédemment dans la formule **I,** notamment dans des conditions telles que celles de la réaction de Mitsunobu en présence dé triphénylphosphine et de diéthylazodicarboxylate dans le THF.

8. Procédé de préparation des composés chimiques selon l'une des revendications **1)** à **5)** de formule générale I pour lesquels **Z**=O **caractérisé en ce que** :
a) l'on protège la fonction alcool d'un dérivé de formule générale VII 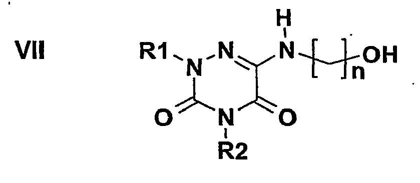 dans lequel **R₁, R₂** et n sont tels que décrit précédemment dans la formule **I** par un groupement protecteur parmi lesquels le tertbutyldiméthylsilane, notamment dans des conditions telles que le THF en utilisant le chlorotertbutyldiméthylsilane et l'imidazole. 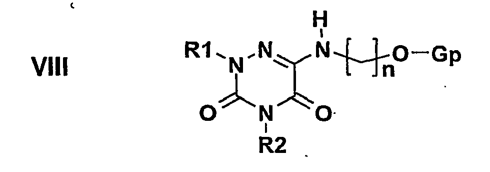
b) l'on alkyle l'azote du composé VIII précédemment obtenu par un dérivé halogéné **R₃**Hal dans lequel le groupement **Hal** représente un halogène tel que CI, Br ou I est **R₃** est tel que décrit précédemment dans la formule **I,** dans des conditions opératoires telles que NaH ou tBuOK dans le DMF. 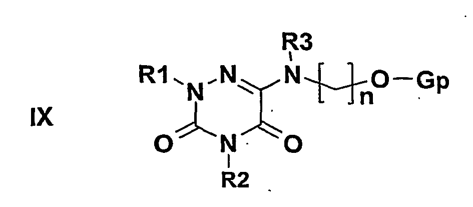
c) l'on déprotège le composé **IX** ainsi obtenu dans des conditions opératoires telles que le fluorure de tétrabutylammonium dans le THF.
d) l'on condense le dérivé obtenu X 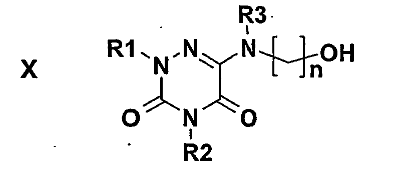 avec un composé de formule générale **VI** 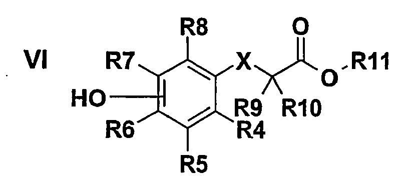
où **X, R₄, R₅, R₆, R₇, R₈, R₉, R₁₀ et R₁₁** sont tels que décrits précédemment dans la formule **I,** notamment dans des conditions telles que celles de la réaction de Mitsunobu en présence de triphénylphosphine et de diéthylazodicarboxylate dans le THF.

9. Procédé de préparation des composés chimiques selon l'une des revendications **1)** à **5)** de formule générale **I** lorsque **Y=N** et **Z=C caractérisé en ce que** :
a) l'on condense un dérivé de formule générale II 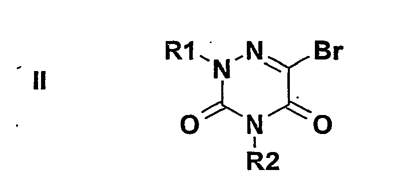 dans lequel **R₁** et **R₂** représentent les groupements tels que décrits précédemment dans la formule **I** avec un dérivé de formule générale **XI** 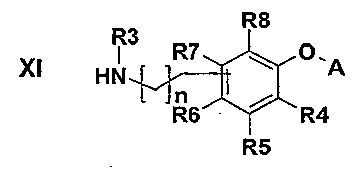 dans lequel **n**, **R₃ R₄, R₅, R₆, R₇, et R₈** sont tels que décrit précédemment dans la formule **I** et A peut être l'hydrogène ou un groupement méthyle, notamment en présence d'une base telle que la triéthylamine dans le nbutanol.
b) après déméthylation (si **A**=Me, dans des conditions telles que BBr₃ dans le dichlorométhane), on alkyle la fonction phénol du dérivé XII 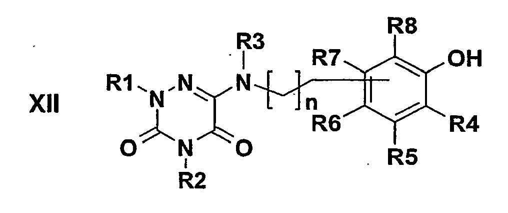 par un dérivé halogéné de formule générale **XIII** (utilisé comme solvant en présence d'une base telle que le carbonate de potassium) 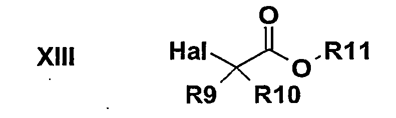 pour lequel le groupement **Hal** représente un halogène tel que CI, Br ou **I**, et **R₉, R₁₀** et **R₁₁** sont tels que décrits précédemment dans la formule générale I.

10. Procédé de préparation des composés chimiques selon l'une des revendications **1)** à **5)** de formule générale **I** lorsque Y=N, **Z**=C **caractérisé en ce que** :
a) l'on alkyle un dérivé de formule générale **XIV** 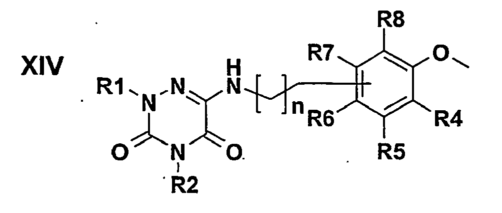 dans lequel **R₁, R₂, R₄, R₅, R₆, R₇, R₈** et n sont tels que décrits précédemment dans la formule **I** avec un dérivé de formule **R₃**Hal dans lequel le groupement **Hal** représente un halogène tel que CI, Br ou **I** est **R₃** est tel que décrit précédemment dans la formule **I**, dans des conditions opératoires telles qu'en présence de NaH ou tBuOK dans le DMF.
b) après déméthylation dans des conditions telles que BBr₃ dans le dichlorométhane, on alkyle la fonction phénol du dérivé **XII** ainsi obtenu 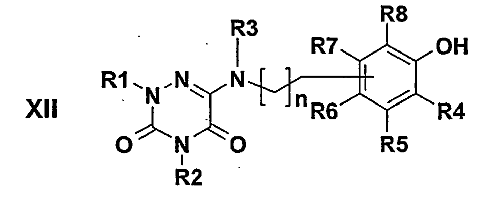 par un dérivé halogéné de formule générale **XIII** (utilisé comme solvant en présence d'une base telle que le carbonate de potassium) 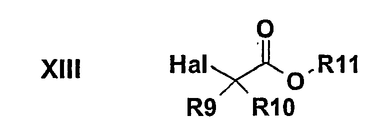 pour lequel le groupement **Hal** représente un halogène tel que CI, Br ou I, et **R₉, R₁₀** et **R₁₁** sont tels que décrits précédemment dans la formule générale I.

11. Procédé de préparation des composés chimiques selon l'une des revendications **1)** à **5) caractérisé en ce que** :
a) l'on place un dérivé de formule générale **I** 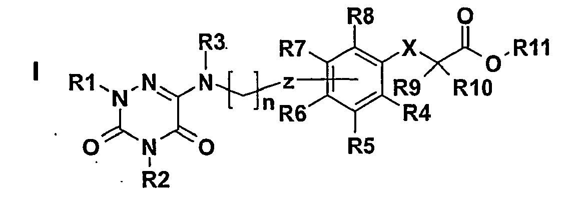 dans lequel **R₁**=(CH₂)₂CN et **R₂, R₃, n, Z; X, R₄, R₅, R₆, R₇, R₈, R₉, R₁₀** et **R₁₁** sont tels que décrits précédemment dans la formule **I** ou **R₂**=(CH₂)₂CN et **R₁, R₃, n, Z, X, R₄, R₅, R₆, R₇, R₈, R₉, R₁₀ et R₁₁** sont tels que décrits précédemment dans la formule **I** dans des conditions opératoires telles qu'en présence d'une base NaH dans le DMF.
b) on alkyle ensuite l'azote de la triazine du dérivé XIVa ou XIVb ainsi obtenu 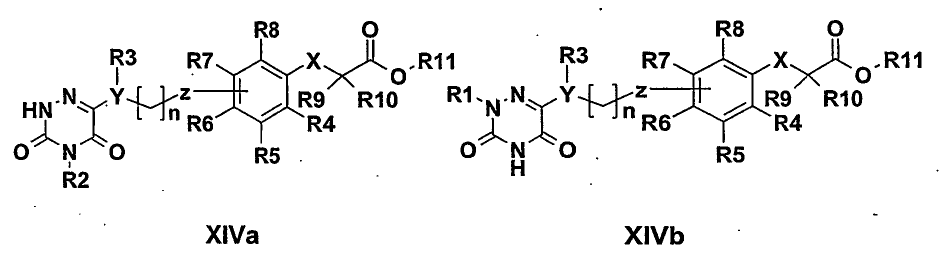 par un dérivé halogéné de formule générale **R₁Hal** dans le cas de l'intermédiaire **XIVa** et de formule générale **R₂Hal** dans le cas de l'intermédiaire **XIVb** pour lequel le groupement **Hal** représente un halogène tel que CI, Br ou I et **R₁** et **R₂** sont tels que décrits précédemment dans la formule **I,** dans des conditions opératoires telles qu'en présence de NaH ou tBuOK dans le DMF.

12. A titre de médicaments nouveaux utilisables dans le traitement des maladies nécessitant des agonistes des récepteurs PPAR alpha et/ou PPAR gamma, les composés définis selon l'une des revendications **1)** à **5).**

13. A titre de médicaments nouveaux utilisables dans la prévention et le traitement des maladies telles que les dyslipidémies diabétiques, l'hypertriglycéridémie, l'hypercholestérolémie, l'hyperinsulinémie, l'hyperglycémie, le syndrome métabolique, l'obésité, l'athérosclérose, les composés définis selon l'une des revendications **1)** à **5).**

14. A titre de médicaments nouveaux utilisables en dermatologie, dans des pathologies à composante inflammatoire ou résultant d'une différentiation cellulaire anormale, les composés définis selon l'une des revendications **1)** à **5).**

15. A titre de médicaments nouveaux utilisables dans le traitement des maladies telles que le psoriasis, l'acné, les dermatites atopiques, le vieillissement cutané, le photovieillissement, les composés définis selon l'une des revendications **1)** à **5).**

16. Composition pharmaceutique **caractérisée en ce qu'**elle contient à titre de principe actif un composé défini selon l'une des revendications **1)** à **5)** en association avec tout excipient approprié.

## Claims

1. 3,5-Dioxo-(2H,4H)-1,2,4-triazine derivatives of general formula **I** 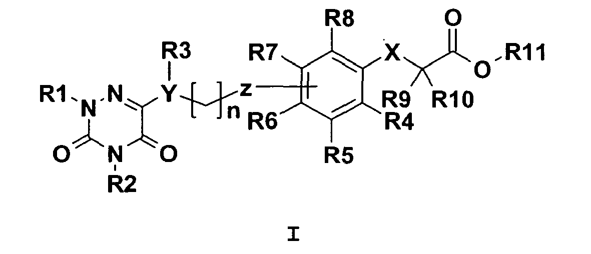 in which
- **R₁** and **R₂** can be identical or different and represent an alkyl or alkenyl radical, linear or branched, at C₁-C₇, an alkyl radical at C₁-C₆ substituted by groups such as trifluoromethyl, cycloalkyl at C₅-C₆, nitrile, alkoxycarbonylvinyl at C₁-C₄, hydroxycarbonylvinyl, alkoxycarbonyl at C₁-C₄, carboxylate, benzyloxy or phenyl (for which the core phenyl is possibly substituted by one or more groups such as alkyl at C₁-C₄, alkoxy at C₁-C₄, nitro, halogen or trifluoromethyl),
- **YR₃** represents oxygen or **NR₃** in which **R₃** represents hydrogen, an alkyl or alkenyl radical, linear or branched at C₁-C₇, an alkyl radical at C₁-C₆ substituted by groups such as trifluoromethyl or phenyl (for which the core phenyl is possibly substituted by one or more groups such as alkyl at C₁-C₄, alkoxy at C₁-C₄, nitro, halogen or trifluoromethyl),
- Z represents an oxygen atom or a carbon atom which can be bound in ortho, meta or para position on the phenyl group of formula I,
- n can range from 0 to 5 when Z=C or from 2 to 4 when Z=O,
- **X** represents oxygen or sulfur,
- **R₄, R₅, R₆, R₇,** and **R₈** represent hydrogen or fluorine,
- **R₉, R₁₀** and **R₁₁** represent hydrogen or an alkyl group, linear or branched, at C₁-C₅,
as well as additive salts with pharmaceutically acceptable bases and the various enantiomers of compounds having asymmetrical carbons, as well as their mixtures in all proportions, including racemic mixtures in particular.

2. 3,5-Dioxo-(2H,4H)-1,2,4-triazine derivatives of general formula **I** according to claim 1 in which:
- **R₁** and **R₂** represent, independently one from the other, an alkyl or alkenyl radical, linear or branched, at C₁-C₇, an alkyl radical at C₁-C₆ substituted by groups such as trifluoromethyl, cycloalkyl at C₆, nitrile, phenyl (for which the core phenyl is possibly substituted by one or more groups such as alkyl at C₁-C₄, alkoxy at C₁-C₄, nitro, halogen or trifluoromethyl),
- **YR₃** represents oxygen or **NR₃** in which **R₃** represents hydrogen, an alkyl or alkenyl radical, linear or branched, at C₁-C₇, an alkyl radical at C₁-C₆ substituted by groups such as trifluoromethyl or phenyl,
- **Z** represents an oxygen atom or a carbon atom which can be bound in ortho, meta or para position on the phenyl group of formula **I,**
- **n** can range from 0 to 5 when **Z**=C or from 2 to 4 when **Z**=O,
- **X** represents oxygen or sulfur,
- **R₄, R₅, R₆, R₇,** and **R₈** represent hydrogen or fluorine,
- **R₉, R₁₀** and R₁₁ represent hydrogen or an alkyl group, linear or branched, at C₁-C₅.

3. 3,5-Dioxo-(2H,4H)-1,2,4-triazine derivatives of general formula **I** according to one of the claims 1 and 2 in which:
- **R₁** and **R₂** represent independently one from the other, an alkyl or alkenyl radical, linear or branched, at C₁-C₇, an alkyl radical at C₁-C₆ substituted by groups such as trifluoromethyl or nitrile,
- **YR₃** represents oxygen or **NR₃** in which **R₃** represents hydrogen or a linear or branched alkyl radical at C₁-C₇,
- **Z** represents a carbon atom which can be bound in ortho, meta or para position on the phenyl group of formula **I**,
- **n** can range from 0 to 5,
- **X** represents oxygen or sulfur,
- **R₄, R₅, R₆, R₇,** and **R₈** represent hydrogen or fluorine,
- **R₉, R₁₀** and **R₁₁** represents hydrogen or a linear or branched alkyl group at C₁-C₅, in particular **R₉** and **R₁₀** represent a methyl group and **R₁₁** hydrogen or an ethyl group.

4. 3,5-Dioxo-(2H,4H)-1,2,4-triazine derivatives of general formula **I** according to one of the claims 1 and 3 in which:
- **R₁** and **R₂** represent independently one from the other, an alkyl or alkenyl radical, linear or branched, at C₁-C₇, possibly substituted at the end of the chain by a trifluoromethyl group,
- **YR₃** represents oxygen or **NR₃** in which **R₃** represents hydrogen or a linear or branched alkyl radical at C₁-C₇,
- **Z** represents a carbon atom which can be bound in meta or para position on the phenyl group of formula **I**,
- **n** can range from 1 to 5,
- **X** represents oxygen or sulfur,
- **R₄** to **R₈** represent hydrogen,
- **R₉ and** R₁₀ represent a methyl radical,
- **R₁₁** represents hydrogen or an ethyl radical.

5. Compounds of general formula **I** according to claim 1) wherein they are selected among:
1. Ethyl 2-{2-[2-(4-butyl-2-methyl-3,5-dioxo-2,3,4,5-tetrahydro-[1,2,4]triazin-6-yloxy)ethyl]phenoxy}-2-methyl-propionate
2. Ethyl 2-{3-[2-(2-heptyl-4-methyl-3,5-dioxo-2,3,4,5-tetrahydro-[1,2,4]triazin-6-yloxy)ethyl]phenoxy}-2-methyl-propionate
3. Ethyl 2-methyl-2-(3-{2-[2-methyl-3,5-dioxo-4-(4,4,4-trifluoro-butyl)-2,3,4,5-tetrahydro-[1,2,4]triazin-6-yloxy]ethyl}phenoxy)propionate
4. Ethyl 2-methyl-2-(3-{3-[4-methyl-3,5-dioxo-2-(4,4,4-trifluoro-butyl)-2,3,4,5-tetrahydro-[1,2,4]triazin-6-yloxy]propyl}phenoxy)propionate
5. Ethyl 2-{3-[3-(2-heptyl-4-methyl-3,5-dioxo-2,3,4,5-tetrahydro-[1,2,4]triazin-6-yloxy)propyl]phenoxy}-2-methyl-propionate
6. Ethyl 2-methyl-2-(3-{3-[2-methyl-3,5-dioxo-4-(4,4,4-trifluoro-butyl)-2,3,4,5-tetrahydro-[1,2,4]triazin-6-yloxy]propyl}phenoxy)propionate
7. Ethyl 2-{3-[3-(4-heptyl-2-methyl-3,5-dioxo-2,3,4,5-tetrahydro-[1,2,4]triazin-6-yloxy)propyl]phenoxy}-2-methyl-propionate
8. Ethyl 2-(3-{3-[4-heptyl-3,5-dioxo-2-(4,4,4-trifluoro-butyl)-2,3,4,5-tetrahydro-[1,2,4]triazin-6-yloxy]propyl}phenoxy)-2-methyl-propionate
9. Ethyl 2-methyl-2-(3-{5-[4-methyl-3,5-dioxo-2-(4,4,4-trifluoro-butyl)-2,3,4,5-tetrahydro-[1,2,4]triazin-6-yloxy]pentyl}phenoxy)propionate
10. Ethyl 2-{3-[5-(2-heptyl-4-methyl-3,5-dioxo-2,3,4,5-tetrahydro-[1,2,4]triazin-6-yloxy)pentyl]phenoxy}-2-methyl-propionate
11. Ethyl 2-{3-[5-(4-butyl-2-methyl-3,5-dioxo-2,3,4,5-tetrahydro-[1,2,4]triazin-6-yloxy)pentyl]phenoxy}-2-methyl-propionate
12. 2-{3-[5-(4-Butyl-2-methyl-3,5-dioxo-2,3,4,5-tetrahydro-[1,2,4]triazin-6-yloxy)pentyl]phenoxy}-2-methyl-propionic acid
13. Ethyl 2-{3-[5-(2,4-dibutyl-3,5-dioxo-2,3,4,5-tetrahydro-[1,2,4]triazin-6-yloxy)pentyl]phenoxy)-2-methyl-propionate
14. Ethyl 2-(3-{5-[4-butyl-3,5-dioxo-2-(4,4,4-trifluoro-butyl)-2,3,4,5-tetrahydro-[1,2,4]triazin-6-yloxy]pentyl}phenoxy)-2-methyl-propionate
15. Ethyl 2-{3-[5-(4-butyl-2-heptyl-3,5-dioxo-2,3,4,5-tetrahydro-[1,2,4]triazin-6-yloxy)pentyl]phenoxy}-2-methyl-propionate
16. Ethyl 2-methyl-2-(3-{5-[2-methyl-3,5-dioxo-4-(4,4,4-trifluoro-butyl)-2,3,4,5-tetrahydro-[1,2,4]triazin-6-yloxy]pentyl}phenoxy)propionate
17. Ethyl 2-f3-[5-(4-heptyl-2-methyl-3,5-dioxo-2,3,4,5-tetrahydro-[1,2,4]triazin-6-yloxy)pentyl]phenoxy}-2-methyl propionate
18. Ethyl 2-(3-{5-[4-heptyl-3,5-dioxo-2-(4,4,4-trifluoro-butyl)-2,3,4,5-tetrahydro-[1,2,4]triazin-6-yloxy]pentyl}phenoxy)-2-methyl-propionate
19. Ethyl 2-{3-[5-(2-benzyl-4-heptyl-3,5-dioxo-2,3,4,5-tetrahydro-[1,2,4]triazin-6-yloxy)pentyl]phenoxy}-2-methyl-propionate
20. Ethyl 2-[4-(2-heptyl-4-methyl-3,5-dioxo-2,3,4,5-tetrahydro-[1,2,4]triazin-6-yloxymethyl)phenoxy]-2-methyl-propionate
21. Ethyl 2-methyl-2-{4-[2-methyl-3,5-dioxo-4-(4,4,4-trifluoro-butyl)-2,3,4,5-tetrahydro-[1,2,4]triazin-6-yloxymethyl]phenoxy}propionate
22. Ethyl 2-[4-(4-heptyl-2-methyl-3,5-dioxo-2,3,4,5-tetrahydro-[1,2,4]triazin-6-yloxymethyl)phenoxy]-2-methyl-propionate
23. Ethyl 2-{4-[4-heptyl-3,5-dioxo-2-(4,4,4-trifluorobutyl)-2,3,4,5-tetrahydro-[1,2,4]triazin-6-yloxymethyl]phenoxy}-2-methyl-propionate
24. Ethyl 2-methyl-2-(4-{2-[4-methyl-3,5-dioxo-2-(4,4,4-trifluoro-butyl)-2,3,4,5-tetrahydro-[1,2,4]triazin-6-yloxy]ethyl}phenoxy)propionate
25. Ethyl 2-{4-[2-(2-heptyl-4-methyl-3,5-dioxo-2,3,4,5-tetrahydro-[1,2,4]triazin-6-yloxy)ethyl]phenoxy}-2-methyl-propionate
26. Ethyl 2-{4-[2-(4-heptyl-2-methyl-3,5-dioxo-2,3,4,5-tetrahydro-[1,2,4]triazin-6-yloxy)ethyl]phenoxy}-2-methyl-propionate
27. Ethyl 2-{4-[3-(2-heptyl-4-methyl-3,5-dioxo-2,3,4,5-tetrahydro-[1,2,4]triazin-6-yloxy)propyl]phenoxy}-2-methyl-propionate
28. Ethyl 2-methyl-2-(4-{4-[4-methyl-3,5-dioxo-2-(4,4,4-trifluoro-butyl)-2,3,4,5-tetrahydro-[1,2,4]triazin-6-yloxy]butyl}phenoxy)propionate
29. Ethyl 2-{4-[4-(2-heptyl-4-methyl-3,5-dioxo-2,3,4,5-tetrahydro-[1,2,4]triazin-6-yloxy)butyl]phenoxy}-2-methyl-propionate
30. Ethyl 2-methyl-2-(4-{4-[2-methyl-3,5-dioxo-4-(4,4,4-trifluoro-butyl)-2,3,4,5-tetrahydro-[1,2,4]triazin-6-yloxy]butyl}phenoxy)propionate
31. Ethyl 2-(4-{4-[4-heptyl-3,5-dioxo-2-(4,4,4-trifluoro-butyl)-2,3,4,5-tetrahydro-[1,2,4]triazin-6-yloxy]butyl}phenoxy)-2-methyl-propionate
32. Ethyl 2-{3-[3-(2-heptyl-4-methyl-3,5-dioxo-2,3,4,5-tetrahydro-[1,2,4]triazin-6-yloxy)propyl]phenylsulfanyl}-2-methyl-propionate
33. Ethyl 2-{3-[3-(2-heptyl-4-methyl-3,5-dioxo-2,3,4,5-tetrahydro-[1,2,4]triazin-6-yloxy)propoxy]phenoxy}-2-methyl-propionate
34. Ethyl 2-{3-[3-(2-heptyl-4-methyl-3,5-dioxo-2,3,4,5-tetrahydro-[1,2,4]triazin-6-yloxy)propoxy]phenylsulfanyl}-2-methyl-propionate
35. Ethyl 2-(3-{3-[3,5-dioxo-2,4-bis-(4,4,4-trifluorobutyl)-2,3,4,5-tetrahydro-[1,2,4]triazin-6-yloxy]propoxy}phenoxy)-2-methyl-propionate
36. Ethyl 2-{3-[4-(2-heptyl-4-methyl-3,5-dioxo-2,3,4,5-tetrahydro-[1,2,4]triazin-6-yloxy)butoxy]phenoxy}-2-methyl-propionate
37. Ethyl 2-{3-[4-(2-heptyl-4-methyl-3,5-dioxo-2,3,4,5-tetrahydro-[1,2,4]triazin-6-yloxy)butoxy]phenylsulfanyl}-2-methyl-propionate
38. Ethyl 2-{4-[2-(2-heptyl-4-methyl-3,5-dioxo-2,3,4,5-tetrahydro-[1,2,4]triazin-6-yloxy)ethoxy]phenylsulfanyl}-2-methyl-propionate
39. Ethyl 2-(4-13-[3,5-dioxo-2,4-bis-(4,4,4-trifluorobutyl)-2,3,4,5-tetrahydro-[1,2,4]triazin-6-yloxy]propoxy}phenoxy)-2-methyl-propionate
40. Ethyl 2-{4-[2-(2-heptyl-4-methyl-3,5-dioxo-2,3,4,5-tetrahydro-[1,2,4]triazin-6-ylamino)ethoxy]phenoxy}-2-methyl-propionate
41. Ethyl 2-(4-{2-[3,5-dioxo-2,4-bis-(4,4,4-trifluorobutyl)-2,3,4,5-tetrahydro-[1,2,4]triazin-6-ylamino]ethoxy}phenoxy)-2-methyl-propionate
42. Ethyl 2-{4-[2-(2,4-diheptyl-3,5-dioxo-2,3,4,5-tetrahydro-[1,2,4]triazin-6-ylamino)ethoxy]phenoxy}-2-methyl-propionate
43. Ethyl 2-(4-{2-[2,4-bis-(3-cyclohexyl-propyl)-3,5-dioxo-2,3,4,5-tetrahydro-[1,2,4]triazin-6-ylamino]ethoxy}phenoxy)-2-methyl-propionate
44. Ethyl 2-methyl-2-(4-{3-[4-methyl-3,5-dioxo-2-(4,4,4-trifluoro-butyl)-2,3,4,5-tetrahydro-[1,2,4]triazin-6-ylamino]propoxy}phenoxy)propionate
45. Ethyl 2-(4-[3-(2-heptyl-4-methyl-3,5-dioxo-2,3,4,5-tetrahydro-[1,2,4]triazin-6-ylamino)propoxy]phenoxy}-2-methyl-propionate
46. Ethyl 2-methyl-2-(4-{3-[2-methyl-3,5-dioxo-4-(4,4,4-trifluoro-butyl)-2,3,4,5-tetrahydro-[1,2,4]triazin-6-ylamino]propoxy}phenoxy)propionate
47. Ethyl 2-{4-[3-(4-heptyl-2-methyl-3,5-dioxo-2,3,4,5-tetrahydro-[1,2,4]triazin-6-ylamino)propoxy]phenoxy}-2-methyl-propionate
48. Ethyl 2-(4-{3-[4-heptyl-3,5-dioxo-2-(4,4,4-trifluoro-butyl)-2,3,4,5-tetrahydro-[1,2,4]triazin-6-ylamino]propoxy}phenoxy)-2-methyl-propionate
49. Ethyl 2-(4-[3-(4-heptyl-2-methyl-3,5-dioxo-2,3,4,5-tetrahydro-[1,2,4]triazin-6-ylamino)propoxy]phenylsulfanyl}-2-methyl-propionate
50. Ethyl 2-{4-[4-(4-heptyl-2-methyl-3,5-dioxo-2,3,4,5-tetrahydro-[1,2,4]triazin-6-ylamino)butoxy]phenoxy}-2-methyl-propionate
51. Ethyl 2-(3-{3-[(2-heptyl-4-methyl-3,5-dioxo-2,3,4,5-tetrahydro-[1,2,4]triazin-6-yl)-(4,4,4-trifluoro-butyl)amino]propoxy}phenoxy)-2-methyl-propionate
52. Ethyl 2-(3-(3-[(2,4-dimethyl-3,5-dioxo-2,3,4,5-tetrahydro-[1,2,4]triazin-6-yl)heptyl-amino]propoxy}phenylsulfanyl)-2-methyl-propionate
53. Ethyl 2-(4-{3-[(2,4-dimethyl-3,5-dioxo-2,3,4,5-tetrahydro-[1,2,4]triazin-6-yl)heptyl-amino]propoxy}phenylsulfanyl)-2-methyl-propionate
54. Ethyl 2-(3-{4-[(2,4-dimethyl-3,5-dioxo-2,3,4,5-tetrahydro-[1,2,4]triazin-6-yl)heptyl-amino]butoxy}phenylsulfanyl)-2-methyl-propionate
55. Ethyl 2-(2-{2-[3,5-dioxo-2,4-bis-(4,4,4-trifluorobutyl)-2,3,4,5-tetrahydro-[1,2,4]triazin-6-ylamino]ethyl}phenoxy)-2-methyl-propionate
56. Ethyl 2-methyl-2-(3-{2-[4-methyl-3,5-dioxo-2-(4,4,4-trifluoro-butyl)-2,3,4,5-tetrahydro-[1,2,4]triazin-6-ylamino]ethyl}phenoxy)propionate
57. Ethyl 2-{3-[2-(2-heptyl-4-methyl-3,5-dioxo-2,3,4,5-tetrahydro-[1,2,4]triazin-6-ylamino)ethyl]phenoxy}-2-methyl-propionate
58. Ethyl 2-methyl-2-(3-{2-[2-methyl-3,5-dioxo-4-(4,4,4-trifluoro-butyl)-2,3,4,5-tetrahydro-[1,2,4]triazin-6-ylamino]ethyl}phenoxy)propionate
59. Ethyl 2-{3-[2-(4-heptyl-2-methyl-3,5-dioxo-2,3,4,5-tetrahydro-[1,2,4]triazin-6-ylamino)ethyl]phenoxy}-2-methyl-propionate
60. Ethyl 2-(3-{2-[4-heptyl-3,5-dioxo-2-(4,4,4-trifluoro-butyl)-2,3,4,5-tetrahydro-[1,2,4]triazin-6-ylamino]ethyl}phenoxy)-2-methyl-propionate
61. Ethyl 2-{3-[2-(4-heptyl-2-methyl-3,5-dioxo-2,3,4,5-tetrahydro-[1,2,4]triazin-6-ylamino)ethyl]phenylsulfanyl}-2-methyl-propionate
62. Ethyl 2-methyl-2-(3-{3-[4-methyl-3,5-dioxo-2-(4,4,4-trifluoro-butyl)-2,3,4,5-tetrahydro-[1,2,4]triazin-6-ylamino]propyl}phenoxy)propionate
63. Ethyl 2-(3-{3-[2-(2-cyano-ethyl)-4-methyl-3,5-dioxo-2,3,4,5-tetrahydro-[1,2,4]triazin-6-ylamino]propyl}phenoxy)-2-methyl-propionate
64. Ethyl 2-{3-[3-(2-heptyl-4-methyl-3,5-dioxo-2,3,4,5-tetrahydro-[1,2,4]triazin-6-ylamino)propyl]phenoxy}-2-methyl-propionate
65. Ethyl 2-methyl-2-(3-{3-[2-methyl-3,5-dioxo-4-(4,4,4-trifluoro-butyl)-2,3,4,5-tetrahydro-[1,2,4]triazin-6-ylamino]propyl}phenoxy)propionate
66. Ethyl 2-(3-{3-[3,5-dioxo-2,4-bis-(4,4,4-trifluorobutyl)-2,3,4,5-tetrahydro-[1,2,4]triazin-6-ylamino]propyl}phenoxy)-2-methyl-propionate
67. Ethyl 2-{3-[3-(4-heptyl-2-methyl-3,5-dioxo-2,3,4,5-tetrahydro-[1,2,4]triazin-6-ylamino)propyl]phenoxy}-2-methyl-propionate
68. Ethyl 2-(3-{3-[4-heptyl-3,5-dioxo-2-(4,4,4-trifluoro-butyl)-2,3,4,5-tetrahydro-[1,2,4]triazin-6-ylamino]propyl}phenoxy)-2-methyl-propionate
69. Ethyl 2-{3-[3-(2,4-diheptyl-3,5-dioxo-2,3,4,5-tetrahydro-[1,2,4]triazin-6-ylamino)propyl]phenoxy}-2-methyl-propionate
70. Ethyl 2-{3-[3-(2-benzyl-4-heptyl-3,5-dioxo-2,3,4,5-tetrahydro-[1,2,4]triazin-6-ylamino)propyl]phenoxy}-2-methyl-propionate
71. Ethyl 2-(3-{3-[4-benzyl-3,5-dioxo-2-(4,4,4-trifluoro-butyl)-2,3,4,5-tetrahydro-[1,2,4]triazin-6-ylamino]propyl}phenoxy)-2-methyl-propionate
72. Ethyl 2-{3-[3-(4-benzyl-2-heptyl-3,5-dioxo-2,3,4,5-tetrahydro-[1,2,4]triazin-6-ylamino)propyl]phenoxy}-2-methyl-propionate
73. Ethyl 2-{3-[3-(4-heptyl-2-methyl-3,5-dioxo-2,3,4,5-tetrahydro-[1,2,4]triazin-6-ylamino)propyl]phenylsulfanyl}-2-methyl-propionate
74. Ethyl 2-methyl-2-(3-{4-[4-methyl-3,5-dioxo-2-(4,4,4-trifluoro-butyl)-2,3,4,5-tetrahydro-[1,2,4]triazin-6-ylamino]butyl}phenoxy)propionate
75. Ethyl 2-(3-[4-(2-heptyl-4-methyl-3,5-dioxo-2,3,4,5-tetrahydro-[1,2,4]triazin-6-ylamino)butyl]phenoxy)-2-methyl-propionate
76. Ethyl 2-methyl-2-(3-{4-[4-(3-methyl-but-2-enyl)-3,5-dioxo-2-(4,4,4-trifluoro-butyl)-2,3,4,5-tetrahydro-[1,2,4]triazin-6-ylamino]butyl}phenoxy)propionate
77. Ethyl 2-methyl-2-(3-{4-[2-methyl-3,5-dioxo-4-(4,4,4-trifluoro-butyl)-2,3,4,5-tetrahydro-[1,2,4]triazin-6-ylamino]butyl}phenoxy)propionate
78. Tert-butyl 2-methyl-2-(3-{4-[2-methyl-3,5-dioxo-4-(4,4,4-trifluoro-butyl)-2,3,4,5-tetrahydro-[1,2,4]triazin-6-ylamino]butyl}phenoxy)propionate
79. 2-Methyl-2-(3-{4-[2-methyl-3,5-dioxo-4-(4,4,4-trifluoro-butyl)-2,3,4,5-tetrahydro-[1,2,4]triazin-6-ylamino]butyl}phenoxy)propionic acid
80. Ethyl 2-(3-{4-[3,5-dioxo-2,4-bis-(4,4,4-trifluorobutyl)-2,3,4,5-tetrahydro-[1,2,4]triazin-6-ylamino]butyl}phenoxy)-2-methyl-propionate
81. Ethyl 2-(3-{4-[2-(2-cyano-ethyl)-3,5-dioxo-4-(4,4,4-trifluoro-butyl)-2,3,4,5-tetrahydro-[1,2,4]triazin-6-ylamino]butyl}phenoxy)-2-methyl-propionate
82. Ethyl 2-(3-{4-[2-heptyl-3,5-dioxo-4-(4,4,4-trifluoro-butyl)-2,3,4,5-tetrahydro-[1,2,4]triazin-6-ylamino]butyl}phenoxy)-2-methyl-propionate
83. Tert-butyl 2-(3-{4-[2-heptyl-3,5-dioxo-4-(4,4,4-trifluoro-butyl)-2,3,4,5-tetrahydro-[1,2,4]triazin-6-ylamino]butyl}phenoxy)-2-methyl-propionate
84. 2-(3-{4-[2-Heptyl-3,5-dioxo-4-(4,4,4-trifluorobutyl)-2,3,4,5-tetrahydro-[1,2,4]triazin-6-ylamino]butyl}phenoxy)-2-methyl-propionic acid
85. Ethyl 2-(3-{4-[4-heptyl-3,5-dioxo-2-(4,4,4-trifluoro-butyl)-2,3,4,5-tetrahydro-[1,2,4]triazin-6-ylamino]butyl}phenoxy)-2-methyl-propionate
86. Ethyl 2-(3-{4-[2-(2-cyano-ethyl)-4-heptyl-3,5-dioxo-2,3,4,5-tetrahydro-[1,2,4]triazin-6-ylamino]butyl}phenoxy)-2-methyl-propionate
87. Ethyl 4-(6-{4-[3-(1-ethoxycarbonyl-1-methyl-ethoxy)phenyl]butylamino}-4-heptyl-3,5-dioxo-4,5-dihydro-3H-[1,2,4]triazin-2-yl)-but-2-enonate
88. Ethyl 2-{3-[4-(2,4-diheptyl-3,5-dioxo-2,3,4,5-tetrahydro-[1,2,4]triazin-6-ylamino)butyl]phenoxy}-2-methyl-propionate
89. Ethyl 2-{3-[4-(2-benzyl-4-heptyl-3,5-dioxo-2,3,4,5-tetrahydro-[1,2,4]triazin-6-ylamino)butyl]phenoxy}-2-methyl-propionate
90. Ethyl 2-(3-{4-[4-benzyl-3,5-dioxo-2-(4,4,4-trifluoro-butyl)-2,3,4,5-tetrahydro-[1,2,4]triazin-6-ylamino]butyl}phenoxy)-2-methyl-propionate
91. Ethyl 2-{3-[4-(4-benzyl-2-heptyl-3,5-dioxo-2,3,4,5-tetrahydro-[1,2,4]triazin-6-ylamino)butyl]phenoxy}-2-methyl-propionate
92. Ethyl 2-(3-{5-[3,5-dioxo-2,4-bis-(4,4,4-trifluorobutyl)-2,3,4,5-tetrahydro-[1,2,4]triazin-6-ylamino]pentyl}phenoxy)-2-methyl-propionate
93. Ethyl 2-methyl-2-(3-{4-[4-methyl-3,5-dioxo-2-(4,4,4-trifluoro-butyl)-2,3,4,5-tetrahydro-[1,2,4]triazin-6-ylamino]butyl}phenylsulfanyl)propionate
94. Ethyl 2-{3-[4-(2-heptyl-4-methyl-3,5-dioxo-2,3,4,5-tetrahydro-[1,2,4]triazin-6-ylamino)butyl]phenylsulfanyl}-2-methyl-propionate
95. Ethyl 2-methyl-2-(3-{4-[2-methyl-3,5-dioxo-4-(4,4,4-trifluoro-butyl)-2,3,4,5-tetrahydro-[1,2,4]triazin-6-ylamino]butyl}phenylsulfanyl)propionate
96. 2-Methyl-2-(3-{4-[2-methyl-3,5-dioxo-4-(4,4,4-trifluoro-butyl)-2,3,4,5-tetrahydro-[1,2,4]triazin-6-ylamino]butyl}phenylsulfanyl)propionic acid
97. Ethyl 2-(3-{4-[4-heptyl-3,5-dioxo-2-(4,4,4-trifluoro-butyl)-2,3,4,5-tetrahydro-[1,2,4]triazin-6-ylamino]butyl}phenylsulfanyl)-2-methyl-propionate
98. Ethyl 2-{3-[4-(2,4-diheptyl-3,5-dioxo-2,3,4,5-tetrahydro-[1,2,4]triazin-6-ylamino)butyl]phenylsulfanyl}-2-methyl-propionate
99. Ethyl 2-{3-[4-(2-benzyl-4-heptyl-3,5-dioxo-2,3,4,5-tetrahydro-[1,2,4]triazin-6-ylamino)butyl]phenylsulfanyl}-2-methyl-propionate
100. Ethyl 2-(3-{4-[4-benzyl-3,5-dioxo-2-(4,4,4-trifluoro-butyl)-2,3,4,5-tetrahydro-[1,2,4]triazin-6-ylamino]butyl}phenylsulfanyl)-2-methyl-propionate
101. Ethyl 2-{3-[4-(4-benzyl-2-heptyl-3,5-dioxo-2,3,4,5-tetrahydro-[1,2,4]triazin-6-ylamino)butyl]phenylsulfanyl}-2-methyl-propionate
102. Ethyl 2-methyl-2-(4-{2-[4-methyl-3,5-dioxo-2-(4,4,4-trifluoro-butyl)-2,3,4,5-tetrahydro-[1,2,4]triazin-6-ylamino]ethyl}phenoxy)propionate
103. Ethyl 2-{4-[2-(2-heptyl-4-methyl-3,5-dioxo-2,3,4,5-tetrahydro-[1,2,4]triazin-6-ylamino)ethyl]phenoxy}-2-methyl-propionate
104. Ethyl 4-[6-{2-[4-(1-ethoxycarbonyl-1-methyl-ethoxy)phenyl]ethylamino}-3,5-dioxo-4-(4,4,4-trifluoro-butyl)-4,5-dihydro-3H-[1,2,4]triazin-2-yl]-but-2-enoate
105. Ethyl 2-(4-{2-[2-(3-cyclohexyl-propyl)-3,5-dioxo-4-(4,4,4-trifluoro-butyl)-2,3,4,5-tetrahydro-[1,2,4]triazin-6-ylamino]ethyl}phenoxy)-2-methyl-propionate
106. Ethyl 2-{4-[2-(4-heptyl-2-methyl-3,5-dioxo-2,3,4,5-tetrahydro-[1,2,4]triazin-6-ylamino)ethyl]phenoxy}-2-methyl-propionate
107. Ethyl 2-(4-{2-[4-heptyl-3,5-dioxo-2-(4,4,4-trifluoro-butyl)-2,3,4,5-tetrahydro-[1,2,4]triazin-6-ylamino]ethyl}phenoxy)-2-methyl-propionate
108. Ethyl 2-{4-[2-(2,4-diheptyl-3,5-dioxo-2,3,4,5-tetrahydro-[1,2,4]triazin-6-ylamino)ethyl]phenoxy}-2-methyl-propionate
109. Ethyl 2-{4-[2-(4-benzyl-2-heptyl-3,5-dioxo-2,3,4,5-tetrahydro-[1,2,4]triazin-6-ylamino)ethyl]phenoxy}-2-methyl-propionate
110. Ethyl 2-{4-[2-(2,4-bis-benzyloxymethyl-3,5-dioxo-2,3,4,5-tetrahydro-[1,2,4]triazin-6-ylamino)ethyl]phenoxy}-2-methyl-propionate
111. Ethyl 2-(4-{2-[(2,4-dimethyl-3,5-dioxo-2,3,4,5-tetrahydro-[1,2,4]triazin-6-yl)-(4,4,4-trifluorobutyl)amino]ethyl}phenoxy)-2-methyl-propionate
112. Ethyl 2-(4-{2-[(2,4-dimethyl-3,5-dioxo-2,3,4,5-tetrahydro-[1,2,4]triazin-6-yl)heptyl-amino]ethyl}phenoxy)-2-methyl-propionate
113. 2-(4-{2-[(2,4-Dimethyl-3,5-dioxo-2,3,4,5-tetrahydro-[1,2,4]triazin-6-yl)heptyl-amino]ethyl}phenoxy)-2-methyl-propionic acid
114. Ethyl 2-(4-{2-[(2,4-dimethyl-3,5-dioxo-2,3,4,5-tetrahydro-[1,2,4]triazin-6-yl)phenethyl-amino]ethyl}phenoxy)-2-methyl-propionate
115. Ethyl 2-(4-{2-[(2,4-dimethyl-3,5-dioxo-2,3,4,5-tetrahydro-[1,2,4]triazin-6-yl)-(3-phenylpropyl)aminolethyl}phenoxy)-2-methyl-propionate
116. Ethyl 2-(4-{2-[(4-heptyl-2-methyl-3,5-dioxo-2,3,4,5-tetrahydro-[1,2,4]triazin-6-yl)phenethyl-amino]ethyl}phenoxy)-2-methyl-propionate
117. Ethyl 2-(4-{2-[(4-heptyl-2-methyl-3,5-dioxo-2,3,4,5-tetrahydro-[1,2,4]triazin-6-yl)-(3-phenylpropyl)amino]ethyl}phenoxy)-2-methyl-propionate
118. 2-Methyl-2-(4-{2-[4-methyl-3,5-dioxo-2-(4,4,4-trifluoro-butyl)-2,3,4,5-tetrahydro-[1,2,4]triazin-6-ylamino]ethyl}phenylsulfanyl)propionic acid
119. Ethyl 2-{4-[2-(2-heptyl-4-methyl-3,5-dioxo-2,3,4,5-tetrahydro-[1,2,4]triazin-6-ylamino)ethyl]phenylsulfanyl}-2-methyl-propionate
120. 2-{4-[2-(2-Heptyl-4-methyl-3,5-dioxo-2,3,4,5-tetrahydro-[1,2,4]triazin-6-ylamino)ethyl]phenylsulfanyl}-2-methyl-propionic acid
121. 2-{4-[2-(4-Butyl-2-heptyl-3,5-dioxo-2,3,4,5-tetrahydro-[1,2,4]triazin-6-ylamino)ethyl]phenylsulfanyl}-2-methyl-propionic acid
122. Ethyl 2-{4-[2-(4-heptyl-2-methyl-3,5-dioxo-2,3,4,5-tetrahydro-[1,2,4]triazin-6-ylamino)ethyl]phenylsulfanyl}-2-methyl-propionate
123. 2-(4-{2-[4-Heptyl-3,5-dioxo-2-(4,4,4-trifluorobutyl)-2,3,4,5-tetrahydro-[1,2,4]triazin-6-ylamino]ethyl}phenylsulfanyl)-2-methyl-propionic acid
124. Ethyl 2-{4-[3-(2-heptyl-4-methyl-3,5-dioxo-2,3,4,5-tetrahydro-[1,2,4]triazin-6-ylamino)propyl]phenylsulfanyl}-2-methyl-propionate

6. Method of preparation of the chemical compounds according to one of the claims 1) to 5) wherein:
a derivative of general formula II is condensed 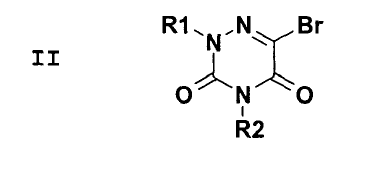
in which **R₁** and **R₂** represent the groups as previously described in formula **I** with a derivative of general formula **III** 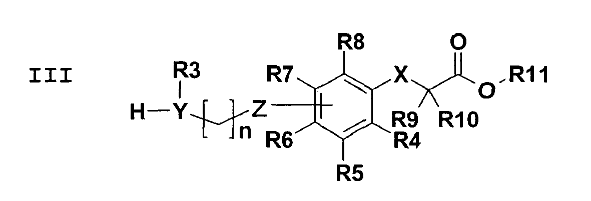 where **YR₃, n, Z, X, R₄, R₅, R₆, R₇, R₈, R₉, R₁₀,** and **R₁₁** are such as described previously in formula **I**, notably in the presence of a base such as triethylamine in n-butanol (when **Y**=N) or potassium carbonate in dimethylformamide (when **YR₃**=O).

7. Method of preparation according to one of the claims **1**) to **5)** of the chemical compounds of general formula **I** in which **Z**=O wherein:
a) a derivative of general formula **II** is condensed 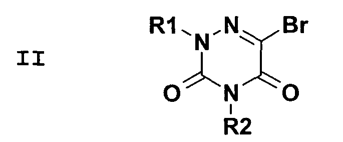 in which **R₁** and **R₂** represent the groups as previously described in formula **I** with a derivative of general formula **IV** 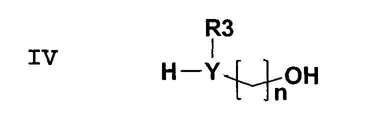 in which **R₃Y** can be equal to NH or O and **n** is such as described previously in formula **I,** notably in the absence of solvent without adding base (in the case where **R₃Y**=NH) or in the presence of a base such as K₂CO₃ (in the case where **R₃Y**=O)**.**
b) the derivative obtained **V** is condensed 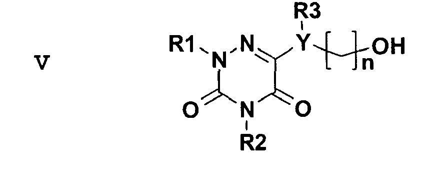 with a compound of general formula **VI** 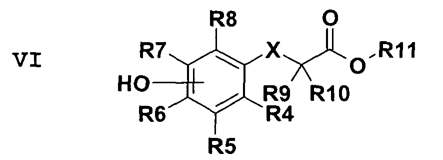
where **X, R₄, R₅, R₆, R₇, R₈, R₉, R₁₀**, and **R₁₁** are as described previously in formula **I**, notably under conditions such as those of the Mitsunobu reaction in the presence of triphenylphosphine and diethylazodicarboxylate in THF.

8. Method of preparation according to one of the claims **1)** to **5)** of the chemical compounds of general formula I in which **Z**=O wherein:
a) the alcohol function of a derivative of general formula **VII** is protected 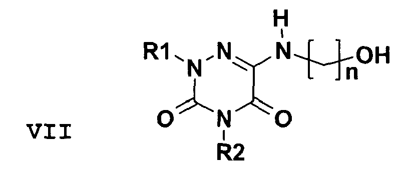 in which **R₁, R₂,** and n are such as described previously in formula **I** by a protection group such as tertbutyldimethylsilane, in particular under conditions such as THF using chlorotertbutyldimethylsilane and imidazole. 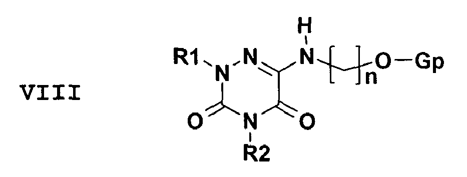
b) the nitrogen of compound **VIII** previously obtained is alkylated by a halogenated derivative R₃Hal in which the **Hal** group represents a halogen such as Cl, Br or **I** and **R₃** is as described previously in formula **I,** under operating conditions such as NaH or tBuOK in DMF. 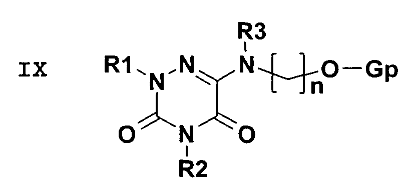
c) the compound **IX** thus obtained is deprotected under operating conditions such as tetrabutylammonium fluoride in THF.
d) the derivative obtained **X** is condensed 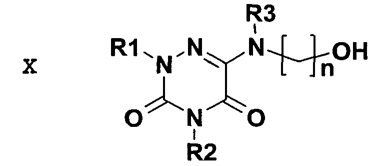 with a compound of general formula VI 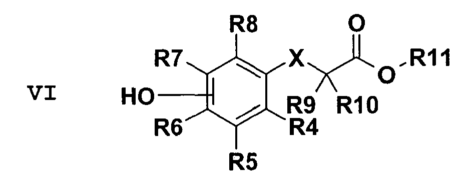
where **X, R₄, R₅, R₆, R₇, R₈, R₉, R₁₀**, and **R₁₁** are as described previously in formula **I,** notably under conditions such as those of the Mitsunobu reaction in the presence of triphenylphosphine and diethylazodicarboxylate in THF.

9. Method of preparation of chemical compounds of general formula **I** according to one of the claims **1)** to **5)** when **Y**=N and which **Z**=C wherein:
a) a derivative of general formula **II** is condensed 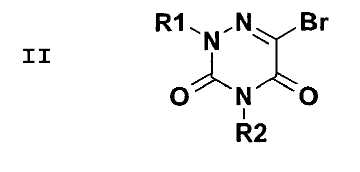 in which **R₁** and **R₂** represent the groups as previously described in formula **I** with a derivative of general formula **XI** 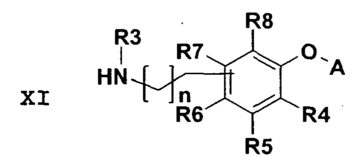 in which **n, R₃ R₄, R₅, R₆, R₇**, and **R₈** are as described previously in formula I and **A** can be hydrogen or a methyl group, notably in the presence of a base such as triethylamine in n-butanol.
b) after demethylation (if **A=**Me, under conditions such as BBr₃ in dichloromethane), the phenol function of derivative **XII** is alkylated 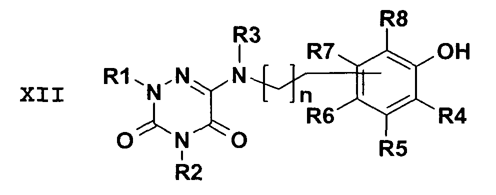 by a halogenated derivative of general formula **XIII** (used as a solvent in the presence of a base such as potassium carbonate) 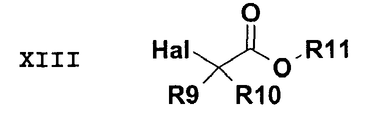
in which the **Hal** group represents a halogen such as Cl, Br or **I**, and **R₉, R₁₀,** and **R₁₁** are as previously described in general formula **I.**

10. Method of preparation of chemical compounds of general formula **I** according to one of the claims **1)** to **5)** when **Y**=N and **Z**=C wherein:
a) a derivative of general formula **XIV** is alkylated 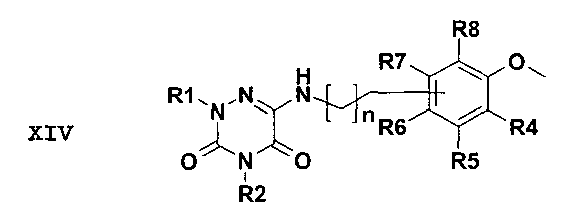 in which **R₁₁ R₂, R₄, R₅, R₆, R₇, R₈**, and n are as described previously in formula I with a derivative of formula R₃Hal in which the **Hal** group represents a halogen such as Cl, Br or I and **R₃** is as described previously in formula **I,** under operating conditions such as NaH or tBuOK in DMF.
b) after demethylation under conditions such as BBr₃ in dichloromethane, the phenol function of the derivative **XII** thus obtained is alkylated 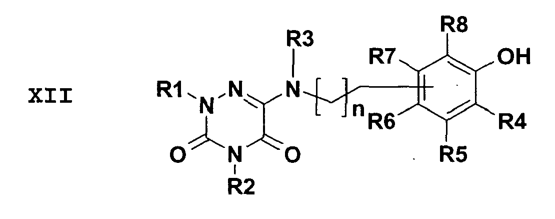 by a halogenated derivative of general formula **XIII** (used as a solvent in the presence of a base such as potassium carbonate) 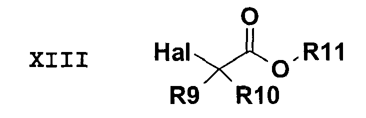
in which the **Hal** group represents a halogen such as Cl, Br or **I**, and **R₉, R₁₀,** and **R₁₁** are as previously described in general formula **I.**

11. Method of preparation of the chemical compounds according to one of the claims **1)** to **5)** wherein:
a) a derivative of general formula I is placed 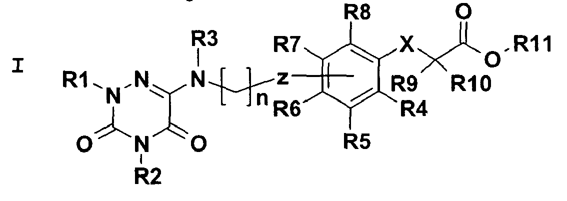 in which **R₁**= (CH₂)₂CN and **R₂, R₃, n, Z, X, R₄, R₅, R₆, R₇**, **R₈, R₉, R₁₀**, and **R₁₁** are as described previously in formula I or **R₂**=(CH₂)₂CN and **R₁, R₃, n, Z, X, R₄, R₅, R₆**, **R₇, R₈, R₉, R₁₀**, and **R₁₁** are as described previously in formula **I** under operating conditions such as in the presence of a base NaH in DMF.
b) the nitrogen of the triazine of derivative **XIVa** or **XIVb** thus obtained is then alkylated 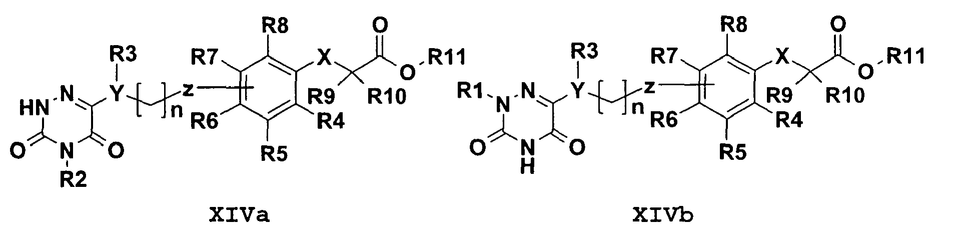 by a halogenated derivative of general formula **R₁Hal** in the case of the intermediate **XIVa** and of general formula **R₂Hal** in the case of the intermediate **XIVb** in which the **Hal** group represents a halogen such as Cl, Br or **I** and **R₁** and **R₂** are as described previously in formula **I,** under operating conditions such as in the presence of NaH or tBuOK in DMF.

12. As novel medicines of use in the treatment of disorders requiring PPAR alpha and/or PPAR gamma receptor agonists, the compounds defined according to one of the claims **1)** to **5).**

13. As novel medicines of use in the prevention and the treatment of diseases such as diabetic dyslipidemia, hypertriglyceridemia, hypercholesterolemia, hyperinsulinemia, hyperglycemia, metabolic syndrome, obesity and atherosclerosis, the compounds defined according to one of the claims **1)** to **5) .**

14. **As** novel medicines of use in dermatology in pathologies with an inflammatory component or resulting from abnormal cell differentiation, the compounds defined according to one of the claims **1)** to **5).**

15. As novel medicines of use in the treatment of diseases such as psoriasis, acne, atopic dermatitis, cutaneous aging and photoaging, the compounds defined according to one of the claims **1)** to **5).**

16. Pharmaceutical compound wherein it contains as an active ingredient a compound defined according to one of the claims **1)** to **5)** in association with a suitable excipient.

## Patentansprüche

1. Derivate von 3,5-Dioxo-(2H,4H)-1,2,4-triazin der allgemeinen Formel **I**, 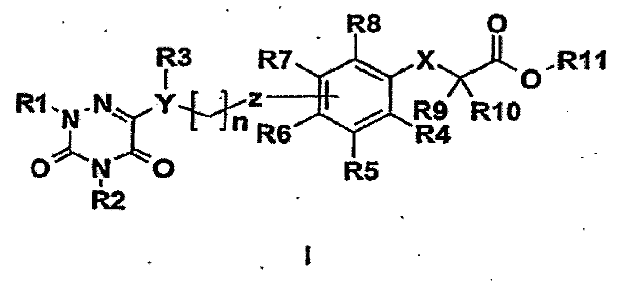 wobei:
- **R₁** und **R₂** gleich oder unterschiedlich sein können und ein lineares oder verzweigtes C₁-C₇-Alkyl- oder Alkenylradikal, ein C₁-C₆-Alkylradikal, substituiert durch Gruppen wie Trifluormethyl, C₅-C₆-Cycloalkyl, Nitril, C₁-C₄-Alkoxycarbonylvinyl, Hydrocarbonylvinyl, C₁-C₄-Alkoxycarbonyl, Carboxylat, Benzyloxy oder Phenyl (für die der Phenylkern eventuell durch eine oder mehrere Gruppen wie C₁-C₄-Alcoyl, C₁-C₄-Alkoxy, Nitro, Halogen, Trifluormethyl, substituiert ist), darstellen,
- **YR₃** Sauerstoff oder NR₃ darstellt, wobei **R₃** Wasserstoff, ein lineares oder verzweigtes C₁-C₇-Alcoyl- oder Alkenylradikal, ein C₁-C₆-Alkylradikal, substituiert durch Gruppen wie Trifluormethyl oder Phenyl (für die der Phenylkern eventuell durch eine oder mehrere Gruppen wie C₁-C₄-Alcoyl, C₁-C₄-Alkoxy, Nitro, Halogen, Trifluormethyl substituiert ist), darstellt,
- **Z** ein Sauerstoffatom oder ein Kohlenstoffatom darstellt, das mit den Ortho-, Meta- oder Parapositionen der Phenylgruppe der Formel **I** verbunden sein kann,
- **n** gleich 0 bis 5 sein kann, wenn Z=C oder 2 bis 4 wenn **Z**=O,
- **X** Sauerstoff oder Schwefel darstellt,
- **R₄, R₅, R₆, R₇** und **R₈** Wasserstoff oder Fluor darstellen,
- **R₉, R₁₀** und **R₁₁** Wasserstoff oder eine lineare oder verzweigte C₁-C₅-Alkylgruppe darstellen
sowie die Zuschlagsalze mit den pharmazeutisch akzeptablen Basen und die verschiedenen Enantiomere der Verbindungen, die asymmetrische Kohlenstoffe enthalten, sowie ihre Gemische in allen Verhältnissen unter besonderem Einschluss der Racemate.

2. Derivate von 3,5-Dioxo-(2H,4H)-1,2,4-triazin der allgemeinen Formel **I** nach Anspruch 1, wobei
- **R₁** und **R₂** unabhängig voneinander ein lineares oder verzweigtes C₁-C₇-Alkyl- oder Alkenylradikal, ein C₁-C₆-Alkylradikal, substituiert durch Gruppen wie Trifluormethyl, C₆-Cycloalkyl, Nitril, Phenyl (für die der Phenylkern eventuell durch eine oder mehrere Gruppen wie C₁-C₄-Alcoyl, C₁-C₄-Alkoxy, Nitro, Halogen, Trifluormethyl substituiert ist), darstellen,
- **YR₃** Sauerstoff oder **NR₃** darstellt, wobei **R₃** Wasserstoff, ein lineares oder verzweigtes C₁-C₇-Alcoyl- oder Alkenylradikal, substituiert durch Gruppen wie Trifluormethyl oder Phenyl, darstellt,
- **Z** ein Sauerstoffatom oder ein Kohlenstoffatom darstellt, das mit den Ortho-, Meta- oder Parapositionen der Phenylgruppe der Formel **I** verbunden sein kann,
- **n** gleich 0 bis 5 sein kann, wenn **Z**=C oder 2 bis 4 wenn **Z**=O,
- **X** Sauerstoff oder Schwefel darstellt,
- **R₄, R₅, R₆, R₇** und **R₈** Wasserstoff oder Fluor darstellen,
- **R₉, R₁₀** und **R₁₁** Wasserstoff oder eine lineare oder verzweigte C₁-C₅-Alkylgruppe darstellen.

3. Derivate von 3,5-Dioxo-(2H,4H)-1,2,4-triazin der allgemeinen Formel **I** nach einem der Ansprüche 1 und 2, wobei
- R₁ und R₂ unabhängig voneinander ein lineares oder verzweigtes C₁-C₇-Alkyl- oder Alkenylradikal, ein C₁-C₆-Alkylradikal, substituiert durch Gruppen wie Trifluormethyl oder Nitril, darstellen,
- **YR₃** Sauerstoff oder **NR₃** darstellt, wobei **R₃** Wasserstoff oder ein lineares oder verzweigtes C₁-C₇-Alcoylradikal darstellt,
- **Z** ein Kohlenstoffatom darstellt, das mit den Ortho-, Meta- oder Parapositionen der Phenylgruppe der Formel **I** verbunden sein kann,
- **n** gleich 0 bis 5 sein kann,
- **X** Sauerstoff oder Schwefel darstellt,
- **R₄, R₅, R₆, R₇** und **R₈** Wasserstoff oder Fluor darstellen,
- **R₉, R₁₀** und **R₁₁** Wasserstoff oder eine lineare oder verzweigte C₁-C₅-Alkylgruppe darstellen, insbesondere **R₉** und **R₁₀** stellen die Methylgruppe dar und **R₁₁** Wasserstoff oder die Ethylgruppe.

4. Derivate von 3,5-Dioxo-(2H,4H)-1,2,4-triazin der allgemeinen Formel **I** nach einem der Ansprüche 1 und 3, wobei
- **R₁** und **R₂** unabhängig voneinander ein lineares oder verzweigtes C₁-C₇-Alkyl - oder Alkenylradikal darstellen, das eventuell am Ende der Kette durch eine Trifluormethylgruppe substituiert ist,
- **YR₃** Sauerstoff oder **NR₃** darstellt, wobei **R₃** Wasserstoff oder ein lineares oder verzweigtes C₁-C₇-Alcoylradikal darstellt,
- **Z** ein Kohlenstoffatom darstellt, das mit den Meta- oder Parapositionen der Phenylgruppe der Formel **I** verbunden sein kann,
- **n** gleich 1 bis 5 sein kann,
- **X** Sauerstoff oder Schwefel darstellt,
- **R₄** bis **R₈** Wasserstoff darstellen,
- **R₉** und **R₁₀** ein Methylradikal darstellen,
- **R₁₁** Wasserstoff oder ein Ethylradikal darstellt.

5. Verbindungen der allgemeinen Formel **I** nach Anspruch 1, **dadurch gekennzeichnet, dass** sie ausgewählt sind aus:
1. Ethyl-2-{2-[2-(4-butyl-2-methyl-3,5-dioxo-. 2,3,4,5-tetrahydro-[1,2,4]triazin-6-yloxy)-ethyl]-phenoxy}-2-methylpropionat
2. Ethyl-2-{3-[2-(2-heptyl-4-methyl-3,5-dioxo-2,3,4,5-tetrahydro-[1,2,4]triazin-6-yloxy)-ethyl]-phenoxy}-2-methylpropionat
3. Ethyl-2-methyl-2-(3-{2-[2-methyl-3,5-dioxo-4-(4,4,4-trifluoro-butyl)-2,3,4,5-tetrahydro-[1,2,4]triazin-6-yloxy]-ethyl}-phenoxy)-propionat
4. Ethyl-2-methyl-2-(3-{3-[4-methyl-3,5-dioxo-2-(4,4,4-trifluoro-butyl)-2,3,4,5-tetrahydro-[1,2,4]triazin-6-yloxy]-propyl}-phenoxy)-propionat
5. Ethyl-2-{3-[3-(2-heptyl-4-methyl-3,5-dioxo-2,3,4,5-tetrahydro-[1,2,4]triazin-6-yloxy)-propyl]-phenoxy}-2-methylpropionat
6. Ethyl-2-methyl-2-(3-{3-[2-methyl-3,5-dioxo-4-(4,4,4-trifluoro-butyl)-2,3,4,5-tetrahydro-[l,2,4]triazin-6-yloxy]-propyl}-phenoxy)-propionat
7. Ethyl-2-{3-[3-(4-heptyl-2-methyl-3,5-dioxo-2,3,4,5-tetrahydro-[1,2,4}triazin-6-yloxy)-propyl]-phenoxy}-2-methylpropionat
8. Ethyl-2-(3-(3-[4-heptyl-3,5-dioxo-2-(4,4,4-trifluoro-butyl)-2,3,4,5-tetrahydro-[1,2,4]triazin-6-yloxy]-propyl}-phenoxy}2-methylpropionat
9. Ethyl-2-methyl-2-(3-{5-[4-methyl-3,5-dioxo-2-(4,4,4-trifluoro-butyl)-2,3,4,5-tetrahydro-[1,2,4]triazin-6-yloxy]-pentyl}-phenoxy}propionat
10. Ethyl-2-{3-[5-(2-heptyl-4-methyl-3,5-dioxo-2,3,4,5-tetrahydro-[1,2,4]triazin-6-yloxy)-pentyl]-phenoxy}-2-methylpropionat
11. Ethyl-2-{3-[5-(4-butyl-2-methyl-3,5-dioxo-2,3,4,5-tetrahydro-[1,2,4]triazin-6-yloxy)-pentyl]-phenoxy}-2-methylpropionat
12. 2-{3-[5-(4-Butyl-2-methyl-3,5-dioxo-2,3,4,5-tetrahydro-[1,2,4]triazin-6-yloxy)-pentyl]-phenoxy}-2-methylpropionsäure
13. Ethyl-2-{3-[5-(2,4-dibutyl-3,5-dioxo-2,3,4,5-tetrahydro-[1,2,4]triazin-6-yloxy)-pentyl]-phenoxy}2-methylpropionat
14. Ethyl-2-(3-{5-[4-butyl-3,5-dioxo-2-(4,4,4trifluoro-butyl}-2,3,4,5-tetrahydro-[1,2,4]triazin-6-yloxy]-pentyl}-phenoxy}2-methylpropionat
15. Ethyl-2-{3-[5-(4-butyl-2-heptyl-3,5-dioxo-2,3,4,5-tetrahydro-[1,2,4]triazin-6-yloxy)-pentyl-phenoxy}-2-methylpropionat
16. Ethyl-2-methyl-2-(3-{5-[2-methyl-3,5-dioxo-4-(4,4,4-trifluoro-butyl)-2,3,4,5-tetrahydro-[1,2,4]triazin-6-yloxy]-pentyl}-phenoxy)-propionat
17. Ethyl-2-{3-[5-(4-heptyl-2-methyl-3,5-dioxo-2,3,4,5-tetrahydro-[1,2,4]triazin-6-yloxy)-pentyl-phenoxy}2-methylpropionat
18. Ethyl-2-(3-{5-[4-heptyl-3,5-dioxo-2-(4,4,4-trifluoro-butyl)-2,3,4,5-tetrahydro-[1,2,4]triazin-6-yloxy]-pentyl}-phenoxy)-2-methylpropionat
19. Ethyl-2-{3-[5-(2-benzyl-4-heptyl-3,5-dioxo-2,3,4,5-tetrahydro-[1,2,4]triazin-6-yloxy)-pentyl]-phenoxy}-2-methylpropionat
20. Ethyl-2-[4-(2-heptyl-4-methyl-3,5-dioxo-2,3,4,5-tetrahydro-[1,2,4]triazin-6-yloxymethyl)-phenoxy]-2-methylpropionat
21. Ethyl-2-methyl-2-{4-[2-methyl-3,5-dioxo-4-(4,4,4-trifluoro-butyl)-2,3,4,5-tetrahydro-[1,2,4]triazin-6-yloxymethyl]-phenoxy}-propionat
22. Ethyl-2-[4-(4-heptyl-2-methyl-3,5-dioxo-2,3,4,5-tetrahydro-[1,2,4]triazin-6-yloxymethyl)-phenoxy]-2-methylpropionat
23. Ethyl-2-{4-[4-heptyl-3,5-dioxo-2-(4,4,4-trifluoro-butyl)-2,3,4,5-tetrahydro-[1,2,4]triazin-6-yloxymethyl]-phenoxy}-2-methylpropionat
24. Ethyl-2-methyl-2-(4-{2-[4-methyl-3,5-dioxo-2-(4,4,4-trifluoro-butyl)-2,3,4,5-tetrahydro-[1,2,4]triazin-6-yloxy]-ethyl}-phenoxy)-propionat
25. Ethyl-2-{4-[2-(2-heptyl-4-methyl-3,5-dioxo-2,3,4,5-tetrahydro-[1,2,4]triazin-6-yloxy)-ethyl]-phenoxy}-2-methylpropionat
26. Ethyl-2-{4-[2-(4-heptyl-2-methyl-3,5-dioxo-2,3,4,5-tetrahydro-[1,2,4]triazin-6-yloxy)-ethyl]-phenoxy)-2-methylpropionat
27. Ethyl-2-{4-[3-(2-heptyl-4-methyl-3,5-dioxo-2,3,4,5-tetrahydro-[1,2,4]triazin-6-yloxy)-propyl]-phenoxy}-2-methylpropionat
28. Ethyl-2-methyl-2-(4-{4-[4-methyl-3,5-dioxo-2-(4,4,4-trifluoro-butyl)-2,3,4,5-tetrahydro-[1,2,4]triazin-6-yloxy]-butyl}-phenoxy)-propionat
29. Ethyl-2-{4-[4-(2-heptyl-4-methyl-3,5-dioxo-2,3,4,5-tetrahydro-[1,2,4]triazin-6-yloxy)-butyl]-phenoxy}-2-methylpropionat
30. Ethyl-2-methyl-2-(4-{4-[2-methyl-3,5-dioxo-4-(4,4,4-trifluoro-butyl)-2,3,4,5-tetrahydro-[1,2,4]triazin-6-yloxy]-butyl}-phenoxy)-propionat
31. Ethyl-2-(4-{4-[4-heptyl-3,5-dioxo-2-(4,4,4-trifluoro-butyl)-2,3,4,5-tetrahydro-[1,2,4]triazin-6-yloxy]-butyl}-phenoxy)-2-methylpropionat
32. Ethyl-2-{3-[3-(2-heptyl-4-methyl-3,5-dioxo-2,3,4,5-tetrahydro-[1,2,4]triazin-6-yloxy)-propyl]-phenylsulfanyl}-2-methylpropionat
33. Ethyl-2-{3-[3-(2-heptyl-4-methyl-3,5-dioxo-2,3,4,5-tetrahydro-[1,2,4]triazin-6-yloxy)-propoxy]-phenoxy}-2-methylpropionat
34. Ethyl-2-{3-[3-(2-heptyl-4-methyl-3,5-dioxo-2,3,4,5-tetrahydro-[1,2,4]triazin-6-yloxy)-propoxy]-phenylsulfanyl}-2-methylpropionat
35. Ethyl-2-(3-{3-[3,5-dioxo-2,4-bis-(4,4,4-trifluoro-butyl)-2,3,4,5-tetrahydro-[1,2,4]triazinr6-yloxy]-propoxy}-phenoxy)-2-methylpropionat
36. Ethyl-2-{3-[4-(2-heptyl-4-methyl-3,5-dioxo-2,3,4,5-tetrahydro-[1,2,4]triazin-6-yloxy)-butoxy]-phenoxy}-2-methylpropionat
37. Ethyl-2-{3-[4-(2-heptyl-4-methyl-3,5-dioxo-2,3,4,5-tetrahydro-[1,2,4]triazin-6-yloxy)-butoxy]-phenylsulfanyl}-2-methylpropionat
38. Ethyl-2-{4-[2-(2-heptyl-4-methyl-3,5-dioxo-2,3,4,5-tetrahydro-[1,2,4]triazin-6-yloxy)-ethoxy]-phenylsulfanyl}-2-methylpropionat
39. Ethyl-2-(4-{3-[3,5-dioxo-2,4-bis-(4,4,4-trifluoro-butyl)-2,3,4,5-tetrahydro-1,2,4]triazin-6-yloxy]-propoxy}-phenoxy)-2-methylpropionat
40. Ethyl-2-{4-[2-(2-heptyl-4-methyl-3,5-dioxo-2,3,4,5-tetrahydro-[1,2,4]triazin-6-ylamino)-ethoxy]-phenoxy}-2-methylpropionat
41. Ethyl-2-(4-{2-[3,5-dioxo-2,4-bis-(4,4,4-trifluoro-butyl)-2,3,4,5-tetrahydro-[1,2,4]triazin-6-ylamino]-ethoxy}-phenoxy)-2-methylpropionat
42. Ethyl-2-{4-[2-(2,4-diheptyl-3,5-dioxo-2,3,4,5-tetrahydro-[1,2,4]triazin-6-ylamino)-ethoxy]-phenoxy}-2-methylpropionat
43. Ethyl-2-(4-{2-[2,4-bis-(3-cyclohexyl-propyl)-3,5-dioxo-2,3,4,5-tetrahydro-(1,2,4]triazin-6-ylamino]-ethoxy}-phenoxy)-2-methylpropionat
44. Ethyl-2-methyl-2-(4-{3-[4-methyl-3,5-dioxo-2-(4,4,4-trifluoro-butyl)-2,3,4,5-tetrahydro-[1,2,4]triazin-6-ylamino]-propoxy}-phenoxy)-propionat
45. Ethyl-2-{4-[3-(2-heptyl-4-methyl-3,5-dioxo-2,3,4,5-tetrahydro-[1,2,4]triazin-6-ylamino)-propoxy]-phenoxy}-2-methylpropionat
46. Ethyl-2-methyl-2-(4-{3-[2-methyl-3,5-dioxo-4-(4,4,4-trifluoro-butyl)-2,3,4,5-tetrahydro-[1,2,4]triazin-6-ylamino]-propoxy}-phenoxy)-propionat
47. Ethyl-2-{4-[3-(4-heptyl-2-methyl-3,5-dioxo-2,3,4,5-tetrahydro-[1,2,4]triazin-6-ylamino)-propoxy]-phenoxy}-2-methylpropionat
48. Ethyl-2-(4-(3-[4-heptyl-3,5-dioxo-2-(4,4,4-trifluoro-butyl}2,3,4,5-tetrahydro-[1,2,4]triazin-6-ylamino]-propoxy}-phenoxy)-2-methylpropionat
49. Ethyl-2-{4-[3-(4-heptyl-2-methyl-3,5-dioxo-2,3,4,5-tetrahydro-[1,2,4]triazin-6-ylamino)-propoxy]-phenylsulfanyl}-2-methylpropionat
50. Ethyl-2-{4-[4-(4-heptyl-2-methyl-3,5-dioxo-2,3,4,5-tetrahydro-[1,2,4]triazin-6-ylamino)-butoxy]-phenoxy}-2-methylpropionat
51. Ethyl-2-(3-{3-[(2-heptyl-4-methyl-3,5-dioxo-2,3,4,5-tetrahydro-[1,2,4]triazin-6-yl)-(4,4,4-trifluoro-butyl)-amino]-propoxy}-phenoxy)-2-methylpropionat
52. Ethyl-2-(3-{3-[(2,4-dimethyl-3,5-dioxo-2,3,4,5-tetrahydro-[1,2,4]triazin-6-yl)-heptyl-amino]-propoxy}-phenylsulfanyl)-2-methylpropionat
53. Ethyl-2-(4-{3-[(2,4-dimethyl-3,5-dioxo-2,3,4,5-tetrahydro-[1,2,4]triazin-6-yl)-heptyl-amino]-propoxy}-phenylsulfanyl)-2-methylpropionat
54. Ethyl-2-(3-{4-[(2,4-dimethyl-3,5-dioxo-2,3,4,5-tetrahydro-[1,2,4] triazin-6-yl)-heptyl-amino]-butoxy}-phenylsulfanyl)-2-methylpropionat
55. Ethyl-2-(2-{2-[3,5-dioxo-2,4-bis-(4,4,4-trifluoro-butyl)-2,3,4,5-tetrahydro-[1,2,4]triazin-6-ylamino]-ethyl}-phenoxy)-2-methylpropionat
56. Ethyl-2-methyl-2-(3-{2-[4-methyl-3,5-dioxo-2-(4,4,4-trifluoro-butyl)-2,3,4,5-tetrahydro-[1,2,4]triazin-6-ylamino]-ethyl}-phenoxy)-propionat
57. Ethyl-2-(3-[2-(2-heptyl-4-methyl-3,5-dioxo-2,3,4,5-tetrahydro-[1,2,4]triazin-6-ylamino)-ethyl]-phenoxy}-2-methylpropionat
58. Ethyl-2-methyl-2-(3-{2-[2-methyl-3,5-dioxo-4-(4,4,4-trifluoro-butyl)-2,3,4,5-tetrahydro-[1,2,4]triazin-6-ylamino]-ethyl}-phenoxy)-propionat
59. Ethyl-2-(3-[2-(4-heptyl-2-methyl-3,5-dioxo-2,3,4,5-tetrahydro-[1,2,4]triazin-6-ylamino)-ethyl]-phenoxy}-2-methylpropionat
60. Ethyl-2-(3-{2-[4-heptyl-3,5-dioxo-2-(4,4,4-trifluoro-butyl)-2,3,4,5-tetrahydro-[1,2,4]triazin-6-ylamino]-ethyl}-phenoxy)-2-methylpropionat
61. Ethyl-2-{3-[2-(4-heptyl-2-methyl-3,5-dioxo-2,3,4,5-tetrahydro-[1,2,4]triazin-6-ylamino)-ethyl]-phenylsulfanyl}-2-methylpropionat
62. Ethyl-2-methyl-2-(3-{3-[4-methyl-3,5-dioxo-2-(4,4,4-trifluoro-butyl)-2,3,4,5-tetrahydro-[1,2,4]triazin-6-ylamino]-propyl}-phenoxy)-propionat
63. Ethyl-2-(3-{3-[2-(2-cyano-ethyl)-4-methyl-3,5-dioxo-2,3,4,5-tetrahydro-[1,2,4]triazin-6-ylamino]-propyl}-phenoxy)-2-methylpropionat
64. Ethyl-2-{3-{3-(2-heptyl-4-methyl-3,5-dioxo-2,3,4,5-tetrahydro-[1,2,4]triazin-6-ylamino)-propyl]-phenoxy}2-methylpropionat
65. Ethyl-2-methyl-2-(3-{3-[2-methyl-3,5-dioxo-4-(4,4,4-trifluoro-butyl)-2,3,4,5-tetrahydro-[1,2,4]triazin-6-ylamino]-propyl}-phenoxy)-propionat
66. Ethyl-2-(3-{3-[3,5-dioxo-2,4-bis-(4,4,4-trifluoro-butyl)-2,3,4,5-tetrahydro-[1,2,4]triazin-6-ylamino]-propyl}-phenoxy)-2-methylpropionat
67. Ethyl-2-{3-[3-(4-heptyl-2-methyl-3,5-dioxo-2,3,4,5-tetrahydro-[1,2,4]triazin-6-ylamino)-propyl]-phenoxy}-2-methylpropionat
68. Ethyl-2-(3-{3-[4-heptyl-3,5-dioxo-2-(4,4,4-trifluoro-butyl)-2,3,4,5-tetrahydro-[1,2,4]triazin-6-ylamino]-propyl}-phenoxy)-2-methylpropionat
69. Ethyl-2-{3-(3-(2,4-diheptyl-3,5-dioxo-2,3,4,5-tetrahydro-[1,2,4]triazin-6-ylamino)-propyl]-phenoxy}-2-methylpropionat
70. Ethyl-2-{3-[3-(2-benzyl-4-heptyl-3,5-dioxo-2,3,4,5-tetrahydro-[1,2,4]triazin-6-ylamino)-propyl]-phenoxy}-2-methylpropionat
71. Ethyl-2-(3-{3-[4-benzyl-3,5-dioxo-2-(4,4,4-trifluoro-butyl)-2,3,4,5-tetrahydro-[1,2,4]triazin-6-ylamino]-propyl}-phenoxy)-2-methylpropionat
72. Ethyl-2-{3-[3-(4-benzyl-2-heptyl-3,5-dioxo-2,3,4,5-tetrahydro-[1,2,4]triazin-6-ylamino}-propyl]-phenoxy}-2-methylpropionat
73. Ethyl-2-{3-[3-(4-heptyl-2-methyl-3,5-dioxo-2,3,4,5-tetrahydro-[1,2,4]triazin-6-ylamino)-propyl]-phenylsulfanyl}-2-methylpropionat
74. Ethyl-2-methyl-2-(3-{4-[4-methyl-3,5-dioxo-2-(4,4,4-trifluoro-butyl)-2,3,4,5-tetrahydro-[1,2,4]triazin-6-ylamino]-butyl}-phenoxy)-propionat
75. Ethyl-2-{3-[4-(2-heptyl-4-methyl-3,5-dioxo-2,3,4,5-tetrahydro-[1,2,4]triazin-6-ylamino)-butyl]-phenoxy}-2-methylpropionat
76. Ethyl-2-methyl-2-(3-{4-[4-(3-methyl-but-2-enyl}-3,5-dioxo-2-(4,4,4-trifluoro-butyl)-2,3,4,5-tetrahydro-[1,2,4]triazin-6-ylamino]-butyl}-phenoxy)-propionat
77. Ethyl-2-methyl-2-(3-{4-[2-methyl-3,5-dioxo-4-(4,4,4-trifluoro-butyl)-2,3,4,5-tetrahydro-[1,2,4]triazin-6-ylamino]-butyl}-phenoxy)-propionat
78. tert-Butyl-2-methyl-2-(3-{4-[2-methyl-3,5-dioxo-4-(4,4,4-trifluorabutyl)-2,3,4,5-tetrahydro-[1,2,4]triazin-6-ylamino]-butyl}-phenoxy)-propionat
79. 2-Methyl-2-(3-{4-[2-methyl-3,5-dioxo-4-(4,4,4-trifluoro-butyl)-2,3,4,5-tetrahydro-[1,2,4]triazin-6-ylamino]-butyl}-phenoxy)-propionsäure
80. Ethyl-2-(3-{4-[3,5-dioxo-2,4-bis-(4,4,4-trifluoro-butyl)-2,3,4,5-tetrahydro-[1,2,4]triazin-6-ylamino]-butyl}-phenoxy)-2-methylpropionat
81. Ethyl-2-(3-{4-[2-(2-cyano-ethyl)-3,5-dioxo-4-(4,4,4-trifluoro-butyl)-2,3,4,5-tetrahydro-[1,2,4]triazin-6-ylamino]-butyl}-phenoxy)-2-methylpropionat
82. Ethyl-2-(3-{4-[2-heptyl-3,5-dioxo-4-(4,4,4-trifluoro-butyl)-2,3,4,5-tetrahydro-[1,2,4]triazin-6-ylamino]-butyl}-phenoxy)-2-methylpropionat
83. tert-Butyl-2-(3-{4-[2-heptyl-3,5-dioxo-4-(4,4,4-trifluoro-butyl)-2,3,4,5-tetrahydro-[1,2,4]triazin-6-ylamino]-butyl}-phenoxy)-2-methylpropionat
84. 2-(3-{4-[2-Heptyl-3,5-dioxo-4-(4,4,4-trifluoro-butyl)-2,3,4,5-tetrahydro-[1,2,4]triazin-6-ylamino]-butyl}-phenoxy)-2-methyl-propionsäure
85. Ethyl-2-(3-{4-[4-heptyl-3,5-dioxo-2-(4,4,4-trifluoro-butyl)-2,3,4,5-tetrahydro-[1,2,4]triazin-6-ylamino]-butyl}-phenoxy)-2-methylpropionat
86. Ethyl-2-(3-{4-[2-(2-cyano-ethyl)-4-heptyl-3,5-dioxo-2,3,4,5-tetrahydro-[1,2,4]triazin-6-ylamino]-butyl}-phenoxy)-2-methylpropionat
87. Ethyl-4-(6-{4-[3-(1-ethoxycarbonyl-1-methyl-ethoxy)-phenyl]-butylamino}-4-heptyl-3,5-dioxo-4,5-dihydro-3H-[1,2,4]triazin-2-yl)-but-2-enonat
88. Ethyl-2-{3-[4-(2,4-diheptyl-3,5-dioxo-2,3,4,5 tetrahydro-[1,2,4]triazin-6-ylamino)-butyl]-phenoxy}-2-methylpropionat
89. Ethyl-2-{3-[4-(2-benzyl-4-heptyl-3,5-dioxo-2,3,4,5-tetrahydro-[1,2,4]triazin-6-ylamino)-butyl]-phenoxy}-2-methylpropionat
90. Ethyl-2-(3-{4-[4-benzyl-3,5-dioxo-2-(4,4,4-trifluoro-butyl)-2,3,4,5-tetrahydro-[1,2,4]triazin-6-ylamino]-butyl}-phenoxy)-2-methylpropionat
91. Ethyl-2-{3-[4-(4-benzyl-2-heptyl-3,5-dioxo-2,3,4,5-tetrahydro-[1,2,4]triazin-6-ylamino)-butyl]-phenoxy}-2-methylpropionat
92. Ethyl-2-(3-{5-[3,5-dioxo-2,4-bis-(4,4,4-trifluoro-butyl)-2,3,4,5-tetrahydro-[1,2,4]triazin-6-ylamino]-pentyl}-phenoxy)-2-methylpropionat
93. Ethyl-2-methyl-2-(3-{4-[4-methyl-3,5-dioxo-2-(4,4,4-trifluoro-butyl)-2,3,4,5-tetrahydro-[1,2,4]triazin-6-ylamino]-butyl}-phenylsulfanyl}-propionat
94. Ethyl-2-{3-[4-(2-heptyl-4-methyl-3,5-dioxo-2,3,4,5-tetrahydro-[1,2,4]triazin-6-ylamino}-butyl]-phenylsulfanyl}-2-methylpropionat
95. Ethyl-2-methyl-2-(3-{4-[2-methyl-3,5-dioxo-4-(4,4,4-trifluoro-butyl)-2,3,4,5-tetrahydro-[1,2,4]triazin-6-ylamino]-butyl}-phenylsulfanyl)-propionat
96. 2-Methyl-2-(3-(4-[2-methyl-3,5-dioxo-4-(4,4,4-trifluoro-butyl)-2,3,4,5-tetrahydro-[1,2,4]triazin-6-ytamino]-butyl)-phenylsulfanyl)-propionsäure
97. Ethyl-2-(3-{4-[4-heptyl-3,5-dioxo-2-(4,4,4-trifluoro-butyl)-2,3,4,5-tetrahydro-[1,2,4]triazin-6-ylamino]-butyl}-phenylsulfanyl)-2-methylpropionat
98. Ethyl-2-{3-[4(2,4-diheptyl-3,5-dioxo-2,3,4,5-tetrahydro-[1,2,4]triazin-6-ylamino)-butyl]-phenylsulfanyl}-2-methylpropionat
99. Ethyl-2-{3-[4-(2-benzyl-4-heptyl-3,5-dioxo-2,3,4,5-tetrahydro-[1,2,4]triazin-6-ylamino)-butyl]-phenylsulfanyl)-2-methylpropionat
100. Ethyl-2-{3-[4(4-benzyl-2-heptyl-3,5-dioxo-2,3,4,5-tetrahydro-[1,2,4]triazin-6-ylamino)-butyl]-phenylsulfanyl}-2-methylpropionat
101. Ethyl-2-{3-[4(4-benzyl-2-heptyl-3,5-dioxo-2,3,4,5-tetrahydro-[1,2,4]triazin-6-ylamino)-butyl]-phenylsulfanyl}-2-methylpropionat
102. Ethyl-2-methyl-2-(4-[2-[4-methyl-3,5dioxo-dioxo-2-(4,4,4-trifluoro-butyl)-2,3,4,5-tetrahydro-[1,2,4]triazin-6-ylamino)-ethyl]-phenoxy)-propionat
103. Ethyl-2-{4-[2-(2-heptyl-4-methyl-3,5-dioxo-2,3,4,5-tetrahydro-[1,2,4]triazin-6-ylamino)-ethyl]-phenoxy}-2-methylpropionat
104. Ethyl-4-[6-{2-[4-(1-ethoxycarbonyl-1-methyl-ethoxy)-phenyl]-ethylamino}-3,5-dioxo-4-(4,4,4,-trifluoro-butyl)-4,5-dihydro-3H-[1,2,4]triazin-2-yl]-but-2-enoat
105. Ethyl-2-(4-{2-[2-(3-cyclohexyl-propyl)-3,5-dioxo-4-(4,4,4-trifluoro-butyl)-2,3,4,5-tetrahydro-[1,2,4]triazin-6-ylamino]-ethyl}-phenoxy)-2-methylproprionat
106. Ethyl-2-{4-[2-(4-heptyl-2-methyl-3,5-dioxo-2,3,4,5-tetrahydro-[1,2,4]triazin-6-ylamino)-ethyl]-phenoxy}-2-methylpropionat
107. Ethyl-2-(4-{2-[4-heptyl-3,5-dioxo-2(4,4,4-trifluoro-butyl)-2,3,4,5-tetrahydro-[1,2,4]triazin-6-ylamino)-ethyl}-phenoxy)-2-methylpropionat
108. Ethyl-2-{4-[2-(2,4-diheptyl-3,5-dioxo-2,3,4,5-tetrahydro-[1,2,4]triazin-6-ylamino)-ethyl]-phenoxy}-2-methylpropionat
109. Ethyl-2-{4-[2-(4-benzyl-2-heptyl-3,5-dioxo-2,3,4,5-tetrahydro-[1,2,4]triazin-6-ylamino)-ethyl]-phenoxy}-2-methylpropionat
110. Ethyl-2-{4-[2-(2,4-bis-benzyloxymethyl-3,5-dioxo-2,3,4,5-tetrahydro-[1,2,4]triazin-6-ylamino)-ethyl]-phenoxy}-2-methylpropionat
111. Ethyl-2(4-{2[(2,4-dimethyl-3,5-dioxo-2,3,4,5-tetrahydro-[1,2,4]triazin-6-yl)-(4,4,4-trifluoro-butyl)-amino]-ethyl}-phenoxy)-2-methylpropionat
112. Ethyl-2(4-{2[(2,4-dimethyl-3,5-dioxo-2,3,4,5-tetrahydro-[1,2,4]triazin-6-yl)-heptyl)-amino]-ethyl}-phenoxy)-2-methylpropionat
113. 2(4-{2[(2,4-dimethyl-3,5-dioxo-2,3,4,5-tetrahydro-[1,2,4]triazin-6-yl)-heptyl-amino]-ethyl}-phenoxy)-2-methyl-propionsäure
114. Ethyl-2(4-(2[(2,4-dimethyl-3,5-dioxo-2,3,4,5-tetrahydro-[1,2,4]triazin-6-yl)-phenethyl-amino]-ethyl)-phenoxy)-2-methylpropionat
115. Ethyl-2(4-{2[(2,4-dimethyl-3,5-dioxo-2,3,4,5-tetrahydro-[1,2,4]triazin-6-yl)-phenyl-propyl)-amino]-ethyl}-phenoxy)-2-methylpropionat
116. Ethyl-2-(4-{2-[(4-heptyl-2-methyl-3,5-dioxo-2,3,4,5-tetrahydro-[1,2,4]triazin-6-yl)-phenethyl-amino]-ethyl}-phenoxy)-2-methylpropionat
117. Ethyl-2-(4-{2-[(4-heptyl-2-methyl-3,5-dioxo-2,3,4,5-tetrahydro-[1,2,4]triazin-6-yl)-(3-phenylpropyl)-amino]-ethyl}-phenoxy)-2-methylpropionat
118. 2-Methyl-2-(4-{2-[4-methyl-3,5-dioxo-2-(4,4,4-trifluoro-butyl)-2,3,4,5-tetrahydro-[1,2,4]triazin-6-ylamino]-ethyl}-phenylsulfanyl)-propionsäure
119. Ethyl-2-{4-[2-(2-heptyl-4-methyl-3,5-dioxo-2,3,4,5-tetrahydro-[1,2,4]triazin-6-ylamino)-ethyl]-phenylsulfanyl}-2-methylpropionat
120. 2-{4-[2-(2-Heptyl-4-methyl-3,5-dioxo-2,3,4,5-tetrahydro-[1,2,4]triazin-6-ylamino)-ethyl]-phenylsulfanyl}-2-methyl-propionsäure
121. 2-{4-12-(4-Butyl-2-heptyl-3,5-dioxo-2,3,4,5-tetrahydro-[1,2,4]triazin-6-ylamino)-ethyl]-phenylsulfanyl}-2-methyl-propionsäure
122. Ethyl-2-{4-[2-(4-heptyl-2-methyl-3,5-dioxo-2,3,4,5-tetrahydro-[1,2,4]triazin-6-ylamino)-ethyl]-phenylsulfanyl}-2-methylpropionat
123. 2-(4-{2-[4-Heptyl-3,5-dioxo-2-(4,4,4-trifluoro-butyl)-2,3,4,5-tetrahydro-[1,2,4]triazin-6-ylamino]-ethyl}-phenylsulfanyl)-2-methyl-propionsäure
124. Ethyl-2-{4-[3-(2-heptyl-4-methyl-3,5-dioxo-2,3,4,5-tetrahydro-[1,2,4]triazin-6-ylamino)-propyl]-phenylsulfanyl}-2-methylpropionat

6. Verfahren zur Zubereitung der chemischen Verbindungen nach einem der Ansprüche 1 bis 5, **dadurch gekennzeichnet, dass**
ein Derivat der allgemeinen Formel **II** 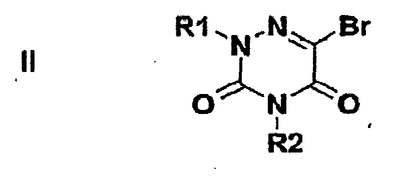 kondensiert wird, wobei **R₁** und **R₂** Gruppen darstellen, wie zuvor in der Formel **I** beschrieben mit einem Derivat der allgemeinen Formel **III**, 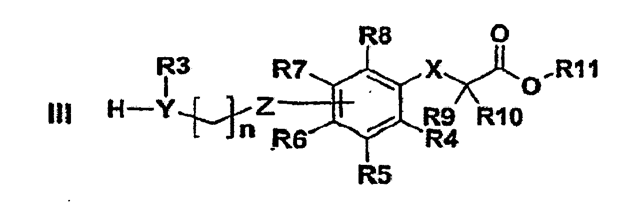 wobei **YR₃, n, Z, X, R₄, R₅, R₆, R₇, R₈, R₉, R₁₀** und **R₁₁** derart sind, wie zuvor in der Formel I beschrieben, vor allem bei Anwesenheit einer Base wie dem Triethylamin im n-Butanol (wenn **Y**=N) oder von Kaliumkarbonat im Dimethylformamid (wenn **YR₃**=O).

7. Verfahren zur Zubereitung der chemischen Verbindungen nach einem der Ansprüche 1 bis 5 der allgemeinen Formel **I** für die **Z**=O, **dadurch gekennzeichnet, dass**
a) ein Derivat der allgemeinen Formel **II** 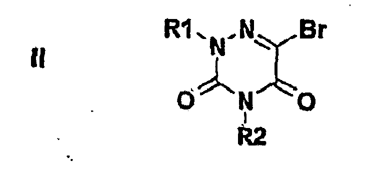 kondensiert wird, wobei **R₁** und **R₂** Gruppen darstellen, wie zuvor in der Formel **I** beschrieben mit einem Derivat der allgemeinen Formel **IV,** 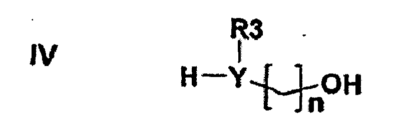 wobei **R₃Y** gleich NH oder O sein kann und **n** derart ist, wie zuvor in der Formel **I** beschrieben, vor allem bei Abwesenheit von Lösungsmittel ohne Hinzugabe von Base (wenn **R₃Y**=NH) oder bei Anwesenheit einer Base wie K₂CO₃ (wenn **R₃Y**=O),
b) das erhaltene Derivat **V** 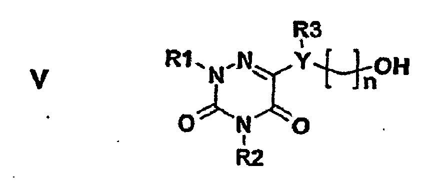 kondensiert wird mit einer Verbindung der allgemeinen Formel **VI,** 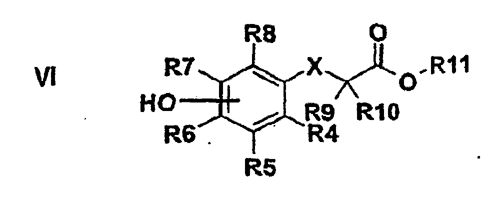
wobei **X, R₄, R₅, R₆**, **R₇, R₈, R₉, R₁₀** und **R₁₁** derart sind, wie zuvor in der Formel **I** beschrieben, vor allem unter den Bedingungen wie denen der Mitsunobu-Reaktion bei Anwesenheit von Triphenylphosphin und von Diethylazodicarboxylat in THF.

8. Verfahren zur Zubereitung der chemischen Verbindungen nach einem der Ansprüche 1 bis 5 der allgemeinen Formel **I** für die **Z**=O, **dadurch gekennzeichnet, dass**
a) die Alkoholfunktion eines Derivats der allgemeinen Formel **VII** 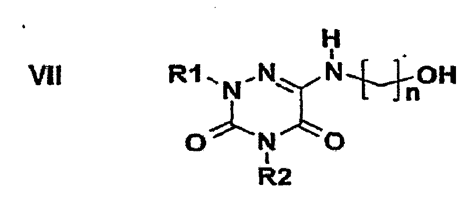 geschützt wird, wobei **R₁, R₂** und **n** derart sind, wie zuvor in der Formel **I** beschrieben, durch eine Schutzgruppe, zu denen tert-Butyldimethylsilan gehört, vor allem unter den Bedingungen wie THF bei Verwendung von tert-Butyl(chlor)dimethylsilan und Imidazol, 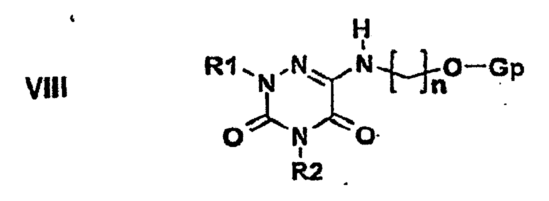
b) der Stickstoff der zuvor hergestellten Verbindung **VIII** alkyliert wird durch ein halogeniertes Derivat **R₃**Hal, wobei die **Hal**-Gruppe ein Halogen darstellt wie Cl, Br oder I und **R₃** derart ist, wie zuvor in der Formel **I** beschrieben, unter den operativen Bedingungen wie NaH oder tBuOK in DMF, 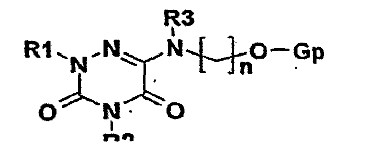
c) der Schutz der damit hergestellten Verbindung **IX** aufgehoben wird, unter operativen Bedingungen wie Tetrabutylammonium-Fluorid in THF,
d) das hergestellte Derivat **X** 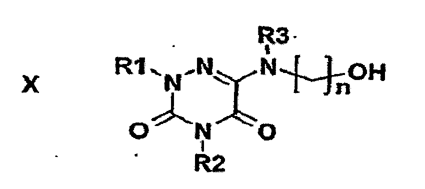 kondensiert wird mit einer Verbindung der allgemeinen Formel **VI,** 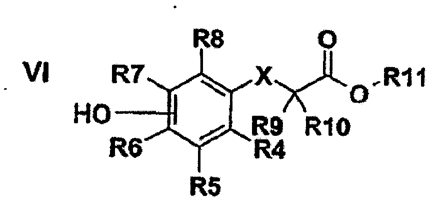
wobei **X, R₄, R₅, R₆, R₇, R_{8,} R₉, R₁₀** und **R₁₁** derart sind, wie zuvor in der Formel I beschrieben, vor allem unter den Bedingungen wie denen der Mitsunobu-Reaktion bei Anwesenheit von Triphenylphosphin und von Diethylazodicarboxylat in THF.

9. Verfahren zur Zubereitung der chemischen Verbindungen nach einem der Ansprüche 1 bis 5 der allgemeinen Formel **I** wenn **Y**=N und **Z**=C, **dadurch gekennzeichnet, dass**
a) ein Derivat der allgemeinen Formel **II** 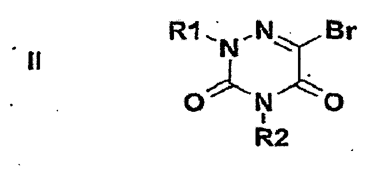 kondensiert wird, wobei **R₁** und **R₂** Gruppen darstellen, wie zuvor in der Formel **I** beschrieben, mit einem Derivat der allgemeinen Formel **XI** 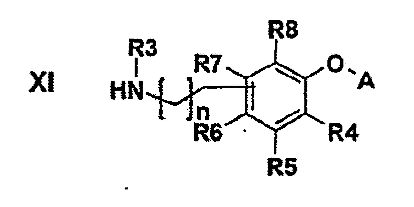 wobei **n, R₃, R₄, R₅, R₆, R₇** und **R₈** derart sind, wie zuvor in der Formel I beschrieben und **A** Wasserstoff oder eine Methylgruppe sein kann, vor allem bei Anwesenheit einer Base wie Triethylamin im n-Butanol,
b) nach Demethylierung (wenn **A**=Me, unter Bedingungen, wie **BBr₃** im Dichlormethan) die Phenolfunktion des Derivats **XII** 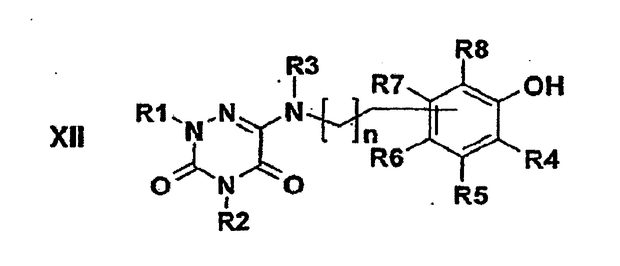 alkyliert wird durch ein halogeniertes Derivat der allgemeinen Formel **XIII** (als Lösungsmittel bei Anwesenheit einer Base wie Kaliumkarbonat verwendet), 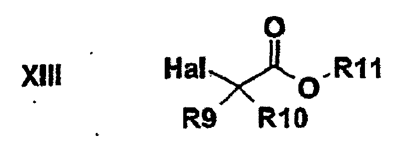
wobei die Hal-Gruppe ein Halogen darstellt wie Cl, Br oder I und **R₉, R₁₀** und **R₁₁** derart sind, wie zuvor in der allgemeinen Formel I beschrieben.

10. Verfahren zur Zubereitung der chemischen Verbindungen nach einem der Ansprüche 1 bis 5 der allgemeinen Formel I wenn **Y**=N, **Z**=C, **dadurch gekennzeichnet, dass**
a) ein Derivat der allgemeinen Formel **XIV** 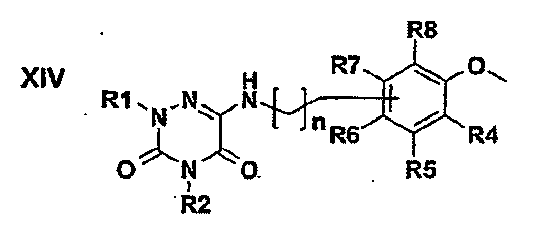 alkyliert wird, wobei **R₁, R₂, R₄, R₅, R₆, R₇ R₈** und **n** derart sind, wie zuvor in der Formel I beschrieben, mit einem Derivat der Formel R₃Hal, wobei die **Hal-**Gruppe ein Halogen darstellt wie Cl, Br oder I und **R₃** derart ist, wie zuvor in der Formel I beschrieben, unter den operativen Bedingungen wie bei Anwesenheit von NaH oder tBuOK in DMF,
b) nach Demethylierung unter den Bedingungen wie BBr₃ im Dichlormethan die Phenolfunktion des derart hergestellten Derivats **XII** 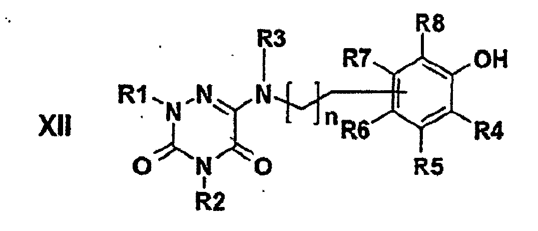 alkyliert wird von einem halogenierten Derivat der allgemeinen Formel **XIII** (verwendet als Lösungsmittel bei Anwesenheit einer Base wie Kaliumkarbonat), 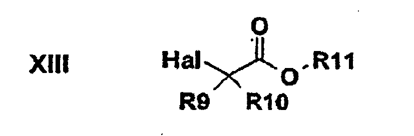
wobei die **Hal**-Gruppe ein Halogen darstellt wie Cl, Br oder I, und **R₉, R₁₀** und **R₁₁** derart sind, wie zuvor in der allgemeinen Formel **I** beschrieben.

11. Verfahren zur Zubereitung der chemischen Verbindungen nach einem der Ansprüche 1 bis 5, **dadurch gekennzeichnet, dass**
a) ein Derivat der allgemeinen Formel **I** 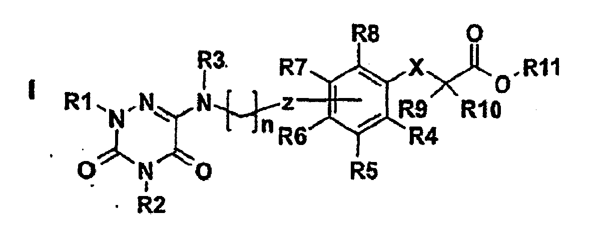 platziert wird, wobei **R₁**=(CH₂)₂CN und **R₂, R₃, n, Z, X, R₄, R₅, R₆, R₇, R₈, R₉, R₁₀** und **R₁₁** derart sind, wie zuvor in der Formel **I** beschrieben oder **R₂**= (CH₂)₂CN und **R₁, R₃, n, Z, X, R₄, R₅, R₆, R₇, R₈, R₉, R₁₀** und **R₁₁** derart sind, wie zuvor in der Formel I beschrieben unter den operativen Bedingungen wie bei Anwesenheit einer Base NaH in DMF,
b) danach der Stickstoff des Triazins des derart hergestellten Derivats **XIVa** oder **XIVb** 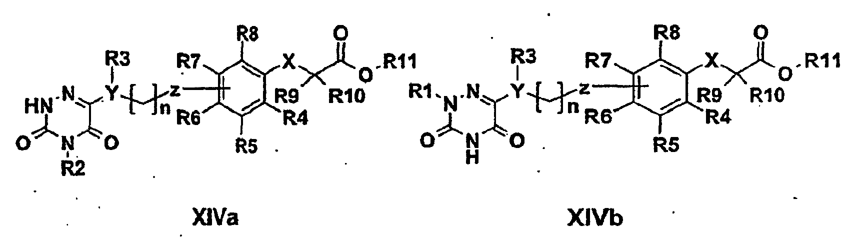 alkyliert wird durch ein halogeniertes Derivat der allgemeinen Formel **R₁Hal** im Fall des Mittlers **XIVa** und der allgemeinen Formel **R₂Hal** im Fall des Mittlers **XIVb,** wobei die **Hal**-Gruppe ein Halogen wie Cl, Br oder I darstellt und **R₁** und **R₂** derart sind, wie zuvor in der Formel I beschrieben, unter den operativen Bedingungen wie bei Anwesenheit von HaH oder tBuOK im DMF.

12. Als neue Arzneimittel, die zur Behandlung von Krankheiten verwendbar sind, die Agonisten der PPARalpha- und/oder PPAR-gamma-Rezeptoren benötigen, die nach einem der Ansprüche 1 bis 5 definierten Verbindungen.

13. Als neue Arzneimittel, die zur Vorbeugung und Behandlung von Krankheiten wie die diabetischen Dyslipidämien, die Hypertriglyceridemie, die Hypercholesterolemie, die Hyperinsulinemie, die Hyperglykämie, das metabolische Syndrom, die Obesität, die Arteriosklerose verwendbar sind, die nach einem der Ansprüche 1 bis 5 definierten Verbindungen.

14. Als neue Arzneimittel, die in der Dermatologie, bei Erkrankungen mit entzündlicher Komponente oder die aus einer abnormen Zelldifferenzierung resultieren, die nach einem der Ansprüche 1 bis 5 definierten Verbindungen.

15. Als neue Arzneimittel, die zur Behandlung von Erkrankungen wie die Psoriasis, die Akne, die atopischen Dermatiden, die Hautalterung, die Fotoalterung verwendbar sind, die nach einem der Ansprüche 1 bis 5 definierten Verbindungen.

16. Pharmazeutische Zusammensetzung, **dadurch gekennzeichnet, dass** sie als Wirkprinzip eine Verbindung enthält, die nach einem der Ansprüche 1 bis 5 definiert ist in Verbindung mit jedem geeigneten Arzneiträger.
